# EUROPEAN PATENT APPLICATION

(11) **EP 4 722 212 A1**
(43) Date of publication of application: **08.04.2026**
(21) Application number: 24811433.2
(22) Date of filing: 24.05.2024
(51) Int. Cl.: C07D 471/04, A61K 31/519, A61P 35/00, A23L 33/10

(54) **NOVEL COMPOUND FOR INHIBITING PROTEIN KINASES AND PHARMACEUTICAL COMPOSITION COMPRISING SAME**

(30) Priority: 25.05.2023 KR 20230067836; 20.05.2024 KR 20240065022
(71) Applicant: Magicbullettherapeutics Co., Ltd., Seoul 03722 (KR)
(72) Inventor: SIM, Tae Bo, Seoul 06635 (KR); CHOI, Ha Soon, Seoul 07225 (KR); SHIN, In Jae, Seoul 04341 (KR); KIM, Nam Kyoung, Seoul 03782 (KR); LEE, Jung Yeol, Daegu 41061 (KR); PARK, Sun You, Daegu 41122 (KR); CHOI, Myeong A, Daegu 41072 (KR); LEE, Ji Hoon, Seoul 04403 (KR); KWON, Ye-Mi, Daegu 41069 (KR); KIM, Sang Kyoon, Daegu 41073 (KR); KIM, Da Yea, Daegu 41071 (KR); MOON, Uk Yeol, Daegu 41086 (KR)
(74) Representative: Mewburn Ellis LLP
(86) International application number: PCT/KR2024/007047
(87) International publication number: WO 2024/242495

(57) **Abstract**

The present invention relates to novel compounds for inhibiting protein kinases and pharmaceutical compositions comprising the same, and more particularly, since the compounds according to the present invention can effectively inhibit various protein kinases, they can be usefully utilized in the development of therapeutics for protein kinase-related diseases, particularly cancer diseases.

## Description

### [TECHNICAL FIELD]

The present invention relates to novel compounds for protein kinase inhibition and pharmaceutical compositions comprising the same. More specifically, the present invention relates to novel 6' - 4 - methylphenyl-5,6'-dihydro-7H-spiro[cyclopropane-1,8'-pyrido[4,3-d]pyrimidin]-7-one compounds having protein kinase inhibitory activity, pharmaceutically acceptable salts thereof, hydrates thereof, or stereoisomers thereof, methods for preparing said compounds, and pharmaceutical compositions comprising said compounds for the prevention, amelioration, or treatment of cancer.

### [Background of the Invention]

Cells contain various cellular signaling systems that regulate cell proliferation, growth, metastasis, and apoptosis, with each component being intricately interconnected. When regulatory functions within cells are disrupted due to genetic or environmental factors, abnormal cell proliferation or disruption of cellular signaling pathways can cause the transformation of normal cells to malignant cells.

Protein Tyrosine Kinase (PTK) plays an important role in cellular signaling related to cell growth, differentiation, and proliferation. Abnormal mutations or overexpression of specific protein tyrosine kinases disrupt normal intracellular signaling systems, which may cause various diseases such as cancer, inflammation, metabolic disorders, and neurological diseases. Protein tyrosine kinase (PTK) catalyzes the phosphorylation of tyrosine residues on substrate proteins, thereby mediating the transduction of extracellular growth factor signals into intracellular responses. Under normal conditions, interactions between growth factors and their receptors regulate cellular proliferation, whereas mutations or overexpression of receptor proteins can cause aberrant signal transduction, leading to tumorigenesis.

Accordingly, there is still an unmet medical need for the development of more selective and effective protein tyrosine kinase inhibitors exhibiting enhanced anticancer efficacy compared to existing kinase inhibitors.

### [Detailed Description]

### [Technical Problem]

The purpose of the present invention is to provide compounds possessing inhibitory activities against protein kinases.

Another purpose of the present invention is to provide a pharmaceutical composition comprising novel compounds that inhibit protein kinases for the prevention or treatment of protein kinase-mediated diseases.

A further purpose of the present invention is to provide healthy functional food comprising novel compounds for ameliorating or preventing protein kinase-mediated diseases.

### [Solution to the Problem]

The present invention provides a compound selected from the group consisting of a compound represented by the following Formula 1, stereoisomers thereof, pharmaceutically acceptable salts thereof, precursors thereof, or solvates thereof: 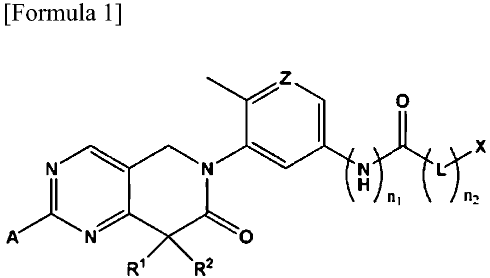 wherein:
R₁ and R₂ may each be independently selected from hydrogen or (C₁-C₄) alkyl, or may be taken together to form a 3- to 5-membered ring;
X is selected from (substituted or unsubstituted) (C₁-C₄)alkyl, (C₃-C₆)cycloalkyl, (C₄-C₆)cycloalkenyl, phenyl, benzyl, or 5- or 6-membered monocyclic or bicyclic heterocyclic rings, wherein the substituent is selected from one or more members of the group consisting of (C₁-C₄)alkyl, (C₁-C₄)alkoxy, (C₃-C₆)cycloalkyl, trifluoromethyl, trifluoromethyl(C₁-C₂)alkoxy, halo, hydroxy, imidazolyl, (C₁-C₂)alkylimidazolyl, (C₁-C₂)alkoxyphenyl, morpholino, cyano, oxo (=O), (C₁-C₂)alkoxy(C₁-C₂)alkyl,
(C₁-C₂)alkylamino, piperazinyl, (C₁-C₂)alkylpiperazinyl, ((C₁-C₂)alkylpiperazin-1-yl)(C₁-C₂)alkyl, and di(C₁-C₂)alkylamino(C₁-C₂)alkyl(C₁-C₂)alkylamino,
A is selected from morpholino or -NH-Y, wherein Y is hydrogen, or substituted or unsubstituted group selected from phenyl, benzyl, (C₁-C₄)alkyl, (C₂-C₃)alkenyl, (C₃-C₆)cycloalkyl, pyridinyl, pyrazolyl, tetrahydropyranyl, benzothiazolyl, benzofuranyl, or azetidinyl, wherein the substituent is selected from one or more members of the group consisting of (C₁-C₂)alkyl, (C₁-C₄)alkoxy, piperazinyl, (C₁-C₂)alkylpiperazinyl, acetylpiperazinyl, morpholino, morpholine-4-carbonyl, (C₁-C₂)alkylpiperazine-1-carbonyl, oxetanyl, ((C₁-C₂)alkylpiperazin-1-yl)(C₁-C₂)alkyl, imidazolyl, pyrrolidinyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, halo, trifluoro(C₁-C₂)alkyl, trifluoro(C₁-C₂)alkoxy, di(C₁-C₂)alkylamino(C₁-C₂)alkyl(C₁-C₂)alkylamino, pyridinyl, furanyl, hydroxy and tert-butoxycarbonyl (BOC); Z is CH or N; L is CH₂ or NH; n₁ or n₂ may be an integer from 0 to 1.

The present invention provides a pharmaceutical composition comprising novel compounds that inhibit protein kinases for the prevention or treatment of protein kinase-mediated diseases.

Further, the present invention provides a health functional food comprising novel compounds for ameliorating or prevention of protein kinase-mediated diseases.

### [Advantageous Effects of the Invention]

The compounds according to the present invention effectively inhibit the activity of protein tyrosine kinases of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19, ZAK/MLTK, and various other protein kinases. Therefore, the compounds may be useful for the prevention, treatment, or amelioration of cancers or cancer-related diseases associated with abnormal cell growth.

The compound according to the present invention, or pharmaceutically acceptable salts thereof, or hydrates thereof, and pharmaceutical compositions comprising the same as an active ingredient for the prevention or treatment of cancers or cancer-related diseases, exhibit low cytotoxicity and high selectivity in inhibitory activity and antiproliferative effects against cancer cells. Accordingly, the compounds and compositions may be effectively used for the prevention or treatment of various cancers such as gastric cancer, lung cancer, liver cancer, colorectal cancer, small intestinal cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, renal cancer, sarcoma, prostate cancer, urethral cancer, bladder cancer, hematologic cancers (including leukemia, multiple myeloma, myelodysplastic syndrome), lymphomas (including Hodgkin's disease, non-Hodgkin's lymphoma), psoriasis, or fibroadenoma.

### [BEST MODE FOR CARRYING OUT THE INVENTION]

Hereinafter, the present invention will be described in more detail.

The present inventors conducted intensive research to develop a more effective protein kinase inhibitor and, as a result, synthesized novel compounds based on 6' - 4 - methylphenyl-5,6'-dihydro-7H-spiro[cyclopropane-1,8'-pyrido[4,3-d]pyrimidin]-7'-one groups. The inventors confirmed that these compounds effectively inhibit various protein kinases, thereby accomplishing the present invention.

Accordingly, the present invention provides a compound selected from the group consisting of a compound represented by the following Formula 1, stereoisomers thereof, pharmaceutically acceptable salts thereof, precursors thereof, or solvates thereof: 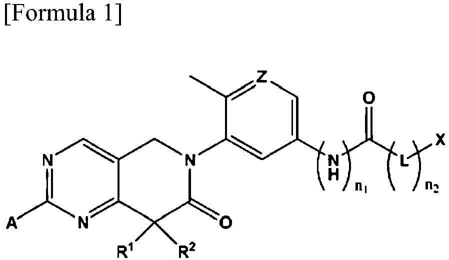 wherein:
R¹ and R² may each independently selected from hydrogen or (C₁-C₄) alkyl, or may be taken together to form a 3- to 5-membered ring;
X is selected from (substituted or unsubstituted) (C₁-C₄)alkyl, (C₃-C₆)cycloalkyl, (C₄-C₆)cycloalkenyl, phenyl, benzyl, or 5- or 6-membered monocyclic or bicyclic heterocyclic rings, wherein the substituent is selected from one or more members of the group consisting of (C₁-C₄)alkyl, (C₁-C₄)alkoxy, (C₃-C₆)cycloalkyl, trifluoromethyl, trifluoromethyl(C1-C2)alkoxy, halo, hydroxy, imidazolyl, (C₁-C₂)alkylimidazolyl, (C₁-C₂)alkoxyphenyl, morpholino, cyano, oxo (=O), (C₁-C₂)alkoxy(C₁-C₂)alkyl, (C₁-C₂)alkylamino, piperazinyl, (C₁-C₂)alkylpiperazinyl, ((C₁-C₂)alkylpiperazin-1-yl)(C₁-C₂)alkyl, and di(C₁-C₂)alkylamino(C₁-C₂)alkyl, and wherein the 5- or 6-membered monocyclic or bicyclic heterocyclic rings are selected from the group consisting of thiophene, furan, pyrazole, oxazole, thiazole, pyridine, pyran, tetrahydropyran, oxazine, thiazine, pyrimidine, piperazine, and benzothiazole,
A is selected from morpholino or -NH-Y, wherein Y is selected from hydrogen, or substituted or unsubstituted group selected from phenyl, benzyl, (C₁-C₄)alkyl, (C₂-C₃)alkenyl, (C₃-C₆)cycloalkyl, pyridinyl, pyrazolyl, tetrahydropyranyl, benzothiazolyl, benzofuranyl, or azetidinyl, wherein the substituent is selected from one or more members of the group consisting of (C₁-C₂)alkyl, (C₁-C₄)alkoxy, piperazinyl, (C₁-C₂)alkylpiperazinyl, acetylpiperazinyl, morpholino, morpholine-4-carbonyl, (C₁-C₂)alkylpiperazine-l-carbonyl, oxetanyl, ((C₁-C₂)alkylpiperazin-1-yl)(C₁-C₂)alkyl, imidazolyl, pyrrolidinyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, halo, trifluoro(C₁-C₂)alkyl, trifluoro(C₁-C₂)alkoxy, di(C₁-C₂)alkylamino(C₁-C₂)alkyl(C₁-C₂)alkylamino, pyridinyl, furanyl, hydroxy and tert-butoxycarbonyl (BOC), Z is CH or N, L is CH₂ or NH, and n₁ or n₂ is an integer from 0 to 1.

Preferably, the compound may be represented by Formula 1-1. 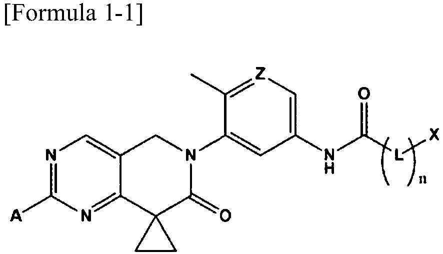 wherein:
X is selected from (substituted or unsubstituted) (C₁-C₄)alkyl, (C₃-C₆)cycloalkyl, (C₄-C₆)cycloalkenyl, phenyl, benzyl, or 5- or 6-membered monocyclic or bicyclic heterocyclic compounds, wherein the substituent is selected from one or more members of the group consisting of (C₁-C₄)alkyl, (C₁-C₄)alkoxy, (C₃-C₆)cycloalkyl, trifluoromethyl, trifluoromethyl(C₁-C₂)alkoxy, halo, hydroxy, imidazolyl, (C₁-C₂)alkylimidazolyl, (C₁-C₂)alkoxyphenyl, morpholino, cyano, oxo (=O), (C₁-C₂)alkoxy(C₁-C₂)alkyl(C₁-C₂)alkylamino, piperazinyl, (C₁-C₂)alkylpiperazinyl, ((C₁-C₂)alkylpiperazin-1-yl)(C₁-C₂)alkyl and di(C₁-C₂)alkylamino(C₁-C₂)alkyl(C₁-C₂)alkylamino, wherein the 5- or 6-membered monocyclic or bicyclic heterocyclic rings are selected from the group consisting of thiophene, furan, pyrazole, oxazole, thiazole, pyridine, pyran, tetrahydropyran, oxazine, thiazine, pyridine, piperazine and benzothiazole,
A is selected from morpholino or -NH-Y, wherein Y is selected from hydrogen, or substituted or unsubstituted group selected from phenyl, benzyl, (C₁-C₄)alkyl, (C₂-C₃)alkenyl, (C₃-C₆)cycloalkyl, pyridinyl, pyrazolyl, tetrahydropyranyl, benzothiazolyl, benzofuranyl, or azetidine, wherein the substituent is selected from one or more members of the group consisting of (C₁-C₂)alkyl, (C₁-C₄)alkoxy, piperazinyl, (C₁-C₂)alkylpiperazinyl, acetylpiperazinyl, morpholino, morpholine-4-carbonyl, (C₁-C₂)alkylpiperazine-l-carbonyl, oxetanyl, ((C₁-C₂)alkylpiperazin-1-yl)(C₁-C₂)alkyl, imidazolyl, pyrrolidinyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, halo, trifluoro(C₁-C₂)alkyl, trifluoro(C₁-C₂)alkoxy, di(C₁-C₂)alkylamino(C₁-C₂)alkyl(C₁-C₂)alkylamino, pyridinyl, furanyl, hydroxy and tert-butoxycarbonyl (tert-butoxycarbonyl, BOC), Z is CH or N, L is CH₂ or NH, and n is an integer from 0 to 1.

More preferably, the compound may be represented by Formula 1-2, 1-3, 1-4, 1-5, or 1-6: 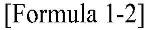 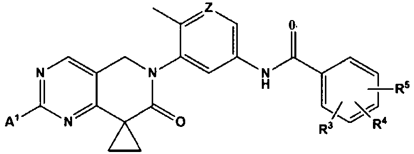 wherein:
R³ to R⁵ may each be the same or different, and are selected from hydrogen, (C₁-C₂)alkyl, (C₁-C₂)alkoxy, trifluoromethyl, halo, hydroxy, (C₁-C₂)alkylimidazolyl, (C₁-C₂)alkoxyphenyl, morpholino, (C₁-C₂)alkoxy(C₁-C₂)alkyl(C₁-C₂)alkylamino, piperazinyl, (C₁-C₂)alkylpiperazinyl, ((C₁-C₂)alkylpiperazin-1-yl)(C₁-C₂)alkyl and di(C₁-C₂)alkylamino(C₁-C₂)alkyl(C₁-C₂)alkylamino;
A¹ is selected from morpholino or -NH-Y¹, wherein Y¹ is selected from hydrogen, (C₃-C₄)cycloalkyl, benzyl, 1H-pyrazolyl, di(C₁-C₂)alkyl-1H-pyrazolyl, (oxetan-3-yl)-1H-pyrazolyl, trifluoro(C₁-C₂)alkyl-1H-pyrazolyl, pyridinyl(C₁-C₂)alkyl, hydroxy(C₁-C₂)alkyl, hydroxy(C₂-C₄)alkenyl, hydroxy(C₃-C₆)cycloalkyl, morpholino(C₃-C₄)alkyl, furanyl(C₁-C₂)alkyl, tert-butoxycarbonyl (BOC) substituted azetidine, tetrahydropyranyl, benzothiazolyl, benzofuranyl or substituted or unsubstituted phenyl or pyridinyl, wherein the substituent is selected from one or more members of the group consisting of (C₁-C₂)alkyl, (C₁-C₂)alkoxy, halo, trifluoro(C₁-C₂)alkoxy, piperazinyl, (C₁-C₂)alkylpiperazinyl, acetylpiperazinyl, morpholino, morpholine-4-carbonyl, (C₁-C₂)alkylpiperazine-l-carbonyl, ((C₁-C₂)alkylpiperazin-1-yl)(C₁-C₂)alkyl, imidazolyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl and di(C₁-C₂)alkylamino(C₁-C₂)alkyl(C₁-C₂)alkylamino. 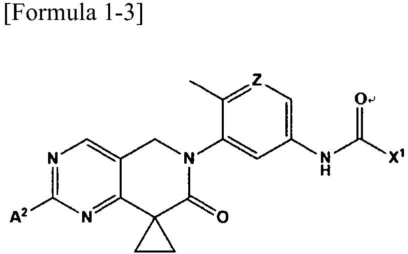
wherein:
   X¹ is selected from (C₃-C₆)cycloalkyl, oxo(C₃-C₆)cycloalkyl, (C₄-C₆)cycloalkenyl, thiophene, (C₁-C₂)alkylthiophene, furan, thiazole, oxazole, benzothiazole, tetrahydropyran, trifluoro(C₁-C₃)alkyl; or pyridinyl substituted with a substituent selected from the group consisting of (C₁-C₂)alkyl, (C₁-C₂)alkoxy, trifluoromethyl, halo and hydroxy;
   A² is selected from morpholino or -NH-Y², wherein Y² is selected from one or more members of the group consisting of hydrogen, benzyl, tetrahydropyranyl, benzothiazolyl, benzofuranyl, (C₁-C₂)alkylpyridinyl, pyridinyl(C₁-C₂)alkyl, hydroxy(C₁-C₂)alkyl, hydroxy(C₂-C₄)alkenyl, hydroxy(C₃-C₆)cycloalkyl, morpholino(C₃-C₄)alkyl, furanyl(C₁-C₂)alkyl, ((C₁-C₂)alkylpiperazin-1-yl)piperidinyl-trifluoro(C₁-C₂)alkoxypyridinyl, and tert-butoxycarbonyl (BOC) substituted azetidine. 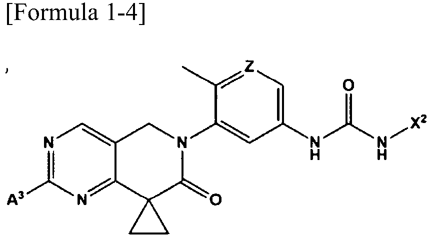
   wherein:
      X² is selected from (C₃-C₆)cycloalkyl, (C₃-C₆)cycloalkyl(C₁-C₂)alkyl, benzyl, or substituted or unsubstituted phenyl, wherein the substituent is selected from one or more members of the group consisting of (C₁-C₂)alkyl, (C₁-C₂)alkoxy, halo, trifluoro(C₁-C₂)alkyl, trifluoro(C₁-C₂)alkoxy and cyano, A³ is selected from morpholino or -NH-Y³, and Y³ may be one or more selected from hydrogen or (C₁-C₂)alkylpyridinyl. 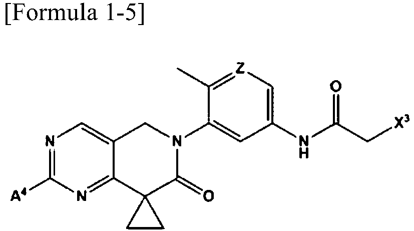
      wherein:
         X³ is selected from substituted or unsubstituted phenyl, wherein the substituent is selected from one or more members of the group consisting of(C₁-C₂) alkyl, (C₁-C₂) alkoxy and halo;
         A⁴ is selected from morpholino or -NH-Y⁴, wherein Y⁴ is selected from one or more members of the group consisting of hydrogen, benzyl, tetrahydropyranyl, benzothiazolyl, benzofuranyl, morpholino(C₃-C₄)alkyl, furanyl(C₁-C₂)alkyl or pyridinyl(C₁-C₂)alkyl. 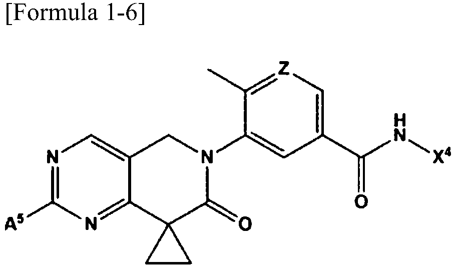
         wherein:
            X⁴ is selected from substituted or unsubstituted phenyl, wherein the substituent is one or more selected from the group consisting of (C₁-C₂) alkyl, (C₁-C₂) alkoxy, halo and trifluoromethyl,
            A⁵ is selected from morpholino or -NH-Y⁵, wherein Y⁵ may be hydrogen, or one or more selected from (C₁-C₂) alkylpyridinyl.

In one Example, the compound may be selected from the group consisting of N-(3-(2'-((1,3-dimethyl-1H-pyrazol-5-yl)amino)-7'-oxo-5'H-spiro[cyclopropane-1,8'-pyrido[4,3-d]pyrimidin]-6'(7'H)-yl)-4-methylphenyl)-3-(trifluoromethyl)benzamide (compound 1), N-(3-(2'-amino-7'-oxo-5'H-spiro[cyclopropane-1,8'-pyrido[4,3-d]pyrimidine]-6'(7'H)-yl)-4-methylphenyl)-3-(trifluoromethyl)benzamide (compound 2), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (compound 3), N-(3-(2'-amino-7'-oxo-5'H-spiro[cyclopropane-1,8'-pyrido[4,3-d]pyrimidine]-6'(7'H)-yl)-4-methylphenyl)-4-fluoro-3-(trifluoromethyl)benzamide (compound 4), N-(3-(2'-amino-7'-oxo-5'H-spiro[cyclopropane-1,8'-pyrido[4,3-d]pyrimidine]-6'(7'H)-yl)-4-methylphenyl)-4-chloro-3-(trifluoromethyl)benzamide (compound 5), N-(3-(2'-amino-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide (compound 6), N-(3-(2'-amino-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamide (compound 7), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide (compound 8), N-(3-(2'-amino-7'-oxo-5'H-spiro[cyclopropane-1,8'-pyrido[4,3-d]pyrimidine]-6'(7'H)-yl)-4-methylphenyl)-3,4-difluorobenzamide (compound 9), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,4-difluorobenzamide (compound 10), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,5-difluorobenzamide (compound 11), N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-methyl-3-(trifluoromethyl)benzamide (compound 12), 3-bromo-N-(3-(2'-(cyclopropylamino)-7'-oxo-5'H-spiro[cyclopropane-1,8'-pyrido[4,3-d]pyrimidine]-6'(7'H)-yl)-4-methylphenyl)-5-(trifluoromethyl)benzamide (compound 13), N-(3-(2'-amino-7'-oxo-5'H-spiro[cyclopropane-1,8'-pyrido[4,3-d]pyrimidine]-6'(7'H)-yl)-4-methylphenyl)-2,4-difluorobenzamide (compound 14), N-(3-(2'-amino-7'-oxo-5'H-spiro[cyclopropane-1,8'-pyrido[4,3-d]pyrimidine]-6'(7'H)-yl)-4-methylphenyl)-3,5-difluorobenzamide (compound 15), N-(3-(2'-amino-7'-oxo-5'H-spiro[cyclopropane-1,8'-pyrido[4,3-d]pyrimidine]-6'(7'H)-yl)-4-methylphenyl)-3-methoxybenzamide (compound 16), N-(3-(2'-amino-7'-oxo-5'H-spiro[cyclopropane-1,8'-pyrido[4,3-d]pyrimidine]-6'(7'H)-yl)-4-methylphenyl)-3-chloro-2-fluoro-5-(trifluoromethyl)benzamide (compound 17), N-(3-(2'-amino-7'-oxo-5'H-spiro[cyclopropane-1,8'-pyrido[4,3-d]pyrimidine]-6'(7'H)-yl)-4-methylphenyl)-2-fluoro-5-(trifluoromethyl)benzamide (compound 18), N-(3-(2'-amino-7'-oxo-5'H-spiro[cyclopropane-1,8'-pyrido[4,3-d]pyrimidine]-6'(7'H)-yl)-4-methylphenyl)-3-methoxy-5-(trifluoromethyl)benzamide (compound 19), N-(3-(2'-amino-7'-oxo-5'H-spiro[cyclopropane-1,8'-pyrido[4,3-d]pyrimidine]-6'(7'H)-yl)-4-methylphenyl)-4'-methoxy-5-(trifluoromethyl)-[1,1'-biphenyl]-3-carboxamide (compound 20), N-(3-(2'-amino-7'-oxo-5'H-spiro[cyclopropane-1,8'-pyrido[4,3-d]pyrimidine]-6'(7'H)-yl)-4-methylphenyl)-6-(trifluoromethyl)picolinamide (compound 21), N-(3-(2'-amino-7'-oxo-5'H-spiro[cyclopropane-1,8'-pyrido[4,3-d]pyrimidine]-6'(7'H)-yl)-4-methylphenyl)-2-(trifluoromethyl)isonicotinamide (compound 22), N-(3-(2'-amino-7'-oxo-5'H-spiro[cyclopropane-1,8'-pyrido[4,3-d]pyrimidine]-6'(7'H)-yl)-4-methylphenyl)-3-hydroxy-5-(trifluoromethyl)benzamide (compound 23), N-(3-(2'-amino-7'-oxo-5'H-spiro[cyclopropane-1,8'-pyrido[4,3-d]pyrimidine]-6'(7'H)-yl)-4-methylphenyl)-5-(trifluoromethyl)nicotinamide (compound 24), N-(3-(2'-amino-7'-oxo-5'H-spiro[cyclopropane-1,8'-pyrido[4,3-d]pyrimidine]-6'(7'H)-yl)-4-methylphenyl)-2-chloro-5-(trifluoromethyl)benzamide (compound 25), N-(3-(2'-amino-7'-oxo-5'H-spiro[cyclopropane-1,8'-pyrido[4,3-d]pyrimidine]-6'(7'H)-yl)-4-methylphenyl)-3-chloro-5-(trifluoromethyl)benzamide (compound 26), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamide (compound 27), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide (compound 28), N-(3-(2'-amino-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide (compound 29), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide (compound 30), N-(3-(2'-amino-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide (compound 31), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide (compound 32), N-(3-(2'-amino-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide (compound 33), N-(3-(2'-amino-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide (compound 34), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide (compound 35), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide (compound 36), N-(3-(2'-amino-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide (compound 37), N-(4-methyl-3-(2'-((4-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 38), N-(3-(2'-((4-(4-acetylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (compound 39), N-(4-methyl-3-(2'-((4-morpholinophenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 40), N-(4-methyl-3-(2'-((4-(4-methylpiperazine-1-carbonyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 41), N-(3-(2'-((4-(4-ethylpiperazin-1-yl)-2-methoxyphenyl)amino)-1'-oxo-5'H-spiro[cyclopropane-1,8'-pyrido[4,3-d]pyrimidine]-6'(7'H)-yl)-4-methylphenyl)-3-(trifluoromethyl)benzamide (compound 42), N-(4-methyl-3-(2'-((4-((4-methylpiperazin-1-yl)methyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 43), N-(3-(2'-((4-(1H-imidazol-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 44), N-(4-methy1-3-(1'-oxo-2'-((4-(piperazin-1-yl)phenyl)amino)-5'H-spiro[cyclopropane-1,8'-pyrido[4,3-d]pyrimidine]-6'(7'H)-yl)phenyl)-3-(trifluoromethyl)benzamide (Compound 45), (S)-N-(3-(2'-((4-(3-(dimethylamino)pyrrolidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 46), N-(4-methyl-3-(7'-oxo-2'-((4-(piperidin-4-yl)phenyl)amino)-5'H-spiro[cyclopropane-1,8'-pyrido[4,3-d]pyrimidine]-6'(7'H)-yl)phenyl)-3-(trifluoromethyl)benzamide (Compound 47), N-(4-methyl-3-(2'-((3-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 48), N-(3-(2'-((4-(1-acetylpiperidin-4-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 49), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-4-((4-methylpiperazin-1-yl)methyl)-3-(trifluoromethyl)benzamide (Compound 50), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 51), N-(4-methyl-3-(2'-((1-(oxetan-3-yl)-1H-pyrazol-4-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 52), N-(3-(2'-((2-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 53), N-(3-(2'-((2-methoxy-4-(morpholine-4-carbonyl)phenyl)amino)-7'-oxo-5'H-spiro [cyclopropane-1,8'-pyrido [4,3-d]pyrimidine]-6'(7'H)-yl)-4-methylphenyl)-3-(trifluoromethyl)benzamide (Compound 54), N-(3-(2'-((3-methoxy-4-(4-methylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 55), N-(3-(2'-((3-methoxy-4-morpholinopheny1)amino)-1'-oxo-5'H-spiro[cyclopropane-1,8'-pyrido[4,3-d]pyrimidine]-6'(7'H)-yl)-4-methylphenyl)-3-(trifluoromethyl)benzamide (Compound 56), N-(3-(2'-((4-(4-ethylpiperazin-1-yl)-3-fluorophenyl)amino)-1'-oxo-5'H-spiro[cyclopropane-1,8'-pyrido[4,3-d]pyrimidine]-6'(7'H)-yl)-4-methylphenyl)-3-(trifluoromethyl)benzamide (Compound 57), N-(3-(2'-((6-(4-ethylpiperazin-1-yl)-2-methoxypyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 58), N-(4-methyl-3-(7'-oxo-2'-(phenylamino)-5'H-spiro[cyclopropane-1,8'-pyrido[4,3-d]pyrimidine]-6'(7'H)-yl)phenyl)-3-(trifluoromethyl)benzamide (Compound 59), N-(3-(2'-((4-(4-(dimethylamino)piperidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 60), N-(3-(2'-amino-7'-oxo-5'H-spiro[cyclopropane-1,8'-pyrido[4,3-d]pyrimidine]-6'(7'H)-yl)-4-methylphenyl)-4-((4-methylpiperazin-1-yl)methyl)-3-(trifluoromethyl)benzamide (Compound 61), N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide (Compound 62), N-(3-(2'-((4-((2-(dimethylamino)ethyl)(methyl)amino)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 63), N-(4-methyl-3-(7'-oxo-2'-((1-(2,2,2-trifluoroethyl)-1H-pyrazol-4-yl)amino)-5'H-spiro[cyclopropane-1,8'-pyrido[4,3-d]pyrimidine]-6'(7'H)-yl)phenyl)-3-(trifluoromethyl)benzamide (Compound 64), N-(3-(2'-amino-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide (Compound 65), N-(3-(2'-((4-(4-acetylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 66), N-(3-(2'-((3-methoxy-4-(piperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 67), N-(3-(2'-((1,3-dimethyl-1H-pyrazol-5-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 68), N-(3-(2'-((2-methoxy-6-(4-methylpiperazin-1-yl)pyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 69), N-(3-(2'-(hydroxyamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 70), 1-(4-fluorophenyl)-3-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)urea (Compound 71), 4-chloro-N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)benzamide (Compound 72), N-(4-methyl-3-(2'-((6-methylpyridin-3-yl)amino)-7'-oxo-5'H-spiro[cyclopropane- 1,8'-pyrido[4,3-d]pyrimidine]-6'(7'H)-yl)phenyl)thiophene-2-carboxamide (Compound 73), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)furan-2-carboxamide (Compound 74), N-(3-(2'-amino-7'-oxo-5'H-spiro[cyclopropane-1,8'-pyrido[4,3-d]pyrimidine]-6'(7'H)-yl)-4-methylphenyl)cyclobutanecarboxamide (Compound 75), N-(3-(2'-amino-7'-oxo-5'H-spiro[cyclopropane-1,8'-pyrido[4,3-d]pyrimidine]-6'(7'H)-yl)-4-methylphenyl)-3-oxocyclobutane-1-carboxamide (Compound 76), N-(3-(2'-amino-7'-oxo-5'H-spiro[cyclopropane-1,8'-pyrido[4,3-d]pyrimidine]-6'(7'H)-yl)-4-methylphenyl)cyclohex-1-ene-1-carboxamide (Compound 77), N-(3-(2'-amino-7'-oxo-5'H-spiro[cyclopropane-1,8'-pyrido[4,3-d]pyrimidine]-6'(7'H)-yl)-4-methylphenyl)-4-methylthiophene-2-carboxamide (Compound 78), N-(3-(2'-amino-7'-oxo-5'H-spiro[cyclopropane-1,8'-pyrido[4,3-d]pyrimidine]-6'(7'H)-yl)-4-methylphenyl)isoxazole-5-carboxamide (Compound 79), N-(3-(2'-amino-7'-oxo-5'H-spiro[cyclopropane-1,8'-pyrido[4,3-d]pyrimidine]-6'(7'H)-yl)-4-methylphenyl)benzo[d]thiazole-2-carboxamide (Compound 80), N-(3-(2'-amino-7'-oxo-5'H-spiro[cyclopropane-1,8'-pyrido[4,3-d]pyrimidine]-6'(7'H)-yl)-4-methylphenyl)-4,4,4-trifluorobutanamide (Compound 81), N-(3-(2'-amino-7'-oxo-5'H-spiro[cyclopropane-1,8'-pyrido[4,3-d]pyrimidine]-6'(7'H)-yl)-4-methylphenyl)thiazole-5-carboxamide (Compound 82), N-(3-(2-amino-7'-oxo-5'H-spiro[cyclopropane-1,8'-pyrido[4,3-d]pyrimidine]-6'(7'H)-yl)-4-methylphenyl)cyclopentanecarboxamide (Compound 83), N-(3-(2-amino-7'-oxo-5'H-spiro[cyclopropane-1,8'-pyrido[4,3-d]pyrimidine]-6'(7'H)-yl)-4-methylphenyl)tetrahydro-2H-pyran-4-carboxamide (Compound 84), N-(3-(2'-(benzylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 85), N-(4-methyl-3-(2'-morpholino-7'-oxo-5'H-spiro[cyclopropane-1,8'-pyrido[4,3-d]pyrimidine]-6'(7'H)-yl)phenyl)-3-(trifluoromethyl)benzamide (Compound 86), N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-3-(trifluoromethyl)benzamide (Compound 87), N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-3-(trifluoromethyl)benzamide (Compound 88), N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 89), N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 90), N-(3-(2'-(benzylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 91), N-(4-methyl-3-(2'-morpholino-7'-oxo-5'H-spiro[cyclopropane-1,8'-pyrido[4,3-d]pyrimidine]-6'(7'H)-yl)phenyl)-5-(trifluoromethyl)nicotinamide (Compound 92), N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-5-(trifluoromethyl)nicotinamide (Compound 93), N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-5-(trifluoromethyl)nicotinamide (Compound 94), N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-5-(trifluoromethyl)nicotinamide (Compound 95), N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 96), N-(3-(2'-(benzylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide (Compound 97), 2-(3,5-difluorophenyl)-N-(4-methyl-3-(2'-morpholino-7'-oxo-5'H-spiro[cyclopropane-1,8'-pyrido[4,3-d]pyrimidine]-6'(7'H)-yl)phenyl)acetamide (Compound 98), 2-(3,5-difluorophenyl)-N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)acetamide (Compound 99), 2-(3,5-difluorophenyl)-N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)acetamide (Compound 100), 2-(3,5-difluorophenyl)-N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)acetamide (Compound 101), 2-(3,5-difluorophenyl)-N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)acetamide (Compound 102), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 103), N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 104), N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 105), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 106), N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 107), N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-5-(trifluoromethyl)nicotinamide (Compound 108), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide (Compound 109), 2-(3,5-difluorophenyl)-N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)acetamide (Compound 110), 2-(3,5-difluorophenyl)-N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)acetamide (Compound 111), 1-(3-(2'-amino-7'-oxo-5'H-spiro[cyclopropane-1,8'-pyrido[4,3-d]pyrimidine]-6'(7'H)-yl)-4-methylphenyl)-3-cyclopropylurea (Compound 112), 1-(3-(2'-amino-7'-oxo-5'H-spiro[cyclopropane-1,8'-pyrido[4,3-d]pyrimidine]-6'(7'H)-yl)-4-methylphenyl)-3-cyclobutylurea (Compound 113), 1-(3-(2'-amino-7'-oxo-5'H-spiro[cyclopropane-1,8'-pyrido[4,3-d]pyrimidine]-6'(7'H)-yl)-4-methylphenyl)-3-cyclopentylurea (Compound 114), 1-(3-(2'-amino-7'-oxo-5'H-spiro[cyclopropane-1,8'-pyrido[4,3-d]pyrimidine]-6'(7'H)-yl)-4-methylphenyl)-3-(cyclopropylmethyl)urea (Compound 115), 1-(3-(2'-amino-7'-oxo-5'H-spiro[cyclopropane-1,8'-pyrido[4,3-d]pyrimidine]-6'(7'H)-yl)-4-methylphenyl)-3-(cyclohexylmethyl)urea (Compound 116), 1-(3-(2'-amino-7'-oxo-5'H-spiro[cyclopropane-1,8'-pyrido[4,3-d]pyrimidine]-6'(7'H)-yl)-4-methylphenyl)-3-benzylurea (Compound 117), 1-(3-(2'-amino-7'-oxo-5'H-spiro[cyclopropane-1,8'-pyrido[4,3-d]pyrimidine]-6'(7'H)-yl)-4-methylphenyl)-3-(4-(trifluoromethoxy)phenyl)urea (Compound 118), 1-(3-(2'-amino-7'-oxo-5'H-spiro[cyclopropane-1,8'-pyrido[4,3-d]pyrimidine]-6'(7'H)-yl)-4-methylphenyl)-3-(3-cyanophenyl)urea (Compound 119), 1-(3-(2'-amino-7'-oxo-5'H-spiro[cyclopropane-1,8'-pyrido[4,3-d]pyrimidine]-6'(7'H)-yl)-4-methylphenyl)-3-(3,4-difluorophenyl)urea (Compound 120), 1-(3-(2'-amino-7'-oxo-5'H-spiro[cyclopropane-1,8'-pyrido[4,3-d]pyrimidine]-6'(7'H)-yl)-4-methylphenyl)-3-(2-methoxyphenyl)urea (Compound 121), 1-(3-(2'-amino-7'-oxo-5'H-spiro[cyclopropane-1,8'-pyrido[4,3-d]pyrimidine]-6'(7'H)-yl)-4-methylphenyl)-3-(3-(trifluoromethyl)phenyl)urea (Compound 122), N-(5-(2'-amino-7'-oxo-5'H-spiro[cyclopropane-1,8'-pyrido[4,3-d]pyrimidine]-6'(7'H)-yl)-6-methylpyridin-3-yl)-3-(trifluoromethyl)benzamide (Compound 123), N-methyl-N-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(trifluoromethyl)benzamide (Compound 124), 1-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(m-tolyl)urea (Compound 125), N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 126), (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 127), N-(3-(2'-(((1r,3r)-3-hydroxycyclobutyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 128),
tert-butyl 3-((6'-(2-methyl-5-(3-(trifluoromethyl)benzamido)phenyl)-7'-oxo-6',7'-dihydro-5'H-spiro[cyclopropane-1,8'-pyrido[4,3-d]pyrimidin]-2'-yl)amino)azetidine-1-carboxylate (Compound 129), N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 130), (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 131), N-(3-(2'-(((1r,3r)-3-hydroxycyclobutyl)amino)-1'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 132),
tert-butyl 3-((6'-(2-methyl-5-(5-(trifluoromethyl)nicotinamido)phenyl)-7'-oxo-6',7'-dihydro-5'H-spiro[cyclopropane-1,8'-pyrido[4,3-d]pyrimidin]-2'-yl)amino)azetidine-1-carboxylate (Compound 133) and 3-(2'-amino-7'-oxo-5'H-spiro[cyclopropane-1,8'-pyrido[4,3-d]pyrimidine]-6'(7'H)-yl)-4-methyl-N-(3-(trifluoromethyl)phenyl)benzamide (Compound 134).

The compounds according to the present invention exhibit inhibitory activity against ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19, and ZAK/MLTK, wherein the compounds are capable of inhibiting one or more protein kinases selected from the group consisting of the foregoing.

### [Definition]

In this specification, the term "stereoisomer" refers to two compounds having the same molecular formula but different spatial arrangements of their atoms, and stereoisomers may be enantiomers which are non-superimposable mirror images of each other, or diastereomers which are not mirror images of each other (for example, cis/trans isomers and conformational isomers). These may include R and S configurations for each asymmetric center, as well as Z and E double bond isomers, and Z and E conformational isomers. Compositions containing not only single stereochemical isomers but also enantiomers, diastereomers, and geometric (or conformational) mixtures of the present compounds fall within the scope of the present invention.

In this specification, the term "pharmaceutically acceptable salt" refers to a pharmaceutically acceptable acid addition salt or base addition salt. The base addition salts may include organic or inorganic base salts such as sodium, potassium, calcium, lithium, magnesium, or cesium salts, as well as aminium, ammonium, triethylammonium, and pyridinium salts, and the like, but are not limited thereto.

In addition, acid addition salts formed by free acids may be prepared as pharmaceutically acceptable acid salts. The free acids may include inorganic or organic acids. Examples of inorganic acid include hydrochloric acid, bromic acid, sulfuric acid, sulfurous acid, phosphoric acid, and the like. Examples of organic acid include citric acid, acetic acid, maleic acid, fumaric acid, gluconic acid, methanesulfonic acid, benzenesulfonic acid, camphorsulfonic acid, oxalic acid, malonic acid, glutaric acid, acetic acid, gluconic acid, succinic acid, tartaric acid, 4-toluenesulfonic acid, galacturonic acid, embonic acid, glutamic acid, citric acid, aspartic acid, and the like. Preferably, hydrochloric acid is used as the inorganic acid, while methanesulfonic acid is used as an organic acid.

Furthermore, the composition according to the present invention may further include pharmaceutically acceptable salts, as well as all salts, hydrates or solvates that may be prepared by conventional methods.

The addition salts according to the present invention may be prepared by conventional methods, for example, by dissolving the compound in a water-miscible organic solvent such as acetone, methanol, ethanol, or acetonitrile, and then either by adding an excess of an organic base or adding by an aqueous solution of an inorganic base, followed by precipitation or crystallization. Alternatively, the addition salts may be obtained either by evaporating the solvent or excess base from the reaction mixture followed by drying, or by filtration of the precipitated salts.

The term "precursor" as used herein refers to a compound capable of providing (directly or indirectly) the compound of the present invention when administered to a subject or patient. Examples of precursor include esterified or hydroxylated compounds in which the ester or hydroxyl group is cleaved in vivo, for example, within the intestine, to produce a compound according to Formula 1.

In this specification, the term "solvate" refers to an adduct of inert solvent molecules with a compound, formed due to intermolecular forces. When the compound is treated with water, the solvate is referred to as a hydrate, for example, the solvate may be a monohydrate or dihydrate or an alkoxide.

Furthermore, the present invention provides a pharmaceutical composition for the treatment or prevention of protein kinase-related diseases, which comprises the compound of the present invention.

Furthermore, the present invention provides a method for treating protein kinase-related diseases, comprising the step of administering the compound of the present invention to a subject suffering from a protein kinase-related disease.

The protein kinase may be one or more selected from the group consisting of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19 and ZAK/MLTK.

Preferably, the compound exhibits an inhibition rate of 60% or higher against a protein kinase enzyme when tested at a single concentration of 1 µM.

The protein kinase-related disease may be a cancer which may be selected from the group consisting of prostate cancer, endometrial cancer, bladder cancer, gastric cancer, lung cancer, liver cancer, colorectal cancer, small intestinal cancer, pancreatic cancer, brain cancer, bone cancer, melanoma, breast cancer, sclerosing adenoma, head and neck cancer, esophageal cancer, thyroid cancer, parathyroid cancer, kidney cancer, sarcoma, urethral cancer, leukemia, multiple myeloma, hematologic cancer, lymphoma, and fibroadenoma, but is not limited thereto.

The pharmaceutical composition may be administered in combination therapy with one or more anticancer agents selected from the group consisting of cytotoxic anticancer agents, targeted anticancer agents, immunooncology agents, and metabolic anticancer agents.

The pharmaceutical composition may be provided in one or more dosage forms selected from the group consisting of gels, emulsions, injections, powders, granules, aerosols, pastes, transdermal absorption preparations, and patches according to conventional methods, but is not limited thereto.

In another embodiment of the present invention, the pharmaceutical composition may further comprise one or more additives selected from the group consisting of suitable carriers, excipients, disintegrants, sweeteners, coating agents, swelling agents, lubricants, glidants, flavoring agents, antioxidants, buffers, bacteriostatic agents, diluents, dispersants, surfactants, binders, and lubricants commonly used in the manufacture of pharmaceutical compositions.

Specifically, carriers, excipients and diluents may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil. Solid preparations for oral administration include tablets, pills, powders, granules, capsules, and the like, and such solid preparations may be prepared by mixing the composition with at least one excipient, for example, starch, calcium carbonate, sucrose or lactose, gelatin, and the like. In addition to simple excipients, lubricants such as magnesium stearate and talc may also be used. Liquid preparations for oral administration include suspensions, solutions, emulsions, syrups, and the like, and in addition to commonly used simple diluents such as water and liquid paraffin, various excipients may be included, for example, wetting agents, sweeteners, flavoring agents, preservatives, and the like. Preparations for parenteral administration include sterile aqueous solutions, non-aqueous solvents, suspensions, emulsions, freeze-dried preparations, suppositories, and the like. Non-aqueous solvents and suspensions may include propylene glycol, polyethylene glycol, vegetable oils such as olive oil, injectable esters such as ethyl oleate, and the like. Suppository bases may include witepsol, macrogol, tween 61, cacao butter, lauric acid, glycerogelatin, and the like.

The pharmaceutical composition may be administered to a subject in a conventional manner through intravenous, intraarterial, intraperitoneal, intramuscular, intrasternal, transdermal, intranasal, inhalation, topical, rectal, oral, intraocular, or intradermal routes.

The preferred dosage of the compound may vary depending on the condition and body weight of the subject, the type and severity of the disease, the drug form, the route of administration and duration, and may be appropriately selected by those skilled in the art. According to one embodiment of the present invention, the daily dosage may be, but is not limited to, 0.01 to 200 mg/kg, specifically 0.1 to 200 mg/kg, more specifically 0.1 to 100 mg/kg. Administration may be once daily or divided into multiple doses, and the scope of the present invention is not limited thereby.

In the present invention, the "subject" may be a mammal including humans but is not limited to these examples.

In addition, the present invention provides healthy functional food for improving or preventing protein kinase-related diseases, which comprises the compound of the present invention.

The protein kinase may be one or more selected from the group consisting of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19, ZAK/MLTK.

The protein kinase-related disease may be a cancer, and the cancer may be selected from the group consisting of prostate cancer, endometrial cancer, bladder cancer, gastric cancer, lung cancer, liver cancer, colorectal cancer, small intestinal cancer, pancreatic cancer, brain cancer, bone cancer, melanoma, breast cancer, sclerosing adenoma, head and neck cancer, esophageal cancer, thyroid cancer, parathyroid cancer, renal cancer, sarcoma, urethral cancer, leukemia, multiple myeloma, hematologic cancer, lymphoma, and fibroadenoma, but is not limited thereto.

In this specification, the term "health functional food" as used herein refers to food manufactured and processed using raw materials or ingredients having functionality beneficial to the human body in accordance with the Health Functional Food Act, and the term "functionality" refers to the intake intended for regulating nutrients for the structure and function of the human body or obtaining beneficial effects for health such as physiological actions.

Health functional food may contain conventional food additives. Unless otherwise specified, the suitability as a "food additive" is determined according to the specifications and standards for the relevant items, in accordance with the general rules and general test methods of the Food Additive Codex as approved by the Ministry of Food and Drug Safety.

Items listed in the "Food Additive Codex" include, for example, chemical synthetic compounds such as ketones, glycine, potassium citrate, nicotinic acid, and cinnamic acid; natural additives such as persimmon color, licorice extract, crystalline cellulose, sorghum color, and guar gum; and mixed preparations such as sodium L-glutamate preparations, noodle-added alkali preparations, preservative preparations, and tar color preparations.

The effective dose of the active ingredient contained in the health functional food may be used in accordance with the effective dose of the therapeutic agent; however, in the case of long-term intake for health and hygiene purposes or for health regulation purposes, it may be below the above range, and it is certain that the active ingredient may be used in amounts above the above range because there are no safety concerns.

There are no particular restrictions on the types of health functional foods, and examples include meat, sausages, bread, chocolate, candies, snacks, confectionery, pizza, ramen, other noodles, gums, dairy products including ice cream, various soups, beverages, tea, drinks, alcoholic beverages, and vitamin complexes.

### [Examples]

Hereinafter, the present invention will be described in more detail by way of embodiments. These embodiments are provided merely to illustrate the present invention more specifically, and it will be apparent to those skilled in the art that the scope of the present invention is not limited thereto without departing from the spirit and scope of the present invention.

### <Example 1> Synthetic Route A for Compound 51

Compound 51 was synthesized according to synthetic route A, as shown in Scheme 1, and other compounds were similarly synthesized by the same method. The synthetic method is described in detail with reference to compound 51.

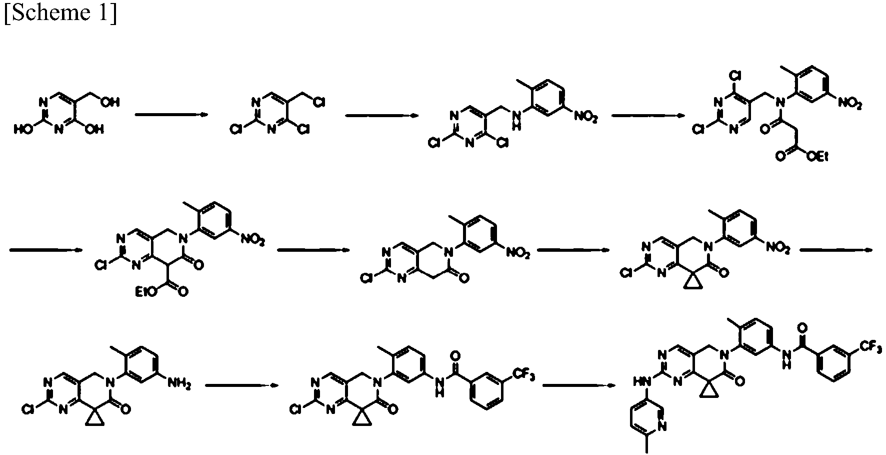

### 1-1) Step 1

5-(Hydroxymethyl)pyrimidine-2,4-diol (8.0 g, 56.32 mmol) and phosphorus oxychloride (POCl₃) (26.3 mL, 281.47 mmol) were added to toluene (180 mL), and nitrogen gas was bubbled through the mixture for 5 minutes. The reaction vessel was cooled in an ice-water bath (0 °C), and N,N-diisopropylethylamine (29.4 mL, 168.88 mmol) was added dropwise . The reaction mixture was then refluxed at 120 °C for 2 hours. After completion of the reaction, the mixture was cooled to room temperature and quenched by dropwise addition of water. The resulting mixture was extracted with ethyl acetate, dried over anhydrous sodium sulfate, and concentrated under reduced pressure. The obtained residue was purified by MPLC (medium pressure liquid chromatography/ethyl acetate: hexane = 1:30 (v/v)), and the solvent was removed under reduced pressure to afford 9.3 g of the target compound as a white solid in 84% yield.
MS(m/z): 195.95[M+1]
¹H NMR (400 MHz, CDCl₃) δ 8.66 (s, 1H), 4.64 (s, 2H)

### 1-2) Step 2

The 2,4-dichloro-5-(chloromethyl)pyrimidine (27.8 g, 140.80 mmol) and 5-methyl-2-nitroaniline (27.8 g, 183.0 mmol) obtained in step 1 were dissolved in acetone (300 mL). Sodium iodide (27.4 g, 183.0 mmol) and potassium carbonate (38.9 g, 281.6 mmol) were then added, and the mixture was heated under reflux at 50 °C for 3 days. After completion of the reaction, the mixture was cooled to room temperature, filtered through a Celite pad, and concentrated under reduced pressure using a rotary evaporator. The residue was diluted with ethyl acetate and water, and aqueous layer was extracted with ethyl acetate. The combined organic layers were dried over anhydrous sodium sulfate and concentrated. The obtained residue was purified by MPLC (ethyl acetate: hexane = 1:20 (v/v)), and the solvent was removed under reduced pressure to afford 34.8 g of the target compound as a yellow solid in 78% yield.
MS(m/z): 313.0[M+1]
¹H NMR (400 MHz, CDCl₃) δ 8.50 (s, 1H), 7.61 (dd, J = 8.2, 2.2 Hz, 1H), 7.27 (d, J = 2.1 Hz, 1H), 7.23 (d, J = 8.2 Hz, 1H), 4.58 (s, 2H), 4.32 (s, 1H), 2.29 (s, 3H)

### 1-3) Step 3

The N-((2,4-dichloropyrimidine-5-yl)methyl)-2-methyl-5-nitroaniline (34.8 g, 111.1 mmol) obtained in step 2 was dissolved in anhydrous tetrahydrofuran (300 mL), and nitrogen gas was bubbled through the mixture for 5 minutes to remove dissolved gases from the mixture. The reaction vessel was then cooed in an ice-water bath (0 °C), and 60% sodium hydride (5.7 g, 144.4 mmol) was added portionwise, followed by stirring at room temperature for 30 minutes. The reaction vessel was cooled in an ice-water bath (0 °C), and ethylmalonyl chloride (20.9 mL, 166.7 mmol) was added dropwise. The reaction mixture was stirred at room temperature for 16 hours. After completion of the reaction, the mixture was cooled to 0 °C and quenched with saturated ammonium chloride solution. The remaining organic solvent was removed under reduced pressure using a rotary evaporator, and the residue was diluted with water and extracted with dichloromethane. The combined organic layers were dried over anhydrous sodium sulfate and concentrated. The obtained residue was purified by MPLC (ethyl acetate: dichloromethane = 20:1 (v/v)), and the solvent was removed under reduced pressure to obtain 43.1 g of the target compound as a white solid in 90% yield.
MS(m/z): 426.9[M+1]
¹H NMR (400 MHz, CDCl₃) δ 8.91 (s, 1H), 8.21 (dd, J = 8.5, 2.3 Hz, 1H), 7.93 (d, J = 2.3 Hz, 1H), 7.56 (d, J = 8.5 Hz, 1H), 5.32 (d, J = 15.4 Hz, 1H), 4.53 (d, J = 15.4 Hz, 1H), 4.14 (q, J = 7.1 Hz, 2H), 3.15 (dd, J = 37.9, 15.7 Hz, 2H), 2.37 (s, 3H), 1.23 (t, J = 7.2 Hz, 3H)

### 1-4) Step 4

The 3-(((2,4-dichloropyrimidin-5-yl)methyl)(2-methyl-5-nitrophenyl)amino)-3-oxopropanoate (39.2 g, 91.75 mmol) obtained in step 3 above was dissolved in dimethyl sulfoxide (500 mL), and nitrogen gas was bubbled through the mixture for 5 minutes. The reaction vessel was cooled in an ice-water bath (0 °C), and cesium carbonate (44.8 g, 137.6 mmol) was added portionwise. The reaction mixture was stirred at room temperature for 2 hours. After completion of the reaction, the mixture was added dropwise to 1N aqueous hydrochloric acid solution (500 mL) at 0 °C and stirred slowly for 15 minutes. The yellow solid was filtered using a Celite pad and washed with water to remove remaining organic solvent. The obtained solid was dried to afford 35.1 g of the target compound as a yellow solid in 97% yield.
MS(m/z): 391.1[M+1]

### 1-5) Step 5

The ethyl 2-chloro-6-(2-methyl-5-nitrophenyl)-7-oxo-5,6,7,8-tetrahydropyrido[4,3-d]pyrimidine-8-carboxylate (35.1 g, 89.8 mmol) obtained in step 4 above was dissolved in 1,4-dioxane (250 mL). A 4N aqueous hydrochloric acid solution in 1,4-dioxane (222.5 mL, 890.1 mmol) was added, and the mixture was stirred under reflux at 100 °C for 16 hours. After completion of the reaction, the mixture was cooled to room temperature, and the solvent was removed under reduced pressure. The residue was diluted with dichloromethane. Insoluble solids were filtered off by a Celite pad, and the combined filtrate was dried over anhydrous sodium sulfate and concentrated. The obtained residue was purified by MPLC (ethyl acetate: dichloromethane = 3:7 (v/v)), and the solvent was concentrated under reduced pressure to obtain 16.7 g of the target compound as a yellow solid in 58% yield.
MS(m/z): 319.05[M+1]
¹H NMR (400 MHz, CDCl₃) δ 8.53 (s, 1H), 8.18 (dd, J = 8.4, 2.3 Hz, 1H), 8.12 (d, J = 2.2 Hz, 1H), 7.51 (d, J = 8.5 Hz, 1H), 4.97 (d, J = 16.1 Hz, 1H), 4.69 (d, J = 16.1 Hz, 1H), 4.02 (s, 2H), 2.29 (s, 3H)

### 1-6) Step 6

The 2-chloro-6-(2-methyl-5-nitrophenyl)-5,8-dihydropyrido[4,3-d]pyrimidine-7(6H)-one (10.0 g, 31.3 mmol) obtained in step 5 above was dissolved in anhydrous dimethylformamide (150 mL), and nitrogen gas was bubbled through the mixture for 5 minutes. The reaction vessel was cooled in ice-water bath (0 °C), 1-bromo-2-chloroethane (4.6 mL, 37.6 mmol) was added, and cesium carbonate (22.4 g, 60.0 mmol) was added portionwise. The mixture was stirred at room temperature for 16 hours. After completion of the reaction, the mixture was added dropwise to 1N aqueous hydrochloric acid solution (150 mL) at 0 °C and stirred slowly for 15 minutes. The resulting yellow solid was filtered and washed with water to remove remaining organic solvent. The obtained solid was dried to afford 9.23 g of the target compound as a yellow solid in 85% yield.
MS(m/z): 344.9[M+1]
¹H NMR (400 MHz, CDCl₃) δ 8.36 (s, 1H), 8.21 - 8.10 (m, 2H), 7.50 (d, J = 8.1 Hz, 1H), 5.07 (d, J = 16.1 Hz, 1H), 4.77 (d, J = 16.1 Hz, 1H), 2.31 (s, 3H), 2.08 - 1.98 (m, 2H), 1.98 - 1.91 (m, 1H), 1.91 - 1.77 (m, 1H)

### 1-7) Step 7

The 2'-chloro-6'-(2-methyl-5-nitrophenyl)-5',6'-dihydro-7'H-spiro[cyclopropane-1,8'-pyrido[4,3-d]pyrimidine]-7'-one (1.00 g, 0.77 mmol) obtained in step 6 above was dissolved in tetrahydrofuran (10.0 mL), and methanol (5.0 mL) and water (3.0 mL) were added dropwise. At room temperature, iron (0.81 g, 14.50 mmol) and ammonium chloride (0.77 g, 14.50 mmol) were added, and the mixture was stirred under reflux at 80 °C for 2 hours. After completion of the reaction, the mixture was cooled to room temperature and filtered through a Celite pad. The filtrate was neutralized by dropwise addition of saturated aqueous sodium bicarbonate solution and extracted with dichloromethane. The combined organic layers were dried over anhydrous sodium sulfate and concentrated to give 0.87 g of the target compound as a yellow solid in 95% yield.
MS(m/z): 315.1[M+1]
¹H NMR (400 MHz, MeOD) δ 8.46 (s, 1H), 7.05 (d, J = 8.2 Hz, 1H), 6.73 - 6.66 (m, 1H), 6.63 (d, J = 2.3 Hz, 1H), 4.99 (d, J = 16.9 Hz, 1H), 4.81 (d, J = 16.9 Hz, 1H), 2.04 (s, 3H), 1.89 (d, J = 4.5 Hz, 2H), 1.76 (dd, J = 4.9, 3.6 Hz, 2H)

### 1-8) Step 8

The 6'-(5-amino-2-methylphenyl)-2'-chloro-5',6'-dihydro-7'H-spiro[cyclopropane-1,8'-pyrido[4,3-d]pyrimidine]-7'-one (0.87 g, 2.77 mmol) obtained in step 7 above was dissolved in dichloromethane (10.0 mL), then 3-(trifluoromethyl)benzoyl chloride (0.42 mL, 2.77 mmol) and potassium carbonate (0.76 g, 5.55 mmol) were added and the mixture was stirred at room temperature for 1 hour. After completion of the reaction, the mixture was diluted with dichloromethane and extracted with water, and the combined organic layers were dried over anhydrous sodium sulfate and concentrated. The obtained residue was purified by MPLC (ethyl acetate: hexane = 3:7 (v/v)), and the solution was concentrated under reduced pressure to obtain 0.87 g of the target compound as a yellow solid in 64% yield.
MS(m/z): 487.1[M+1]
¹H NMR (400 MHz, MeOD) δ 8.48 (s, 1H), 8.25 (s, 1H), 8.19 (d, J = 7.8 Hz, 1H), 7.89 (d, J = 7.9 Hz, 1H), 7.79 (d, J = 2.1 Hz, 1H), 7.73 (t, J = 7.8 Hz, 1H), 7.59 (dd, J = 8.3, 2.1 Hz, 1H), 7.35 (d, J = 8.3 Hz, 1H), 5.08 (s, 1H), 4.89 (d, J = 16.8 Hz, 1H), 2.19 (s, 3H), 1.91 (t, J = 4.9 Hz, 2H), 1.80 (dt, J = 14.7, 10.5 Hz, 2H)

### 1-9) Step 9

N-(3-(2'-chloro-7'-oxo-5'H-spiro[cyclopropane-1,8'-pyrido[4,3-d]pyrimidine]-6'(7'H)-yl)-4-methylphenyl)-3-(trifluoromethyl)benzamide (0.12 g, 0.24 mmol) obtained in step 8 above was dissolved in 2-butanol (10.0 mL), and potassium carbonate (0.17 g, 1.23 mmol), 6-methylpyridine-3-amine (39 mg, 0.36 mmol), and tris(dibenzylideneacetone)dipalladium(0) (46 mg, 0.050 mmol) were added dropwise at room temperature. The reaction mixture was stirred at 100 °C for 1 hour. After completion of the reaction, the mixture was cooled to room temperature, filtered through a Celite pad, and concentrated under reduced pressure. The obtained residue was purified by flash column chromatography (0-10% dichloromethane: methanol), and the solution was concentrated under reduced pressure to afford 95.0 mg of the target compound as a brown solid (Compound 51) in 71% yield.
MS(m/z): 559.2[M+1]
¹H NMR (400 MHz, DMSO-*d₆*) δ 10.57 (s, 1H), 10.36 (s, 1H), 9.11 (s, 1H), 8.47 (s, 1H), 8.34 (d, J = 8.9 Hz, 1H), 8.31 (s, 1H), 8.27 (d, J = 7.6 Hz, 1H), 7.99 (d, J = 7.6 Hz, 1H), 7.84 - 7.75 (m, 2H), 7.71 (d, J = 7.4 Hz, 1H), 7.63 (dd, J = 8.3, 2.0 Hz, 1H), 7.34 (d, J = 8.4 Hz, 1H), 4.96 (d, J = 16.2 Hz, 1H), 4.78 (d, J = 16.1 Hz, 1H), 2.59 (s, 3H), 2.11 (s, 3H), 1.82 - 1.63 (m, 4H)

### 1-10) Synthesis of other compounds through synthesis pathway A

In step 9 above, instead of 6-methylpyridin-3-amine, pyrazolamine, phenylamine or pyrimidinamine derivatives corresponding to each final synthetic compound were used to synthesize the following compounds through synthesis pathway A:
Compound 1 N-(3-(2'-((1,3-dimethyl-1H-pyrazol-5-yl)amino)-7'-oxo-5'H-spiro[cyclopropane-1,8'-pyrido[4,3-d]pyrimidin]-6'(7'H)-yl)-4-methylphenyl)-3-(trifluoromethyl)benzamide, Compound 38 N-(4-methyl-3-(2'-((4-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide, Compound 39 N-(3-(2'-((4-(4-acetylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide, Compound 40 [N-(4-methyl-3-(2'-((4-morpholinophenyl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 41 [N-(4-methyl-3-(2'-((4-(4-methylpiperazine-1-carbonyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 42 [N-(3-(2'-((4-(4-ethylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 43 [N-(4-methyl-3-(2'-((4-((4-methylpiperazin-1-yl)methyl)phenyl)amino)-1'-oxo-5'H-spiro[cyclopropane-1,8'-pyrido[4,3-d]pyrimidine]-6'(7'H)-yl)phenyl)-3-(trifluoromethyl)benzamide], Compound 44 [N-(3-(2'-((4-(1H-imidazol-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 45 [N-(4-methyl-3-(7'-oxo-2'-((4-(piperazin-1-yl)phenyl)amino)-5'H-spiro[cyclopropane-1,8'-pyrido[4,3-d]pyrimidine]-6'(7'H)-yl)phenyl)-3-(trifluoromethyl)benzamide], Compound 46 [(S)-N-(3-(2'-((4-(3-(dimethylamino)pyrrolidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 47 [N-(4-methyl-3-(1'-oxo-2'-((4-(piperidin-4-yl)phenyl)amino)-5'H-spiro[cyclopropane-1,8'-pyrido[4,3-d]pyrimidine]-6'(7'H)-yl)phenyl)-3-(trifluoromethyl)benzamide], Compound 48 [N-(4-methyl-3-(2'-((3-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 49 [N-(3-(2'-((4-(1-acetylpiperidin-4-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 51 [N-(4-methyl-3-(2'-((6-methylpyridin-3-yl)amino)-7'-oxo-5'H-spiro[cyclopropane-1,8'-pyrido
[4,3-d]pyrimidine]-6'(7'H)-yl)phenyl)-3-(trifluoromethyl)benzamide], Compound 52 [N-(4-methyl-3-(2'-((1-(oxetan-3-yl)-1H-pyrazol-4-yl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 53 [N-(3-(2'-((2-methoxy-4-morpholinophenyl)amino)-1'-oxo-5'H-spiro[cyclopropane-1,8'-pyrido[4,3-d]pyrimidine]-6'(7'H)-yl)-4-methylphenyl)-3-(trifluoromethyl)benzamide], Compound 54 [N-(3-(2'-((2-methoxy-4-(morpholine-4-carbonyl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 55 [N-(3-(2'-((3-methoxy-4-(4-methylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 56 [N-(3-(2'-((3-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 57 [N-(3-(2'-((4-(4-ethylpiperazin-1-yl)-3-fluorophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 58 [N-(3-(2'-((6-(4-ethylpiperazin-1-yl)-2-methoxypyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 59 [N-(4-methyl-3-(7'-oxo0-2'-(phenylamino)-5'H-spiro[cyclopropane-1,8'-pyrido[4,3-d]pyrimidine]-6'(7'H)-yl)phenyl)-3-(trifluoromethyl)benzamide], Compound 60 [N-(3-(2'-((4-(4-(dimethylamino)piperidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 63 [N-(3-(2'-((4-((2-(dimethylamino)ethyl)(methyl)amino)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 64 [N-(4-methyl-3-(7'-oxo-2'-((1-(2,2,2-trifluoroethyl)-1H-pyrazol-4-yl)amino)-5'H-spiro[cyclopropane-1,8'-pyrido[4,3-d]pyrimidine]-6'(7'H)-yl)phenyl)-3-(trifluoromethyl)benzamide], Compound 66 [N-(3-(2'-((4-(4-acetylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 67 [N-(3-(2'-((3-methoxy-4-(piperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 68 [N-(3-(2'-((1,3-dimethyl-1H-pyrazol-5-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 69 [N-(3-(2'-((2-methoxy-6-(4-methylpiperazin-1-yl)pyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 71 [1-(4-fluorophenyl)-3-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)urea], Compound 72 [4-chloro-N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)benzamide], Compound 73 [N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)thiophene-2-carboxamide], Compound 74 [N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)furan-2-carboxamide], Compound 103 [N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 104 [N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 105 [N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 106 [N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 107 [N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide],Compound 108 [N-(4-methyl-3-(2-((6-(4-(4-methylpiperazin-1-yl)piperidin-1-yl)-2-(2,2,2-trifluoroethoxy)pyridin-3-yl)amino)-7-oxo-5H-spiro[cyclopropane-1,8'-pyrido[4,3-d]pyrimidin]-6'(7'H)-yl)phenyl)-5-(trifluoromethyl)nicotinamide], Compound 109 [N-(3-(2'-(benzo[d]thiazol-5-ylamino)-7-oxo-5'H-spiro[cyclopropane-1,8'-pyrido[4,3-d]pyrimidin]-6'(7'H)-yl)-4-methylphenyl)-2-(3,5-difluorophenyl)acetamide], Compound 110 [2-(3,5-difluorophenyl)-N-(3-(2'-((2,3-dihydrobenzofuran-4-yl)amino)-7-oxo-5'H-spiro[cyclopropane-1,8'-pyrido[4,3-d]pyrimidin]-6'(7'H)-yl)-4-methylphenyl)acetamide], and Compound 111 [2-(3,5-difluorophenyl)-N-(4-methyl-3-(2-((6-(4-(4-methylpiperazin-1-yl)piperidin-1-yl)-2-(2,2,2-trifluoroethoxy)pyridin-3-yl)amino)-7'-oxo-5'H-spiro[cyclopropane- 1,8'-pyrido[4,3-d]pyrimidin]-6'(7'H)-yl)phenyl)acetamide].

### <Example 2> Synthesis Route B for Compound 8

Compound 8 was synthesized via synthesis route B as shown in the following Scheme 2, and other compounds were also synthesized in the same manner via synthesis route B. The synthesis method is described in detail based on compound 8.

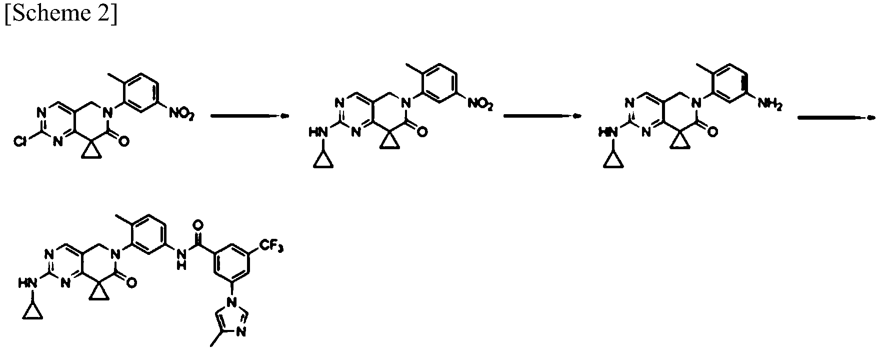

### 2-1) Step 1

The 2-chloro-6-(2-methyl-5-nitrophenyl)-5,8-dihydropyrido[4,3-d]pyrimidine-7(6H)-one (0.2 g, 0.58 mmol) obtained in step 5 of Example 1 was dissolved in anhydrous dimethylacetamide (5.0 mL), cyclopropylamine (1.2 g, 0.38 mmol) and potassium carbonate (70 mg, 0.51 mmol) were added, and the mixture was stirred under reflux at 120 °C for 16 hours. The mixture was concentrated under reduced pressure, and the obtained residue was dissolved in dichloromethane and washed with water. The combined organic layers were dried over anhydrous sodium sulfate and concentrated, and the obtained residue was purified by MPLC (dichloromethane: ethyl acetate = 7:3 (v/v)) and concentrated to obtain 97 mg of the target compound in 46% yield.
MS(m/z): 366.15[M+1]
¹H NMR (400 MHz, CDCl₃) δ 8.19 - 8.05 (m, 3H), 7.47 (d, J = 9.2 Hz, 1H), 4.93 (d, J = 14.8 Hz, 1H), 4.61 (d, J = 14.8 Hz, 1H), 2.75 (dq, J = 6.6, 3.5 Hz, 1H), 2.30 (s, 3H), 1.87 - 1.68 (m, 4H), 0.82 (td, J = 6.7, 5.2 Hz, 2H), 0.59 - 0.48 (m, 2H)

### 2-2) Step 2

The 2'-(cyclopropylamino)-6'-(2-methyl-5-nitrophenyl)-5',6'-dihydro-7'H-spiro[cyclopropane-1,8'-pyrido[4,3-d]pyrimidine]-7'-one (98 mg, 0.22 mmol) obtained in step 1 was dissolved in tetrahydrofuran (2.0 mL), and methanol (1.0 mL) and water (0.5 mL) were added dropwise. Iron (61 mg, 1.10 mmol) and ammonium chloride (59 mg, 1.10 mmol) were added at room temperature, and the mixture was stirred under reflux at 80 °C for 2 hours. After completion of the reaction, the mixture was cooled to room temperature and filtered through a Celite pad. The filtrate was neutralized by dropwise addition of saturated aqueous sodium bicarbonate solution and extracted with dichloromethane. The combined organic layers were dried over anhydrous sodium sulfate and concentrated to give 73 mg of the target compound as a yellow solid in 99% yield.
MS(m/z): 336.0[M+1]
¹H NMR (400 MHz, DMSO-*d₆*) δ 8.15 (s, 1H), 7.32 (s, 1H), 6.92 (d, J = 8.1 Hz, 1H), 6.47 (dd, J = 8.1, 2.1 Hz, 1H), 5.00 (s, 2H), 4.71 (d, J = 15.5 Hz, 1H), 4.53 (d, J = 15.4 Hz, 1H), 2.64 (ddd, J = 10.5, 7.1, 3.6 Hz, 1H), 1.91 (s, 3H), 1.65 - 1.47 (m, 4H), 0.68 - 0.58 (m, 2H), 0.48 - 0.37 (m, 2H)

### 2-3) Step 3

The 6'-(5-amino-2-methylphenyl)-2'-(cyclopropylamino)-5',6'-dihydro-7'H-spiro[cyclopropane-1,8'-pyrido[4,3-d]pyrimidine]-7'-one (50 mg, 0.159 mmol) obtained in step 2 above was dissolved in dichloromethane (2.0 mL), and 3-(4-methyl-1H-imidazol-1-yl)-5-(trifluoromethyl)benzoic acid (60 mg, 0.224 mmol), hexafluorophosphate azabenzotriazole tetramethyl uronium (111 mg, 0.298 mmol), and N,N-diisopropylethylamine (77.8 µL, 0.447 mmol) were added, followed by stirring at room temperature for 16 hours. The mixture was concentrated under reduced pressure, and the resulting residue was dissolved in dichloromethane and washed with water. The combined organic layers were dried over anhydrous sodium sulfate and concentrated, and the resulting residue was purified by HPLC (5:95 → 95:5 (v/v), for 40 min) and concentrated to afford 21 mg of the target compound (Compound 8) in 23% yield.
MS(m/z): 588.20[M+1]
¹H NMR (400 MHz, DMSO-*d₆*) δ 10.65 (s, 1H), 9.50 (s, 1H), 8.57 (s, 1H), 8.42 (s, 2H), 8.15 (d, J = 24.9 Hz, 2H), 7.72 - 7.66 (m, 2H), 7.41 (s, 1H), 7.35 (d, J = 8.9 Hz, 1H), 4.80 (d, J = 15.3 Hz, 1H), 4.65 (d, J = 15.3 Hz, 1H), 2.34 (s, 3H), 2.10 (s, 3H), 1.77 - 1.48 (m, 4H), 0.65 (dd, J = 6.9, 2.3 Hz, 2H), 0.51 - 0.28 (m, 2H)

### 2-4) Synthesis of other compounds through synthesis pathway B

In step 3 above, the following compounds were also synthesized through synthesis route B using benzoic acid or pyridinecarboxylic acid derivatives corresponding to each final synthetic compound instead of 3-(4-methyl-1H-imidazol-1-yl)-5-(trifluoromethyl)benzoic acid:
Compound 3 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 8 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide], Compound 10 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3,4-difluorobenzamide], Compound 11 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3,5-difluorobenzamide], Compound 12 [N-(3-(2-(cyclopropylamino)-7'-oxo-5'H-spiro[cyclopropane-1,8'-pyrido [4,3-d]pyrimidine]-6'(7'H)-yl)-4-methylphenyl)-4-methyl-3-(trifluoromethyl)benzamide
Compound 13 [3-bromo-N-(3-(2-(cyclopropylamino)-7'-oxo-5'H-spiro[cyclopropane-1,8'-pyrido[4,3-d]pyrimidine]-6'(7'H)-yl)-4-methylphenyl)-5-(trifluoromethyl)benzamide], Compound 27 [N-(3-(2'-(cyclopropylamino)-7-oxo-5'H-spiro[cyclopropane-1,8'-pyrido[4,3-d]pyrimidine]-6'(7'H)-yl)-4-methylphenyl)-3-fluoro-5-(trifluoromethyl)benzamide], Compound 28 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide], Compound 30 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide], Compound 32 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide], Compound 35 [N-(3-(2'-(cyclopropylamino)-7-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide], Compound 36 [N-(3-(2-(cyclopropylamino)-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide], Compound 50 [N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-4-((4-methylpiperazin-1-yl)methyl)-3-(trifluoromethyl)benzamide], Compound 62 [N-(3-(2-(cyclopropylamino)-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide], Compound 70 [N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 85 [N-(3-(2-(benzylamino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 86 [N-(4-methyl-3-(2'-morpholino-7'-oxo-5'H-spiro[cyclopropane-1,8'-pyrido[4,3-d]pyrimidine]-6'(7'H)-yl)phenyl)-3-(trifluoromethyl)benzamide], Compound 87 [N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-3-(trifluoromethyl)benzamide], Compound 88 [N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-3-(trifluoromethyl)benzamide], Compound 89 [N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-3-(trifluoromethyl)benzamide], Compound 90 [N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 91 [N-(3-(2-(benzylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 92 [N-(4-methyl-3-(2'-morpholino-7'-oxo-5'H-spiro[cyclopropane-1,8'-pyrido[4,3-d]pyrimidine]-6'(7'H)-yl)phenyl)-5
(trifluoromethyl)nicotinamide], Compound 93 [N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-5-(trifluoromethyl)nicotinamide], Compound 94 [N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-5-(trifluoromethyl)nicotinamide], Compound 95 [N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-5-(trifluoromethyl)nicotinamide], Compound 96 [N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 97 [N-(3-(2-(benzylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide], Compound 98 [2-(3,5-difluorophenyl)-N-(4-methyl-3-(2'-morpholino-7'-oxo-5'H-spiro[cyclopropane-1,8'-pyrido[4,3-d]pyrimidine]-6'(7'H)-yl)phenyl)acetamide], Compound 99 [2-(3,5-difluorophenyl)-N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)acetamide], Compound 100 [2-(3,5-difluorophenyl)-N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)acetamide], Compound 101 [2-(3,5-difluorophenyl)-N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)acetamide], Compound 102 [2-(3,5-difluorophenyl)-N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)acetamide], Compound 126 [N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 127 [(S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 128 [N-(3-(2'-(((1r,3r)-3-hydroxycyclobutyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 129
[tert-butyl 3-((6'-(2-methyl-5-(3-(trifluoromethyl)benzamido)phenyl)-7'-oxo-6',7'-dihydro-5'H-spiro[cyclopropane-1,8'-pyrido[4,3-d]pyrimidin]-2'-yl)amino)azetidine-1-carboxylate,
Compound 130 [N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 131 [(S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 132 [N-(3-(2'-(((1r,3r)-3-hydroxycyclobutyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide], and Compound 133
tert-butyl 3-((6'-(2-methyl-5-(5-(trifluoromethyl)nicotinamido)phenyl)-7'-oxo-6',7'-dihydro-5'H-spiro[cyclopropane-1,8'-pyrido[4,3-d]pyrimidin]-2'-yl)amino)azetidine-1-carboxylate.

### <Example 3> Synthetic Route C for Compound 20

Compound 20 was synthesized via synthetic route C as shown in the following Scheme 3, and other compounds were also synthesized in the same manner via synthetic route C. The synthetic method is described in detail based on compound 20.

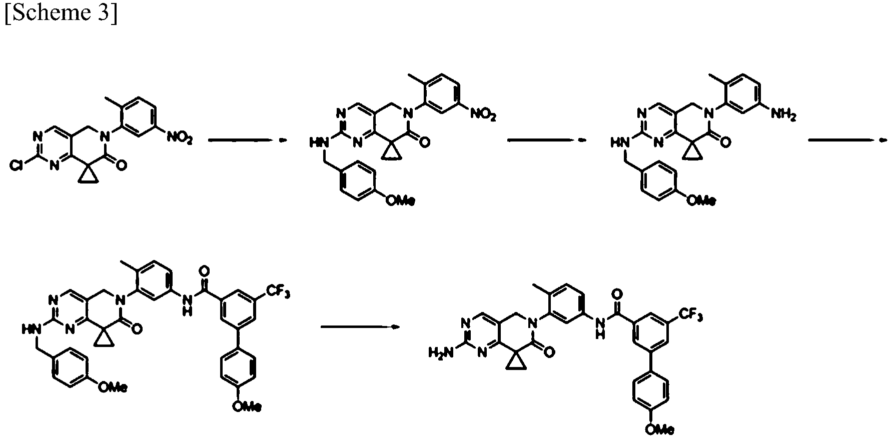

### 3-1) Step 1

The 2'-chloro-6'-(2-methyl-5-nitrophenyl)-5',6'-dihydro-7'H-spiro[cyclopropane-1,8'-pyrido[4,3-d]pyrimidine]-7'-one (0.10 g, 0.29 mmol) obtained in step 6 of Example 1 was dissolved in dimethylformamide (1.0 mL), and 4-methoxybenzylamine (0.06 mL, 0.43 mmol) and potassium carbonate (0.08 g, 0.58 mmol) were added, followed by reflux at 90 °C for 16 hours. After completion of the reaction, the mixture was diluted with water. The mixture was extracted with ethyl acetate, dried over anhydrous sodium sulfate, and concentrated under reduced pressure. The obtained residue was purified by MPLC (dichloromethane: ethyl acetate = 7:3 (v/v)), and the solvent was concentrated under reduced pressure to obtain 45.0 mg of the target compound as a yellow solid in 45% yield.
MS(m/z): 446.20[M+1]
¹H NMR (400 MHz, CDCl₃) δ 8.20 - 8.09 (m, 2H), 8.01 (s, 1H), 7.47 (d, J = 9.1 Hz, 1H), 7.27 (d, J = 6.9 Hz, 2H), 6.88 (d, J = 8.5 Hz, 2H), 4.90 (d, J = 14.8 Hz, 1H), 4.58 (d, J = 14.8 Hz, 1H), 4.52 (d, J = 5.7 Hz, 2H), 3.80 (s, 3H), 2.30 (s, 3H), 1.91 - 1.63 (m, 4H)

### 3-2) Step 2

The 2'-((4-methoxybenzyl)amino)-6'-(2-methyl-5-nitrophenyl)-5',6'-dihydro-7'H-spiro[cyclopropane-1,8'-pyrido[4,3-d]pyrimidine]-7'-one (5 g, 12.0 mmol) obtained in step 1 above was dissolved in tetrahydrofuran (120 mL), and methanol (60 mL) and water (30 mL) were added dropwise. Iron (2.7 g, 48.3 mmol) and ammonium chloride (2.82 g, 52.7 mmol) were added at room temperature, and the mixture was stirred under reflux at 80 °C for 2 hours. After completion of the reaction, the mixture was cooled to room temperature and filtered through a Celite pad. The filtrate was neutralized by dropwise addition of saturated aqueous sodium bicarbonate solution and extracted with dichloromethane. The combined organic layers were dried over anhydrous sodium sulfate and concentrated to obtain 4.65 g of the target compound as a yellow solid in 99% yield.
MS(m/z): 416.20[M+1]
¹H NMR (400 MHz, DMSO-*d₆*) δ 8.11 (s, 1H), 7.22 (d, J = 8.5 Hz, 2H), 6.91 (d, J = 8.1 Hz, 1H), 6.85 (d, J = 8.6 Hz, 2H), 6.46 (dd, J = 8.1, 2.2 Hz, 1H), 6.41 (d, J = 2.1 Hz, 1H), 4.99 (s, 2H), 4.68 (d, J = 15.5 Hz, 1H), 4.50 (d, J = 15.5 Hz, 1H), 4.34 (s, 48H), 3.71 (s, 3H), 1.89 (s, 3H), 1.52 (s, 4H)

### 3-3) Step 3

The 6'-(5-amino-2-methylphenyl)-2'-((4-methoxybenzyl)amino)-5',6'-dihydro-7'H-spiro[cyclopropane-1,8'-pyrido[4,3-d]pyrimidine]-7'-one (40 mg, 0.10 mmol) obtained in step 2 above was dissolved in dimethylformamide (1.2 mL), then 3-methoxy-5-(trifluoromethyl)benzoic acid (42.8 mg, 0.14 mmol), O-(7-azabenzotriazol-1-yl)-N,N,N',N'-tetramethyluronium hexafluorophosphate (73.2 mg, 0.19 mmol), and N,N-diisopropylethylamine (50.0 µL, 0.29 mmol) were added and the mixture was stirred at room temperature for 3 hours. After completion of the reaction, the mixture was diluted with ethyl acetate and extracted with water, and the combined organic layers were dried over anhydrous sodium sulfate and concentrated. The obtained residue was purified by MPLC (dichloromethane: ethyl acetate = 6:4 (v/v)), and the solution was concentrated under reduced pressure to obtain 40 mg of the target compound as a yellow solid in 60% yield.
MS(m/z): 694.20[M+1]
¹H NMR (400 MHz, DMSO-*d₆*) δ 10.55 (s, 1H), 8.47 (s, 1H), 8.18 (s, 1H), 8.14 (s, 2H), 7.86 - 7.77 (m, 2H), 7.74 - 7.59 (m, 3H), 7.32 (d, J = 8.4 Hz, 1H), 7.23 (d, J = 8.6 Hz, 2H), 7.14 - 7.05 (m, 2H), 6.86 (d, J = 8.6 Hz, 2H), 4.79 (d, J = 15.4 Hz, 1H), 4.61 (d, J = 15.4 Hz, 1H), 4.36 (d, J = 5.0 Hz, 2H), 3.83 (s, 3H), 3.71 (s, 3H), 2.08 (s, 3H), 1.66 - 1.40 (m, 4H)

### 3-4) Step 4

The 4'-methoxy-N-(3-(2'-((4-methoxybenzyl)amino)-7'-oxo-5'H-spiro[cyclopropane-1,8'-pyrido[4,3-d]pyrimidine]-6'(7'H)-yl)-4-methylphenyl)-5-(trifluoromethyl)-[1,1'-biphenyl]-3-carboxamide (73.3 mg, 0.106 mmol) obtained in step 3 was dissolved in a mixed solution of dichloromethane (2.0 mL) and trifluoroacetic acid (2.0 mL), and the mixture was stirred under reflux at 65 °C for 48 hours. The reaction solution was concentrated under reduced pressure, and the resulting residue was purified by HPLC (5:95 → 95:5 (v/v), for 40 min) to obtain 8.5 mg of the target compound (Compound 20) in 14% yield.
MS(m/z): 574.05[M+1]
¹H NMR (400 MHz, DMSO-*d₆*) δ 10.58 (s, 1H), 8.48 (s, 1H), 8.25 - 8.07 (m, 3H), 7.83 (d, J = 8.3 Hz, 2H), 7.72 (s, 1H), 7.68 (d, J = 9.1 Hz, 1H), 7.33 (d, J = 8.3 Hz, 1H), 7.10 (d, J = 8.3 Hz, 2H), 6.67 (s, 2H), 4.79 (d, J = 15.5 Hz, 1H), 4.62 (d, J = 14.7 Hz, 1H), 3.83 (s, 3H), 2.09 (s, 3H), 1.67 - 1.44 (m, 4H)

3-5) Synthesis of Other Compounds via Synthetic Route C

In step 3 above, the following compounds were also synthesized through synthetic route C by using benzoic acid or pyridinecarboxylic acid derivatives corresponding to each final synthetic compound instead of 3-methoxybenzene-5-(trifluoromethyl)benzoic acid:
Compound 2 [N-(3-(2'-amino-7'-oxo-5'H-spiro[cyclopropane-1,8'-pyrido[4,3-d]pyrimidine]-6'(7'H)-yl)-4-methylphenyl)-3-(trifluoromethyl)benzamide], Compound 4 [N-(3-(2'-amino-7'-oxo-5'H-spiro[cyclopropane-1,8'-pyrido[4,3-d]pyrimidine]-6'(7'H)-yl)-4-methylphenyl)-4-fluoro-3-(trifluoromethyl)benzamide], Compound 5 [N-(3-(2'-amino-7'-oxo-5'H-spiro[cyclopropane-1,8'-pyrido[4,3-d]pyrimidine]-6'(7'H)-yl)-4-methylphenyl)-4-chloro-3-(trifluoromethyl)benzamide], Compound 6 [N-(3-(2'-amino-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide], Compound 7 [N-(3-(2'-amino-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamide], Compound 9 [N-(3-(2'-amino-7'-oxo-5'H-spiro[cyclopropane-1,8'-pyrido[4,3-d]pyrimidine]-6'(7'H)-yl)-4-methylphenyl)-3,4-difluorobenzamide], Compound 14 [N-(3-(2'-amino-7'-oxo-5'H-spiro[cyclopropane-1,8'-pyrido[4,3-d]pyrimidine]-6'(7'H)-yl)-4-methylphenyl)-2,4-difluorobenzamide], Compound 15 [N-(3-(2'-amino-7'-oxo-5'H-spiro[cyclopropane-1,8'-pyrido[4,3-d]pyrimidine]-6'(7'H)-yl)-4-methylphenyl)-3,5-difluorobenzamide], Compound 16 [N-(3-(2'-amino-7'-oxo-5'H-spiro[cyclopropane-1,8'-pyrido[4,3-d]pyrimidine]-6'(7'H)-yl)-4-methylphenyl)-3-methoxybenzamide], Compound 17 [N-(3-(2'-amino-7'-oxo-5'H-spiro[cyclopropane-1,8'-pyrido[4,3-d]pyrimidine]-6'(7'H)-yl)-4-methylphenyl)-3-chloro-2-fluoro-5-(trifluoromethyl)benzamide], Compound 18 [N-(3-(2'-amino-7'-oxo-5'H-spiro[cyclopropane-1,8'-pyrido[4,3-d]pyrimidine]-6'(7'H)-yl)-4-methylphenyl)-2-fluoro-5-(trifluoromethyl)benzamide], Compound 19 [N-(3-(2'-amino-7'-oxo-5'H-spiro[cyclopropane-1,8'-pyrido[4,3-d]pyrimidine]-6'(7'H)-yl)-4-methylphenyl)-3-methoxy-5-(trifluoromethyl)benzamide], Compound 20 [N-(3-(2'-amino-7'-oxo-5'H-spiro[cyclopropane-1,8'-pyrido[4,3-d]pyrimidine]-6'(7'H)-yl)-4-methylphenyl)-4'-methoxy-5-(trifluoromethyl)-[1,1'-biphenyl]-3-carboxamide], Compound 21 [N-(3-(2'-amino-7'-oxo-5'H-spiro[cyclopropane-1,8'-pyrido[4,3-d]pyrimidine]-6'(7'H)-yl)-4-methylphenyl)-6-(trifluoromethyl)picolinamide], Compound 22 [N-(3-(2'-amino-7'-oxo-5'H-spiro[cyclopropane-1,8'-pyrido[4,3-d]pyrimidine]-6'(7'H)-yl)-4-methylphenyl)-2-(trifluoromethyl)isonicotinamide], Compound 23 [N-(3-(2'-amino-7'-oxo-5'H-spiro[cyclopropane-1,8'-pyrido[4,3-d]pyrimidine]-6'(7'H)-yl)-4-methylphenyl)-3-hydroxy-5-(trifluoromethyl)benzamide], Compound 24 [N-(3-(2'-amino-7'-oxo-5'H-spiro[cyclopropane-1,8'-pyrido[4,3-d]pyrimidine]-6'(7'H)-yl)-4-methylphenyl)-5-(trifluoromethyl)nicotinamide], Compound 25 [N-(3-(2'-amino-7'-oxo-5'H-spiro[cyclopropane-1,8'-pyrido[4,3-d]pyrimidine]-6'(7'H)-yl)-4-methylphenyl)-2-chloro-5-(trifluoromethyl)benzamide], Compound 26 [N-(3-(2'-amino-7'-oxo-5'H-spiro[cyclopropane-1,8'-pyrido[4,3-d]pyrimidine]-6'(7'H)-yl)-4-methylphenyl)-3-chloro-5-(trifluoromethyl)benzamide], Compound 29 [N-(3-(2'-amino-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide], Compound 31 [N-(3-(2'-amino-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide], Compound 33 [N-(3-(2'-amino-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide], Compound 34 [N-(3-(2'-amino-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide], Compound 37 [N-(3-(2'-amino-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide], Compound 61 [N-(3-(2'-amino-7'-oxo-5'H-spiro[cyclopropane-1,8'-pyrido[4,3-d]pyrimidine]-6'(7'H)-yl)-4-methylphenyl)-4-((4-methylpiperazin-1-yl)methyl)-3-(trifluoromethyl)benzamide], Compound 65 [N-(3-(2'-amino-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide], Compound 75 [N-(3-(2'-amino-7'-oxo-5'H-spiro[cyclopropane-1,8'-pyrido[4,3-d]pyrimidine]-6'(7'H)-yl)-4-methylphenyl)cyclobutanecarboxamide], Compound 76 [N-(3-(2'-amino-7'-oxo-5'H-spiro[cyclopropane-1,8'-pyrido[4,3-d]pyrimidine]-6'(7'H)-yl)-4-methylphenyl)-3-oxocyclobutane-1-carboxamide], Compound 77 [N-(3-(2'-amino-7'-oxo-5'H-spiro[cyclopropane-1,8'-pyrido[4,3-d]pyrimidine]-6'(7'H)-yl)-4-methylphenyl)cyclohex-1-ene-1-carboxamide], Compound 78 [N-(3-(2'-amino-7'-oxo-5'H-spiro[cyclopropane-1,8'-pyrido[4,3-d]pyrimidine]-6'(7'H)-yl)-4-methylphenyl)-4-methylthiophene-2-carboxamide], Compound 79 [N-(3-(2'-amino-7'-oxo-5'H-spiro[cyclopropane-1,8'-pyrido[4,3-d]pyrimidine]-6'(7'H)-yl)-4-methylphenyl)isoxazole-5-carboxamide], Compound 80 [N-(3-(2'-amino-7'-oxo-5'H-spiro[cyclopropane-1,8'-pyrido[4,3-d]pyrimidine]-6'(7'H)-yl)-4-methylphenyl)benzo[d]thiazole-2-carboxamide], Compound 81 [N-(3-(2'-amino-7'-oxo-5'H-spiro[cyclopropane-1,8'-pyrido[4,3-d]pyrimidine]-6'(7'H)-yl)-4-methylphenyl)-4,4,4-trifluorobutanamide], Compound 82 [N-(3-(2'-amino-7'-oxo-5'H-spiro[cyclopropane-1,8'-pyrido[4,3-d]pyrimidine]-6'(7'H)-yl)-4-methylphenyl)thiazole-5-carboxamide], Compound 83 [N-(3-(2'-amino-7'-oxo-5'H-spiro[cyclopropane-1,8'-pyrido[4,3-d]pyrimidine]-6'(7'H)-yl)-4-methylphenyl)cyclopentanecarboxamide], and Compound 84 [N-(3-(2'-amino-7'-oxo-5'H-spiro[cyclopropane-1,8'-pyrido[4,3-d]pyrimidine]-6'(7'H)-yl)-4-methylphenyl)tetrahydro-2H-pyran-4-carboxamide].

### <Example 4> Synthetic Route D for Compound 112

Compound 112 was synthesized via synthetic route D as shown in Scheme 4 below, and other compounds were also synthesized by the same method via synthetic route D. The synthetic method is described in detail based on compound 112.

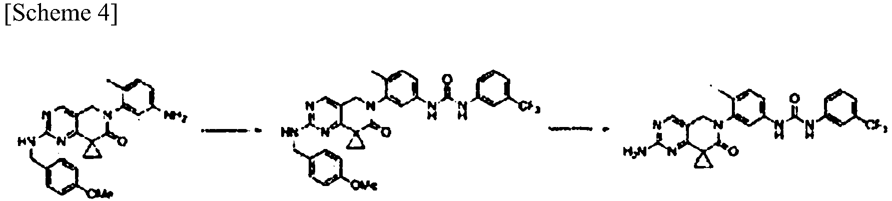

### 4-1) Step 1

The 6'-(5-amino-2-methylphenyl)-2'-((4-methoxybenzyl)amino)-5',6-dihydro-7'H-spiro[cyclopropane-1,8'-pyrido[4,3-d]pyrimidine]-7'-one (35.0 mg, 0.08 mmol) obtained in step 2 of Example 3 was dissolved in tetrahydrofuran (1.5 mL), then 3-(trifluoromethyl)phenyl isocyanate (19.0 mg, 0.10 mmol) was added and the mixture was stirred at room temperature for 2 hours. After completion of the reaction, the solvent was removed under reduced pressure, and the residue was diluted with acetonitrile. The insoluble solid was filtered using a Celite pad and washed with acetonitrile. The obtained solid was dried to afford 12.6 mg of the target compound as a white solid in 25% yield.
MS(m/z): 603.20[M+1]

### 4-2) Step 2

The 1-(3-(2-((4-methoxybenzyl)amino)-7'-oxo-5'H-spiro[cyclopropane-1,8'-pyrido[4,3-d]pyrimidine]-6'(7'H)-yl)-4-methylphenyl)-3-(3-(trifluoromethyl)phenyl)urea (9.0 mg, 15 µmol) obtained in step 1 was dissolved in trifluoroacetic acid (1.0 mL), anisole (3.0 µL, 30 µmol) was added, and the mixture was stirred under reflux at 75°C for 3 hours. After completion of the reaction, the mixture was slowly added dropwise to a saturated aqueous sodium bicarbonate solution at 0 °C and stirred slowly for 15 minutes. The reaction mixture was extracted with dichloromethane: methanol (9:1) solvent, and the collected organic layer was dried over anhydrous sodium sulfate. The solvent was concentrated under reduced pressure, and the obtained residue was purified by HPLC (5:95→95:5 (v/v), for 40 min) to obtain 1.5 mg of the target compound (compound 112) in 14% yield.
MS(m/z): 483.15[M+1]
¹H NMR (400 MHz, DMSO-*d₆*) δ 9.11 (s, 1H), 8.84 (s, 1H), 8.12 (s, 1H), 8.05 (s,1H), 7.62-7.44 (m, J=6.6, 2.9, 1.2 Hz, 3H), 7.31 (d, J=7.1 Hz, 1H), 7.28-7.16 (m,2H), 6.63 (s, 2H), 4.80 (d, J = 15.0 Hz, 1H), 4.58 (d, J = 15.5 Hz, 1H), 2.08 (s, 3H), 1.66-1.45 (m, 4H)

### 4-3) Synthesis of other compounds via synthetic route D

The following compounds were synthesized via synthetic route D:
Compound 112 [1-(3-(2'-amino-7'-oxo-5H-spiro[cyclopropane-1,8'-pyrido[4,3-d]pyrimidine]-6'(7'H)-yl)-4-methylphenyl)-3-cyclopropylurea], Compound 113 [1-(3-(2'-amino-7'-oxo-5'H-spiro[cyclopropane-1,8'-pyrido[4,3-d]pyrimidine]-6'(7'H)-yl)-4-methylphenyl)-3-cyclobutylurea], Compound 114 [1-(3-(2'-amino-7'-oxo-5'H-spiro[cyclopropane-1,8'-pyrido[4,3-d]pyrimidine]-6'(7'H)-yl)-4-methylphenyl)-3-cyclopentylurea], Compound 115 [1-(3-(2'-amino-7'-oxo-5'H-spiro[cyclopropane-1,8'-pyrido[4,3-d]pyrimidine]-6'(7'H)-yl)-4-methylphenyl)-3-(cyclopropylmethyl)urea], Compound 116 [1-(3-(2'-amino-7'-oxo-5'H-spiro[cyclopropane-1,8'-pyrido[4,3-d]pyrimidine]-6'(7'H)-yl)-4-methylphenyl)-3-(cyclohexylmethyl)urea], Compound 117 [1-(3-(2'-amino-7'-oxo-5'H-spiro[cyclopropane-1,8'-pyrido[4,3-d]pyrimidine]-6'(7'H)-yl)-4-methylphenyl)-3-benzylurea], Compound 118 [1-(3-(2'-amino-7'-oxo-5'H-spiro[cyclopropane-1,8'-pyrido[4,3-d]pyrimidine]-6'(7'H)-yl)-4-methylphenyl)-3-(4-(trifluoromethoxy)phenyl)urea], Compound 119 [1-(3-(2'-amino-7'-oxo-5'H-spiro[cyclopropane-1,8'-pyrido[4,3-d]pyrimidine]-6'(7'H)-yl)-4-methylphenyl)-3-(3-cyanophenyl)urea], Compound 120 [1-(3-(2'-amino-7'-oxo-5'H-spiro[cyclopropane-1,8'-pyrido[4,3-d]pyrimidine]-6'(7'H)-yl)-4-methylphenyl)-3-(3,4-difluorophenyl)urea], Compound 121 [1-(3-(2'-amino-7'-oxo-5'H-spiro[cyclopropane-1,8'-pyrido[4,3-d]pyrimidine]-6'(7'H)-yl)-4-methylphenyl)-3-(2-methoxyphenyl)urea], Compound 122 [1-(3-(2'-amino-7'-oxo-5'H-spiro[cyclopropane-1,8'-pyrido[4,3-d]pyrimidine]-6'(7'H)-yl)-4-methylphenyl)-3-(3-(trifluoromethyl)phenyl)urea], and Compound 125 [1-(4-methyl-3-(2-((6-methylpyridin-3-yl)amino)-7'-oxo-5'H-spiro[cyclopropane- 1,8'-pyrido[4,3-d]pyrimidine]-6'(7'H)-yl)phenyl)-3-(m-tolyl)urea].

### <Example 5> Synthetic Route E for Compound 123

Compound 123 was synthesized via synthetic route E as shown in the following Scheme 5, and the synthetic method is described below.

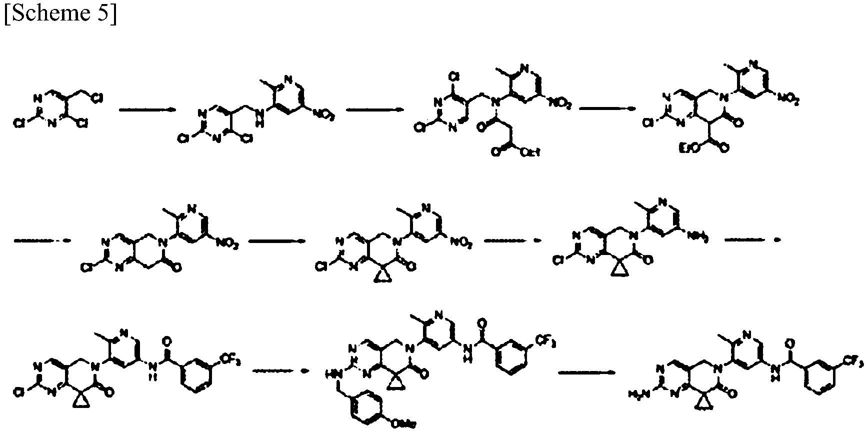

### 5-1) Step 1

The 2,4-dichloro-5-(chloromethyl)pyrimidine (6.95 g, 35.20 mmol) obtained in step 1 of Example 1 and 2-methyl-5-nitro-3-pyridinamine (7.01 g, 45.76 mmol) were dissolved in acetone (70 mL), then sodium iodide (6.86 g, 45.76 mmol) and potassium carbonate (9.73 g, 70.40 mmol) were added and the mixture was stirred under reflux at 50 °C for 2 hours. After completion of the reaction, the reaction mixture was cooled to room temperature, filtered through a Celite pad, and concentrated under reduced pressure using a rotary evaporator. The concentrate was diluted with ethyl acetate and water and extracted with ethyl acetate. The combined organic layers were dried over anhydrous sodium sulfate and concentrated. The obtained residue was purified by MPLC (dichloromethane: ethyl acetate = 7:3 (v/v)), and the solvent was concentrated under reduced pressure to obtain 3.4 g of the target compound as a yellow solid in 31% yield.
MS(m/z): 313.9[M+1]
¹H NMR (400 MHz, CDCl₃) δ 8.79(d, J=2.2Hz, 1H), 8.52 (s, 1H), 7.45(d, J=2.2 Hz, 1H), 4.57 (d, J = 5.9 Hz, 2H), 4.40 (t, J = 5.6 Hz, 1H), 2.57 (s, 3H)

### 5-2) Step 2

The N-((2,4-dichloropyrimidine-5-yl)methyl)-2-methyl-5-nitropyridine-3-amine (3.80 g, 12.10 mmol) obtained in step 1 was dissolved in anhydrous tetrahydrofuran (100 mL), and nitrogen gas was bubbled through the mixture for 5 minutes to remove dissolved gases from the mixture solution. The reaction vessel was then cooled in ice-water bath (0 °C), and 60% sodium hydride (0.51 g, 12.70 mmol) was added portionwise, followed by stirring at room temperature for 1 hour. The reaction vessel was cooled in ice-water bath (0 °C), ethyl malonyl chloride (2.28 mL, 18.15 mmol) was added dropwise, and the mixture was stirred at room temperature for 16 hours. After completion of the reaction, the mixture was cooled in ice-water bath (0 °C), and the reaction was quenched using saturated ammonium chloride solution. The remaining organic solvent was concentrated using a rotary evaporator under reduced pressure, the concentrate was diluted with water and extracted with dichloromethane. The combined organic layers were dried over anhydrous sodium sulfate and concentrated. The obtained residue was purified by MPLC (ethyl acetate: hexane = 3:7 (v/v)), and the solution was concentrated under reduced pressure to obtain 3.5 g of the target compound as a white solid in 68% yield.
MS(m/z): 428.00 [M+1]
¹H NMR (400 MHz, CDC1₃) δ 9.40 (d, J = 2.4 Hz, 1H), 8.95 (s, 1H), 8.20 (d, J = 2.4 Hz, 1H), 5.36 (d, J= 15.3 Hz, 1H), 4.52 (d, J =15.3 Hz, 1H) , 4.13 (qd, J = 7.1 , 2.0 Hz,2H), 3.25 (d, J= 15.7 Hz, 1H), 3.09 (d, J= 15.7 Hz, 1H), 2.64 (s, 3H), 1.23 (t, J=7.2 Hz, 3H)

### 5-3) Step 3

The ethyl 3-(((2,4-dichloropyrimidine-5-yl)methyl)(2-methyl-5-methylpyrimidine)) derived from step 2: Nitrophyridine-3-yl)amino)-3-oxopropane ether (1.66 g, 3.88 mmol) was dissolved in dimethylsulfoxide (30 mL) and nitrogen gas was bubbled through the mixture for 5 minutes. The reaction vessel was cooled in ice-water bath (0 °C), and cesium carbonate (1.51 g, 4.65 mmol) was added portionwise. After the reaction was completed, the mixture was quenched to 1N hydrochloric acid solution (200 mL) in the 0 °C and slowly stirred for 15 minutes. The mixture was extracted with dichloromethane. The combined organic layer was dried over anhydrous sodium sulfate and concentrated.
MS(m/z): 392.00[M+1]

### 5-4) Step 4

The ethyl 2-chloro-6-(2-methyl-5-nitropyridin-3-yl)-7-oxo-5,6,7,8-tetrahydropyrido[4,3-d]pyrimidine-8-carboxylate (0.34 g, 0.87 mmol) obtained in step 3 above was dissolved in 1,4-dioxane (10 mL). A 4N aqueous hydrochloric acid solution (0.56 mL, 8.68 mmol) dissolved in 1,4-dioxane was added, and the mixture was stirred under reflux at 100 °C for 2 hours. After completion of the reaction, the mixture was cooled to room temperature, and the residue was removed under reduced pressure. The residue was diluted with dichloromethane. The insoluble solid was filtered using a Celite pad, and the collected filtrate was dried over anhydrous sodium sulfate and concentrated. The obtained residue was purified by MPLC (ethyl acetate: dichloromethane = 6:4 (v/v)), and the solvent was concentrated under reduced pressure to obtain 0.15 g of the target compound as a yellow solid in 53% yield.
MS(m/z): 319.95 [M+1]
¹H NMR (400 MHz, CDCl₃) δ 9.37 (d, J = 2.4 Hz, 1H), 8.55 (s, 1H), 8.36 (d, J = 2.4 Hz, 1H), 4.95 (d, J = 15.9 Hz, 1H), 4.75 (d, J = 15.8 Hz, 1H), 4.04 (s, 2H), 2.55 (s, 3H)

### 5-5) Step 5

The 2-chloro-6-(2-methyl-5-nitropyridin-3-yl)-5,8-dihydropyrido[4,3-d]pyrimidin-7(6H)-one (0.12 g, 0.37 mmol) obtained in step 4 was dissolved in anhydrous dimethylformamide (3.0 mL), and nitrogen gas was bubbled through the mixture for 5 minutes. Then, (2-bromoethyl)diphenylsulfonium trifluoromethanesulfonate (0.25 g, 0.56 mmol) was added and the mixture was stirred at room temperature for 5 minutes. Triethylamine (0.16 mL, 0.56 mmol) was slowly added, and the mixture was stirred at room temperature for 1 hour. After completion of the reaction, the mixture was quenched with saturated ammonium chloride solution and extracted with dichloromethane. The combined organic layers were dried over anhydrous sodium sulfate and concentrated. The obtained residue was purified by MPLC (ethyl acetate: dichloromethane = 3:7 (v/v)), and the solvent was concentrated under reduced pressure to obtain 87.0 mg of the target compound as a yellow solid in 67% yield.
MS(m/z): 346.05[M+1]
¹H NMR (400 MHz, CDCl₃) δ 9.36 (d, J = 2.4 Hz, 1H), 8.39 (d, J = 2.5 Hz, 2H), 5.06 (d, J = 15.8 Hz, 1H), 4.82 (d, J = 15.8 Hz, 1H), 2.57 (s, 3H), 2.10 - 1.85 (m, 4H)

### 5-6) Step 6

The 2'-chloro-6'-(2-methyl-5-nitropyridin-3-yl)-5',6'-dihydro-7'H-spiro[cyclopropane-1,8'-pyrido[4,3-d]pyrimidine]-7'-one (0.08 g, 0.23 mmol) obtained in step 5 above was dissolved in tetrahydrofuran (4.0 mL), and methanol (2.0 mL) and water (1.0 mL) were added. Iron (0.06 g, 1.16 mmol) and ammonium chloride (0.06 g, 1.16 mmol) were added at room temperature, and the mixture was stirred under reflux at 80 °C for 2 hours. After completion of the reaction, the mixture was cooled to room temperature and filtered through a Celite pad. The filtrate was diluted with water and extracted with dichloromethane. The combined organic layer was dried over anhydrous sodium sulfate and concentrated to obtain 62.6 mg of the target compound as a pale-yellow solid in 91% yield.
MS(m/z): 316.10[M+1]
¹H NMR (400 MHz, CDC1₃) δ 8.36 (s, 1H), 8.06 (d, J = 2.6 Hz, 1H), 6.89 (d, J =2.6 Hz, 1H), 4.90 (d, J= 16.5 Hz, 1H), 4.79 (d, J= 16.4 Hz, 1H), 3.72 (s, 2H), 2.33 (s, 3H), 2.08 - 2.00 (m, 2H), 1.92 - 1.83 (m, 2H).

### 5-7) Step 7

The 6'-(5-amino-2-methylpyridin-3-yl)-2'-chloro-5',6'-dihydro-7'H-spiro[cyclopropane-1,8'-pyrido[4,3-d]pyrimidin]-7'-one (0.07 g, 0.22 mmol) obtained in step 6 was dissolved in dichloromethane (5.0 mL), then 3-(trifluoromethyl)benzoyl chloride (0.06 mL, 0.40 mmol) and potassium carbonate (0.06 g, 0.44 mmol) were added and the mixture was stirred at room temperature for 1 hour. After completion of the reaction, the mixture was quenched with water and extracted with dichloromethane. The combined organic layers were dried over anhydrous sodium sulfate and concentrated. The obtained residue was purified by MPLC (ethyl acetate: dichloromethane = 4:6 (v/v)), and the solvent was concentrated under reduced pressure to afford 62.6 mg of the target compound as a yellow solid in 58% yield.
MS(m/z): 488.00[M+1]
¹H NMR (400 MHz, CDCl₃) δ 8.83 (s, 1H), 8.42 (d, J = 2.3 Hz, 1H), 8.37 (s, 1H), 8.29 (d, J = 2.4 Hz, 1H), 8.17 (s, 1H), 8.13 (d, J = 7.8 Hz, 1H), 7.86 (d, J = 7.8 Hz, 1H), 7.67 (t, J = 7.8 Hz, 1H), 5.05 (d, J = 16.3 Hz, 1H), 4.80 (d, J = 16.6 Hz, 1H), 2.19 (s, 3H), 2.14-1.88 (m, 4H)

### 5-8) Step 8

The N-(5-(2'-chloro-7'-oxo-5'H-spiro[cyclopropane-1,8'-pyrido[4,3-d]pyrimidine]-6'(7'H)-yl)-6-methylpyridin-3-yl)-3-(trifluoromethyl)benzamide (0.06 g, 0.12 mmol) obtained in step 7 above was dissolved in dimethyl sulfoxide (3.0 mL), and 4-methoxybenzylamine (0.16 mL, 1.23 mmol) was added dropwise at room temperature. The reaction mixture was stirred at 90 °C for 2 hours. After completion of the reaction, the mixture was diluted with water and extracted with ethyl acetate, and the combined organic layer was dried over anhydrous sodium sulfate and concentrated. The obtained residue was purified by MPLC (dichloromethane: methanol = 95:5 (v/v)), and the solvent was concentrated under reduced pressure to afford 65.8 mg of the target compound as a yellow solid (Compound 123) in 91% yield.
MS(m/z): 589.20[M+1]
¹H NMR (400 MHz, DMSO-*d₆*) δ 8.78 (d, J = 2.3 Hz, 1H), 8.33 (s, 1H), 8.29 (d, J = 7.9 Hz, 1H), 8.17 (d, J = 2.3 Hz, 1H), 8.14 (s, 1H), 8.01 (d, J = 7.8 Hz, 1H), 7.82 (t, J = 7.8 Hz, 1H), 6.90 - 6.84 (m, 4H), 4.85 (d, J = 15.1 Hz, 1H), 4.66 (d, J = 15.2 Hz, 1H), 3.73 (s, 3H), 3.66 (s, 2H), 2.28 (s, 3H), 1.68 - 1.45 (m, 4H)

### 5-9) Step 9

The N-(5-(2'-((4-methoxybenzyl)amino)-7'-oxo-5'H-spiro[cyclopropane-1,8'-pyrido[4,3-d]pyrimidine]-6'(7'H)-yl)-6-methylpyridin-3-yl)-3-(trifluoromethyl)benzamide (0.65 mg, 0.11 mmol) obtained in step 8 above was dissolved in a solution of trifluoroacetic acid (3.0 mL), anisole (23.0 µL, 0.22 mmol) was added, and the mixture was stirred under reflux at 75 °C for 3 hours. After completion of the reaction, the mixture was concentrated under reduced pressure, and the obtained residue was purified by MPLC (dichloromethane: methanol = 95:5 (v/v)). The solvent was concentrated under reduced pressure to obtain 32.0 mg of the target compound as a pale-yellow solid (Compound 123, N-(5-(2'-amino-7'-oxo-5'H-spiro[cyclopropane-1,8'-pyrido[4,3-d]pyrimidine]-6'(7'H)-yl)-6-methylpyridin-3-yl)-3-(trifluoromethyl)benzamide) in 62% yield.
MS(m/z): 469.10[M+1]
¹H NMR (400 MHz, DMSO-*d₆*) δ 10.76 (s, 1H), 8.78 (d, J=2.3 Hz, 1H), 8.34 (s, 1H), 8.29 (d, J=7.9 Hz, 1H), 8.20 (d, J=2.3 Hz, 1H), 8.13 (s, 1H), 8.01 (d, J=7.8 Hz, 1H), 7.82 (t, J=7.8 Hz, 1H), 6.68 (s, 2H), 4.85 (d, J=15.1 Hz, 1H), 4.67 (d, J=15.2 Hz, 1H), 2.30 (s, 3H), 1.68-1.50 (m, 4H).

### <Example 6> Synthetic Route F for Compound 124

Compound 124 was synthesized through synthetic route F as shown in the following Scheme 6. The synthetic method is described in detail based on compound 124.

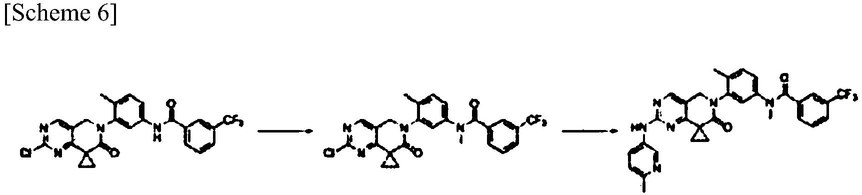

### 6-1) Step 1

To N,N-dimethylformamide (3 mL) were added N-(3-(2'-chloro-7'-oxo-5'H-spiro[cyclopropane-1,8'-pyrido[4,3-d]pyrimidine]-6'(7'H)-yl)-4-methylphenyl)-3-(trifluoromethyl)benzamide (250 mg, 0.51 mmol), methyl iodide (0.16 mL, 2.57 mmol), and potassium carbonate (213 mg, 1.54 mmol), and the mixture was stirred under reflux at 120 °C for 12 hours. After completion of the reaction, the mixture was cooled to room temperature and water was added dropwise. The obtained residue was purified by flash column chromatography (0-5% dichloromethane: methanol), and the solvent was concentrated under reduced pressure to obtain 120 mg of the target compound as a solid in 47% yield.
MS(m/z): 501[M+1]

### 6-2) Step 2

The N-(3-(2-chloro-7'-oxo-5'H-spiro[cyclopropane-1,8'-pyrido[4,3-d]pyrimidine]-6'(7'H)-yl)-4-methylphenyl)-N-methyl-3-(trifluoromethyl)benzamide (0.12 g, 0.24 mmol) obtained in step 1 was dissolved in 2-butanol (10.0 mL), and potassium carbonate (0.17 g, 1.23 mmol), 6-methylpyridine-3-amine (39 mg, 0.36 mmol), and tris(dibenzylideneacetone)dipalladium(0) (46 mg, 0.050 mmol) were added dropwise at room temperature. The reaction mixture was stirred at 100 °C for 1 hour. After completion of the reaction, the mixture was cooled to room temperature, filtered through a Celite pad, and concentrated under reduced pressure. The obtained residue was purified by flash column chromatography (0-5% dichloromethane: methanol), and the solvent was concentrated under reduced pressure to afford 104 mg of the target compound as a brown solid (Compound 124, N-methyl-N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide) in 76% yield.
MS(m/z): 573[M+1]

### <Example 7> Synthetic Route G for Compound 134

Compound 134 was synthesized via synthetic route G as shown in the following Scheme 7, and the synthetic method is described below.

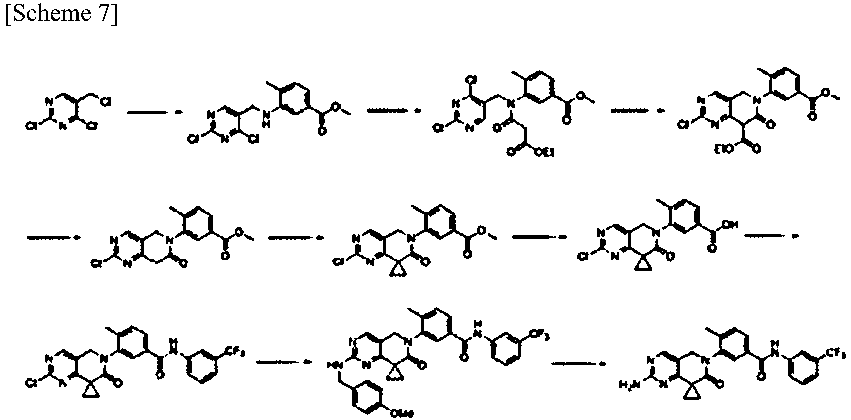

### 7-1) Step 1

2,4-Dichloro-5-(chloromethyl)pyrimidine (27.8 g, 140.80 mmol) obtained in Step 1 of Example 1 and methyl 3-amino-4-methylbenzoate (30.24 g, 183.04 mmol) were dissolved in acetone (300 mL), then sodium iodide (27.44 g, 183.04 mmol) and potassium carbonate (38.92 g, 281.60 mmol) were added, and the mixture was stirred under reflux at 50 °C for 2 hours. After completion of the reaction, the mixture was cooled to room temperature, filtered through a Celite pad, and concentrated using a rotary evaporator. The concentrate was diluted with ethyl acetate and water and extracted with ethyl acetate The combined organic layers were dried over anhydrous sodium sulfate and concentrated. The solvent was concentrated under reduced pressure to obtain 36.3 g of the target compound as a yellow oil in 79.0% yield, which was used in the next reaction without purification.
MS(m/z): 326 [M+1]

### 7-2) Step 2

The methyl 3-(((2,4-dichloropyrimidin-5-yl)methyl)amino)-4-methylbenzoate (36.3 g, 111.13 mmol) obtained in step 1 was dissolved in anhydrous tetrahydrofuran (300 mL), and nitrogen gas was bubbled through the mixture for 5 minutes to remove dissolved gases from the mixed solution. The reaction vessel was then cooled in an ice-water bath (0 °C), and 60% sodium hydride (5.78 g, 144.47 mmol) was added portionwise, followed by stirring at room temperature for 1 hour. The reaction vessel was placed in an ice-water bath at 0 °C, ethyl malonyl chloride (20.92 mL, 166.69 mmol) was added dropwise, and the mixture was stirred at room temperature for 16 hours. After completion of the reaction, the reaction vessel was cooled in an ice-water bath (0 °C), and the reaction was quenched with saturated ammonium chloride solution. The remaining organic solvent was concentrated under reduced pressure using a rotary evaporator, and the concentrate was diluted with water and extracted with dichloromethane. The combined organic layers were dried over anhydrous sodium sulfate and concentrated. The obtained residue was purified by MPLC (ethyl acetate: hexane = 3:7 (v/v)), and the solution was concentrated under reduced pressure to obtain 20.2 g of the target compound as a yellow oil in 41.2% yield.
MS(m/z): 440[M+1]

### 7-3) Step 3

The methyl 3-(N-((2,4-dichloropyrimidine-5-yl)methyl)-3-ethoxy-3-oxopropanamido)-4-methylbenzoate (0.3 g, 0.68 mmol) obtained in step 2 above was dissolved in tetrahydrofuran (6.0 mL), and nitrogen gas was bubbled through the mixture for 5 minutes. The reaction vessel was cooled in an ice-water bath (0 °C), and sodium hydride 60% (0.04 g, 1.02 mmol) was added portionwise, followed by stirring at 30 °C for 12 hours. After completion of the reaction, water was added to quench the reaction, and the mixture was extracted with a solution of dichloromethane: methanol (9:1). The combined organic layers were dried over anhydrous sodium sulfate and concentrated. The obtained residue was purified by MPLC (hexane: ethyl acetate = 4:6 (v/v)), and the solvent was concentrated under reduced pressure to afford 0.25 g of the target compound as a yellow solid in 91% yield.
MS(m/z): 404 [M+1]

### 7-4) Step 4

The ethyl 2-chloro-6-(5-(methoxycarbonyl)-2-methylphenyl)-7-oxo-5,6,7,8-tetrahydropyrido[4,3-d]pyrimidine-8-carboxylate (0.3 g, 0.74 mmol) obtained in step 3 above was dissolved in 1,4-dioxane (15 mL). A 4N hydrochloric acid aqueous solution (0.3 mL, 7.4 mmol) dissolved in 1,4-dioxane was added, and the mixture was stirred under reflux at 80 °C for 1 hour. After completion of the reaction, the mixture was cooled to room temperature and the residue was removed under reduced pressure, then the residue was diluted with dichloromethane. The insoluble solids were filtered using a Celite pad and the collected filtrate was dried over anhydrous sodium sulfate and concentrated. The obtained residue was purified by MPLC (hexane: ethyl acetate = 4:6 (v/v)), and the solution was concentrated under reduced pressure to obtain 0.1 g of the target compound as a yellow solid in 63% yield.
MS(m/z): 331[M+1]

### 7-5) Step 5

The methyl 3-(2-chloro-7-oxo-7,8-dihydropyrido[4,3-d]pyrimidine-6(5H)-yl)-4-methylbenzoate (0.03 g, 0.093 mmol) obtained in step 4 above was dissolved in anhydrous dimethylformamide (1.0 mL), and nitrogen gas was bubbled through the mixture for 5 minutes. (2-Bromoethyl)diphenylsulfonium trifluoromethanesulfonate (0.06 g, 0.14 mmol) was then added and the mixture was stirred at room temperature for 5 minutes. Triethylamine (0.04 mL, 0.28 mmol) was slowly added to the reaction mixture and stirred at room temperature for 1 hour. After completion of the reaction, the reaction was quenched with saturated ammonium chloride solution and extracted with dichloromethane. The combined organic layers were dried over anhydrous sodium sulfate and concentrated. The obtained residue was purified by MPLC (hexane: ethyl acetate = 4:6 (v/v)), and the solvent was concentrated under reduced pressure to obtain 28.3 mg of the target compound as a pale-yellow solid in 85% yield.
MS(m/z): 358[M+1]

### 7-6) Step 6

Methyl 3-(2-chloro-7'-oxo-5'H-spiro[cyclopropane-1,8'-pyrido[4,3-d]pyrimidine]-6'(7'H)-yl)-4-methylbenzoate (0.03 g, 0.014 mmol) obtained in step 5 was dissolved in tetrahydrofuran (0.5 mL) and water (0.5 mL) was added. Lithium hydroxide (0.06 g, 1.16 mmol) and ammonium chloride (18.0 mg, 0.42 mmol) were added at room temperature, and the mixture was stirred under reflux at 30 °C for 2 hours. After completion of the reaction, 1N aqueous hydrochloric acid solution was added dropwise to the reaction mixture to adjust the pH to 2-3, then the mixture was diluted with water and extracted with dichloromethane. The combined organic layers were dried over anhydrous sodium sulfate and concentrated to proceed to the next reaction without purification. The target compound was obtained as a pale yellow solid (17.0 mg, 60% yield).
MS(m/z): 344[M+1]

### 7-7) Step 7

The 3-(2-chloro-7'-oxo-5'H-spiro[cyclopropane-1,8'-pyrido[4,3-d]pyrimidine]-6'(7'H)-yl)-4-methyl-N-(3-(trifluoromethyl)phenyl)benzamide (17.0 mg, 0.05 mol) obtained in step 6 was dissolved in dimethylformamide (1 mL), then HATU (28.0 mg, 0.074 mmol), N,N-diisopropylethylamine (0.03 mL, 0.15 mmol), and 3-(trifluoromethyl)aniline (0.01 mL, 0.074 mmol) were added and the mixture was stirred at 60 °C for 3 hours. After completion of the reaction, the mixture was quenched with water and extracted with ethyl acetate. The combined organic layers were dried over anhydrous sodium sulfate and concentrated. The obtained residue was purified by MPLC (hexane: ethyl acetate = 5:5 (v/v)), and the solution was concentrated under reduced pressure to afford 7 mg of the target compound as a pale-yellow solid in 29% yield.
MS(m/z): 487[M+1]

### 7-8) Step 8

The 3-(2-chloro-7'-oxo-5'H-spiro[cyclopropane-1,8'-pyrido[4,3-d]pyrimidine]-6'(7'H)-yl)-4-methyl-N-(3-(trifluoromethyl)phenyl)benzamide (6.5 mg, 0.013 mmol) obtained in step 7 above was dissolved in dimethyl sulfoxide (0.5 mL), and 4-methoxybenzylamine (17.0 µL, 0.13 mmol) was added dropwise at room temperature. The reaction mixture was stirred at 90 °C for 2 hours. After completion of the reaction, the mixture was diluted with water and extracted with ethyl acetate, and the combined organic layer was dried over anhydrous sodium sulfate and concentrated. The obtained residue was purified by MPLC (hexane: ethyl acetate = 3:7 (v/v)), and the solution was concentrated under reduced pressure to afford 4 mg of the target compound as a pale-yellow solid in 52% yield.
MS(m/z): 588[M+1]

### 7-9) Step 9

The N-(3-(2-amino-7'-oxo-5'H-spiro[cyclopropen-1,8-pyrido[4,3-d]pyrimidine]-6'(7'H)-yl)-4-methylphenyl)-3-(trifluoromethyl)benzamide (4 mg, 0.009 mmol) obtained in step 8 above was dissolved in a solution of trifluoroacetic acid (0.2 mL), then anisole (2.0 mL, 0.017 mmol) was added and the mixture was stirred under reflux at 75 °C for 3 hours. After completion of the reaction, the mixture was concentrated under reduced pressure, and the obtained residue was purified by Prep HPLC (water: acetonitrile = 10 to 95%). The solvent was concentrated under reduced pressure to afford 1 mg of the target compound as a white solid (Compound 134, 3-(2-amino-7'-oxo-5'H-spiro[cyclopropen-1,8-pyrido[4,3-d]pyrimidine]-6'(7'H)-yl)-4-methyl-N-(3-(trifluoromethyl)phenyl)benzamide) in 23% yield.
MS(m/z): 468 [M+1]
¹H NMR (400 MHz, DMSO) δ 10.50 (s, 1H), 8.22 (s, 1H), 8.12 (s, 1H), 8.08 (d, J = 8.7 Hz, 1H), 7.96 (s, 1H), 7.91 (d, J = 7.8 Hz, 1H), 7.63-7.57 (m, 1H), 7.48 (dd, J = 17.5, 8.0 Hz, 2H), 6.67 (s, 2H), 4.91 (d, J = 15.3 Hz, 1H), 4.64 (d, J = 15.4 Hz, 1H), 2.19 (s, 3H), 1.65-1.49 (m, 4H).

Information on the example compounds synthesized in the present invention is provided in Table 1 through Table 6 below.

**[Table 1]**

| No. | Structural formula | Compound name | Scheme | ¹H NMR; MS[M+H] |
|---|---|---|---|---|
| 1 | 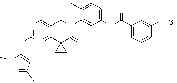 | N-(3-(2'-((1,3-dimethyl-1H-pyrazol-5-yl)amino)-7'-oxo-5'H-spiro[cyclopropane-1,8'-pyrido[4,3-d]pyrimidin]-6'(7'H)-yl)-4-methylphenyl)-3-(trifluoromethyl)benzamid e | A | ¹H NMR (400 MHz, MeOD) δ 8.25 (s, 1H), 8.19 (d, J = 7.8 Hz, 1H), 8.14 (s, 1H), 7.89 (d, J = 7.8 Hz, 1H), 7.78 (d, J = 2.0 Hz, 1H), 7.73 (t, J = 7.8 Hz, 1H), 7.57 (dd, J = 8.3, 2.0 Hz, 1H), 7.34 (d, J = 8.4 Hz, 1H), 4.94 (d, J = 15.7 Hz, 1H), 4.72 (d, J = 15.7 Hz, 1H), 3.72 (t, J = 5.6 Hz, 2H), 3.54 (t, J = 5.6 Hz, 2H), 2.18 (s, 3H), 1.95 - 1.74 (m, 3H); 563.0 [M+H] |
| 2 | 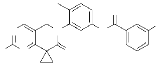 | N-(3-(2'-amino-7'-oxo-5'H-spiro[cyclopropane-1,8'-pyrido[4,3-d]pyrimidine]-6'(7'H)-yl)-4-methylphenyl)-3-(trifluoromethyl)benzamide | C | ¹H NMR (400 MHz, DMSO-*d₆*) δ 10.51 (s, 1H), 8.29 (s, 1H), 8.25 (d, J = 8.1 Hz, 1H), 8.13 (s, 1H), 7.96 (d, J = 7.8 Hz, 1H), 7.79 (t, J = 7.8 Hz, 1H), 7.72 (d, J = 2.1 Hz, 1H), 7.63 (dd, J = 8.3, 2.2 Hz, 1H), 7.31 (d, J = 8.4 Hz, 1H), 4.79 (d, J = 15.5 Hz, 1H), 4.62 (d, J = 15.5 Hz, 1H), 2.08 (s, 3H), 1.65 - 1.50 (m, 4H); 468.05 [M+H] |
| 3 | 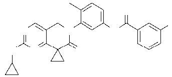 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(trifluoromethyl)benzamid e | B | ¹H NMR (400 MHz, MeOD) δ 8.25 (s, 1H), 8.22 - 8.16 (m, J = 7.4 Hz, 2H), 7.90 (d, J = 7.8 Hz, 1H), 7.81 (d, J = 2.1 Hz, 1H), 7.73 (t, J = 7.8 Hz, 1H), 7.56 (dd, J = 8.3, 2.2 Hz, 1H), 7.35 (d, J = 8.3 Hz, 1H), 4.97 (d, J = 16.2 Hz, 1H), 4.75 (d, J = 16.2 Hz, 1H), 2.70 (ddd, J = 10.5, 6.9, 3.6 Hz, 1H), 2.19 (s, 3H), 1.96 - 1.79 (m, 4H), 0.96 - 0.83 (m, J = 6.9 Hz, 3H), 0.69 - 0.58 (m, 2H); 508.1 [M+H] |
| 4 | 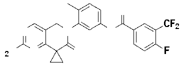 | N-(3-(2'-amino-7'-oxo-5'H-spiro[cyclopropane-1,8'-pyrido[4,3-d]pyrimidine]-6'(7'H)-yl)-4-methylphenyl)-4-fluoro-3-(trifluoromethyl)benzamid e | C | ¹H NMR (400 MHz, MeOD) δ 8.30 (d, J = 6.8 Hz, 1H), 8.28 - 8.21 (m, 1H), 8.13 (s, 1H), 7.81 (d, J = 2.1 Hz, 1H), 7.59 - 7.44 (m, 2H), 7.33 (d, J = 8.4 Hz, 1H), 4.96 (s, 1H), 4.73 (d, J = 15.9 Hz, 1H), 2.18 (s, 3H), 2.00 - 1.80 (m, 4H); 486. 1 [M+H] |
| 5 | 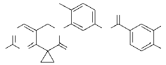 | N-(3-(2'-amino-7'-oxo-5'H-spiro[cyclopropane-1,8'-pyrido[4,3-d]pyrimidine]-6'(7'H)-yl)-4-methylphenyl)-4-chloro-3-(trifluoromethyl)benzamid e | C | ¹H NMR (400 MHz, DMSO-*d₆*) δ 10.59 (s, 1H), 8.40 (d, J = 1.7 Hz, 1H), 8.25 (dd, J = 8.4, 1.8 Hz, 1H), 8.17 (s, 1H), 7.94 (d, J = 8.4 Hz, 1H), 7.74 (d, J = 2.0 Hz, 1H), 7.61 (dd, J = 8.3, 2.0 Hz, 1H), 7.32 (d, J = 8.4 Hz, 1H), 4.80 (d, J = 15.5 Hz, 1H), 4.63 (d, J = 15.5 Hz, 1H), 2.09 (s, 3H), 1.79 - 1.48 (m, J = 13.9, 9.8, 6.0 Hz, 4H); 502. 1 [M+H] |
| 6 | 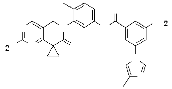 | N-(3-(2'-amino-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamid e | C | ¹H NMR (400 MHz, MeOD) δ 9.37 (s, 1H), 8.54 (s, 1H), 8.47 (s, 1H), 8.32 (s, 1H), 8.11 (s, 1H), 7.92 (s, 1H), 7.80 (d, J = 2.1 Hz, 1H), 7.62 (dd, J = 8.3, 2.1 Hz, 1H), 7.37 (d, J = 8.3 Hz, 1H), 4.72 (d, J = 15.7 Hz, 1H), 2.45 (d, J = 0.8 Hz, 3H), 2.20 (s, 3H), 1.87 - 1.65 (m, 4H); 548. 1 [M+H] |
| 7 | 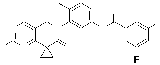 | N-(3-(2'-amino-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamid e | C | ¹H NMR (400 MHz, DMSO-*d₆*) δ 10.58 (s, 1H), 8.14 (s, 2H), 8.07 (d, J = 9.1 Hz, 1H), 7.94 (d, J = 8.3 Hz, 1H), 7.69 (d, J = 2.0 Hz, 1H), 7.61 (dd, J = 8.3, 2.1 Hz, 1H), 7.32 (d, J = 8.4 Hz, 1H), 4.79 (d, J = 15.4 Hz, 1H), 4.62 (d, J = 15.5 Hz, 1H), 2.08 (s, 3H), 1.62 (m, 4H); 486.05 [M+H] |
| 8 | 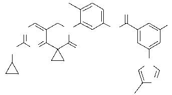 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamid e | B | ¹H NMR (400 MHz, DMSO-*d₆*) δ 10.65 (s, 1H), 9*.*50 (s, 1H), 8.57 (s, 1H), 8.42 (s, 2H), 8.15 (d, J = 24.9 Hz, 2H), 7.72 - 7.66 (m, 2H), 7.41 (s, 1H), 7.35 (d, J = 8.9 Hz, 1H), 4.80 (d, J = 15.3 Hz, 1H), 4.65 (d, J = 15.3 Hz, 1H), 2.34 (s, 3H), 2.10 (s, 3H), 1.77 - 1.48 (m, 4H), 0.65 (dd, J = 6.9, 2.3 Hz, 2H), 0.51 - 0.28 (m, 2H); 588.2[M+H] |
| 9 | 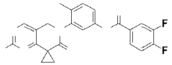 | N-(3-(2'-amino-7'-oxo-5'H-spiro[cyclopropane-1,8'-pyrido[4,3-d]pyrimidine]-6'(7'H)-yl)-4-methylphenyl)-3,4-difluorobenzamide | C | ¹H NMR (400 MHz, MeOD) δ 8.13 (s, 1H), 7.88 (ddd, J = 11.2, 7.6, 2.2 Hz, 1H), 7.83 - 7.77 (m, 1H), 7.76 (d, J = 2.2 Hz, 1H), 7.53 (dd, J = 8.3, 2.2 Hz, 1H), 7.46 - 7.38 (m, 1H), 7.34 (d, J = 8.4 Hz, 1H), 4.94 (d, J = 16.1 Hz, 1H), 4.72 (d, J = 16.1 Hz, 1H), 2.18 (s, 3H), 1.93 - 1.79 (m, 4H); 436.15 [M+H] |
| 10 | 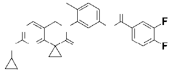 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,4-difluorobenzamide | B | ¹H NMR (400 MHz, DMSO-*d₆*) δ 10.35 (s, 1H), 8.16 (s, 1H), 8.02 (ddd, J = 11.5, 7.8, 2.1 Hz, 1H), 7.91 - 7.81 (m, 1H), 7.71 (d, J = 2.1 Hz, 1H), 7.66 - 7.57 (m, 2H), 7.36 - 7.25 (m, 2H), 4.80 (d, J = 15.4 Hz, 1H), 4.62 (d, J = 15.5 Hz, 1H), 2.66 (dt, J = 10.8, 3.6 Hz, 1H), 2.08 (s, 3H), 1.70 - 1.51 (m, 4H), 0.68 - 0.60 (m, 2H), 0.50 - 0.38 (m, 2H); 476.2[M+H] |
| 11 | 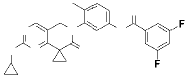 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,5-difluorobenzamide | B | ¹H NMR (400 MHz, DMSO-d₆) δ 10.40 (s, 1H), 8.17 (s, 1H), 7.72 (d, J = 2.1 Hz, 1H), 7.71 - 7.64 (m, 2H), 7.61 (dd, J = 8.3, 2.1 Hz, 1H), 7.52 (tt, J = 9.1, 2.3 Hz, 1H), 7.35 - 7.25 (m, 2H), 4.80 (d, J = 15.4 Hz, 1H), 4.62 (d, J = 15.4 Hz, 1H), 2.66 (dt, J = 10.6, 3.5 Hz, 1H), 2.08 (s, 3H), 1.73 - 1.49 (m, 4H), 0.71 - 0.59 (m, 2H), 0.53 - 0.38 (m, 2H); 476.2[M+H] |
| 12 | 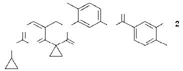 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-methyl-3-(trifluoromethyl)benzamid e | B | ¹H NMR (400 MHz, DMSO-*d₆*) δ 10.44 (s, 1H), 8.25 (s, 1H), 8.19 - 8.13 (m, 2H), 7.72 (d, J = 2.1 Hz, 1H), 7.67 - 7.60 (m, 2H), 7.31 (d, J = 8.5 Hz, 2H), 4.82 (d, J = 15.4 Hz, 1H), 4.63 (d, J = 15.4 Hz, 1H), 2.68 (dd, J = 3.6, 2.1 Hz, 1H), 2.53 (s, 3H), 2.09 (s, 3H), 1.72 - 1.50 (m, 4H), 0.68 - 0.62 (m, 2H), 0.47 - 0.42 (m, 2H); 522.2[M+H] |
| 13 | 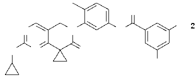 | 3-bromo-N-(3-(2'-(cyclopropylamino)-7'-oxo-5'H-spiro[cyclopropane-1,8'-pyrido[4,3-d]pyrimidine]-6'(7'H)-yl)-4-methylphenyl)-5-(trifluoromethyl)benzamid e | B | ¹H NMR (400 MHz, DMSO-*d₆*) δ 10.56 (s, 1H), 8.46 (s, 1H), 8.29 (s, 1H), 8.23 (s, 1H), 8.17 (s, 1H), 7.70 (d, J = 2.0 Hz, 1H), 7.64 (dd, J = 8.3, 2.0 Hz, 1H), 7.33 (d, J = 8.5 Hz, 1H), 7.30 (d, J = 3.4 Hz, 1H), 4.81 (d, J = 15.4 Hz, 1H), 4.63 (d, J = 15.3 Hz, 1H), 2.67 (td, J = 7.0, 3.5 Hz, 1H), 2.09 (s, 3H), 1.72 - 1.50 (m, 4H), 0.72 - 0.59 (m, 2H), 0.53 - 0.39 (m, 2H); 586.1[M+H] |
| 14 | 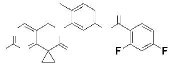 | N-(3-(2'-amino-7'-oxo-5'H-spiro[cyclopropane-1,8'-pyrido[4,3-d]pyrimidine]-6'(7'H)-yl)-4-methylphenyl)-2,4-difluorobenzamide | C | 436.1[M+H] |
| 15 | 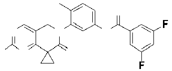 | N-(3-(2'-amino-7'-oxo-5'H-spiro[cyclopropane-1,8'-pyrido[4,3-d]pyrimidine]-6'(7'H)-yl)-4-methylphenyl)-3,5-difluorobenzamide | C | 436.1[M+H] |
| 16 | 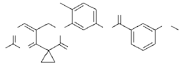 | N-(3-(2'-amino-7'-oxo-5'H-spiro[cyclopropane-1,8'-pyrido[4,3-d]pyrimidine]-6'(7'H)-yl)-4-methylphenyl)-3-methoxybenzamide | C | ¹H NMR (400 MHz, DMSO-*d₆*) δ 10.25 (s, 1H), 8.11 (s, 1H), 7.73 (s, 1H), 7.63 (d, J = 8.1 Hz, 1H), 7.53 (d, J = 7.5 Hz, 1H), 7.48 (s, 1H), 7.44 (t, J = 7.9 Hz, 1H), 7.28 (d, J = 8.3 Hz, 1H), 7.15 (dd, J = 8.1, 1.7 Hz, 1H), 6.57 (s, 2H), 4.78 (d, J = 15.3 Hz, 1H), 4.60 (d, J = 15.3 Hz, 1H), 3.83 (s, 3H), 2.07 (s, 3H), 1.62 - 1.48 (m, 4H); 430.1[M+H] |
| 17 | 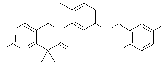 | N-(3-(2'-amino-7'-oxo-5'H-spiro[cyclopropane-1,8'-pyrido[4,3-d]pyrimidine]-6'(7'H)-yl)-4-methylphenyl)-3-chloro-2-fluoro-5-(trifluoromethyl)benzamid e | C | ¹H NMR (400 MHz, DMSO-*d₆*) δ 11.01 (s, 1H), 8.42 (s, 1H), 8.13 (s, 1H), 8.06 (s, 1H), 7.65 (d, J = 1.9 Hz, 1H), 7.57 (dd, J = 8.3, 1.7 Hz, 1H), 7.36 (d, J = 8.5 Hz, 1H), 6.80 (s, 2H), 4.80 (d, J = 15.3 Hz, 1H), 4.62 (d, J = 15.0 Hz, 1H), 2.10 (s, 3H), 1.73 - 1.44 (m, 4H); 520.10 [M+H] |
| 18 | 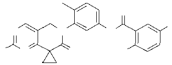 | N-(3-(2'-amino-7'-oxo-5'H-spiro[cyclopropane-1,8'-pyrido[4,3-d]pyrimidine]-6'(7'H)-yl)-4-methylphenyl)-2-fluoro-5-(trifluoromethyl)benzamid e | C | ¹H NMR (400 MHz, DMSO-*d₆*) δ 10.67 (s, 1H), 8.14 (s, 1H), 8.06 (dd, J = 6.0, 2.1 Hz, 1H), 8.02 - 7.95 (m, 1H), 7.72 (d, J = 2.1 Hz, 1H), 7.63 (t, J = 9.1 Hz, 1H), 7.51 (dd, J = 8.3, 2.2 Hz, 1H), 7.31 (d, J = 8.4 Hz, 1H), 6.89 (s, 2H), 4.79 (d, J = 15.5 Hz, 1H), 4.61 (d, J = 15.4 Hz, 1H), 2.08 (s, 3H), 1.70 - 1.48 (m, 4H); 486.15 [M+H] |
| 19 | 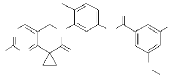 | N-(3-(2'-amino-7'-oxo-5'H-spiro[cyclopropane-1,8'-pyrido[4,3-d]pyrimidine]-6'(7'H)-yl)-4-methylphenyl)-3-methoxy-5-(trifluoromethyl)benzamid e | C | ¹H NMR (400 MHz, DMSO-*d₆*) δ 10.48 (s, 1H), 8.15 (s, 1H), 7.87 (s, 1H), 7.79 (s, 1H), 7.72 (d, J = 2.0 Hz, 1H), 7.64 (dd, J = 8.3, 2.1 Hz, 1H), 7.48 (s, 1H), 7.32 (d, J = 8.4 Hz, 1H), 6.94 (s, 2H), 4.79 (d, J = 15.5 Hz, 1H), 4.62 (d, J = 15.5 Hz, 1H), 3.92 (s, 3H), 2.08 (s, 3H), 1.68 - 1.52 (m, 4H); 498.40 [M+H] |
| 20 | 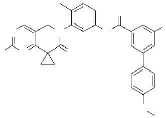 | N-(3-(2'-amino-7'-oxo-5'H-spiro[cyclopropane-1,8'-pyrido[4,3-d]pyrimidine]-6'(7'H)-yl)-4-methylphenyl)-4'-methoxy-5-(trifluoromethyl)-[1,1'-biphenyl]-3-carboxamide | C | ¹H NMR (400 MHz, DMSO-*d₆*) δ 10.58 (s, 1H), 8.48 (s, 1H), 8.25 - 8.07 (m, 3H), 7.83 (d, J = 8.3 Hz, 2H), 7.72 (s, 1H), 7.68 (d, J = 9.1 Hz, 1H), 7.33 (d, J = 8.3 Hz, 1H), 7.10 (d, J = 8.3 Hz, 2H), 6.67 (s, 2H), 4.79 (d, J = 15.5 Hz, 1H), 4.62 (d, J = 14.7 Hz, 1H), 3.83 (s, 3H), 2.09 (s, 3H), 1.67 - 1.44 (m, 4H); 574.05 [M+H] |
| 21 | 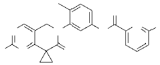 | N-(3-(2'-amino-7'-oxo-5'H-spiro[cyclopropane-1,8'-pyrido[4,3-d]pyrimidine]-6'(7'H)-yl)-4-methylphenyl)-6-(trifluoromethyl)picolinam ide | C | ¹H NMR (400 MHz, DMSO-*d₆*) δ 10.47 (s, 1H), 8.42 - 8.32 (m, 2H), 8.18 (d, J = 7.0 Hz, 1H), 8.14 (s, 1H), 7.86 - 7.75 (m, 2H), 7.33 (d, J = 8.0 Hz, 1H), 6.95 (s, 2H), 4.82 (d, J = 15.3 Hz, 1H), 4.63 (d, J = 15.3 Hz, 1H), 2.09 (s, 3H), 1.71 - 1.49 (m, 4H); 469.25 [M+H] |
| 22 | 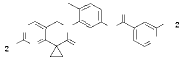 | N-(3-(2'-amino-7'-oxo-5'H-spiro[cyclopropane-1,8'-pyrido[4,3-d]pyrimidine]-6'(7'H)-yl)-4-methylphenyl)-2-(trifluoromethyl)isonicotin amide | C | ¹H NMR (400 MHz, DMSO-*d₆*) δ 10.75 (s, 1H), 9.00 (s, 1H), 8.37 (s, 1H), 8.17 (d, J = 15.6 Hz, 2H), 7.74 (s, 1H), 7.63 (d, J = 7.2 Hz, 1H), 7.34 (d, J = 7.4 Hz, 1H), 6.94 (s, 2H), 4.79 (d, J = 15.4 Hz, 1H), 4.63 (d, J = 14.4 Hz, 1H), 2.09 (s, 3H), 1.75 - 1.45 (m, 4H); 469.25 [M+H] |
| 23 | 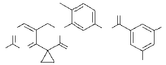 | N-(3-(2'-amino-7'-oxo-5'H-spiro[cyclopropane-1,8'-pyrido[4,3-d]pyrimidine]-6'(7'H)-yl)-4-methylphenyl)-3-hydroxy-5-(trifluoromethyl)benzamid e | C | ¹H NMR (400 MHz, DMSO-*d₆*) δ 10.55 (s, 1H), 10.43 (s, 1H), 8.14 (s, 1H), 7.73 (s, 2H), 7.61 (s, 2H), 7.30 (d, J = 7.9 Hz, 1H), 7.25 (s, 1H), 6.91 (s, 2H), 4.79 (d, J = 15.4 Hz, 1H), 4.62 (d, J = 15.2 Hz, 1H), 2.08 (s, 3H), 1.72 - 1.46 (m, 4H); 484.20 [M+H] |
| 24 | 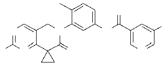 | N-(3-(2'-amino-7'-oxo-5'H-spiro[cyclopropane-1,8'-pyrido[4,3-d]pyrimidine]-6'(7'H)-yl)-4-methylphenyl)-5-(trifluoromethyl)nicotinam ide | C | ¹H NMR (400 MHz, DMSO-d₆) δ 10.69 (s, 1H), 9.37 (d, J = 1.6 Hz, 1H), 9.19 (d, J = 1.2 Hz, 1H), 8.69 (s, 1H), 8.16 (s, 1H), 7.74 (d, J = 2.0 Hz, 1H), 7.62 (dd, J = 8.3, 2.1 Hz, 1H), 7.34 (d, J = 8.4 Hz, 1H), 7.05 (s, 2H), 4.80 (d, J = 15.4 Hz, 1H), 4.63 (d, J = 15.5 Hz, 1H), 2.09 (s, 3H), 1.71 - 1.52 (m, 4H); 469.25 [M+H] |
| 25 | 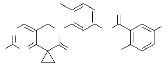 | N-(3-(2'-amino-7'-oxo-5'H-spiro[cyclopropane-1,8'-pyrido[4,3-d]pyrimidine]-6'(7'H)-yl)-4-methylphenyl)-2-chloro-5-(trifluoromethyl)benzamid e | C | ¹H NMR (400 MHz, DMSO-*d₆*) δ 10.72 (s, 1H), 8.15 (s, 1H), 8.02 (s, 1H), 7.89 (dd, J = 8.5, 1.4 Hz, 1H), 7.83 (d, J = 8.5 Hz, 1H), 7.74 (d, J = 1.4 Hz, 1H), 7.48 (dd, J = 8.3, 1.5 Hz, 1H), 7.31 (d, J = 8.4 Hz, 1H), 6.94 (s, 2H), 4.79 (d, J = 15.4 Hz, 1H), 4.61 (d, J = 15.4 Hz, 1H), 2.08 (s, 3H), 1.75 - 1.46 (m, 4H); 502.15 [M+H] |
| 26 | 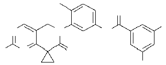 | N-(3-(2'-amino-7'-oxo-5'H-spiro[cyclopropane-1,8'-pyrido[4,3-d]pyrimidine]-6'(7'H)-yl)-4-methylphenyl)-3-chloro-5-(trifluoromethyl)benzamid e | C | ¹H NMR (400 MHz, DMSO-*d₆*) δ 10.60 (s, 1H), 8.32 (s, 1H), 8.25 (s, 1H), 8.17 (s, 1H), 8.14 (s, 1H), 7.73 (d, J = 2.1 Hz, 1H), 7.62 (dd, J = 8.3, 2.1 Hz, 1H), 7.33 (d, J = 8.4 Hz, 1H), 7.10 (s, 2H), 4.80 (d, J = 15.5 Hz, 1H), 4.63 (d, J = 15.5 Hz, 1H), 2.09 (s, 3H), 1.72 - 1.52 (m, 4H); 502.15 [M+H] |
| 27 | 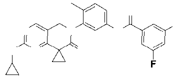 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamid e | B | ¹H NMR (400 MHz, DMSO-*d₆*) δ 10.58 (s, 1H), 8.18 (s, 1H), 8.12 (d, J = 9.2 Hz, 1H), 8.00 (d, J = 8.3 Hz, 1H), 7.72 (d, J = 1.1 Hz, 1H), 7.64 (dd, J = 8.4, 1.4 Hz, 1H), 7.43 - 7.28 (m, 2H), 4.81 (d, J = 15.4 Hz, 1H), 4.63 (d, J = 15.3 Hz, 1H), 2.66 (dt, J = 10.8, 3.6 Hz, 1H), 2.09 (s, 3H), 1.72 - 1.47 (m, 4H), 0.70 - 0.56 (m, 2H), 0.49 - 0.35 (m, 2H); 526.30 [M+H] |
| 28 | 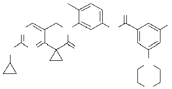 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamid e | B | ¹H NMR (400 MHz, DMSO-*d₆*) δ 10.39 (s, 1H), 8.17 (s, 1H), 7.72 (s, 1H), 7.68 (d, J = 2.0 Hz, 1H), 7.67 - 7.64 (m, 1H), 7.41 (d, J = 11.2 Hz, 2H), 7.31 (d, J = 8.4 Hz, 1H), 4.81 (d, J = 15.5 Hz, 1H), 4.63 (d, J = 15.5 Hz, 1H), 3.82 - 3.71 (m, 4H), 3.49 - 3.35 (m, 1H), 3.33 - 3.22 (m, 4H), 2.09 (s, 3H), 1.70 - 1.50 (m, J = 15.5, 8.9 Hz, 4H), 0.65 (td, J = 6.8, 4.7 Hz, 2H), 0.49 - 0.38 (m, 2H); 593.15 [M+H] |
| 29 | 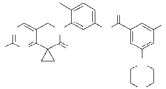 | N-(3-(2'-amino-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamid e | C | ¹H NMR (400 MHz, DMSO-*d₆*) δ 10.39 (s, 1H), 8.13 (s, 1H), 7.71 (s, 1H), 7.68 (d, J = 2.0 Hz, 1H), 7.67 - 7.62 (m, 2H), 7.39 (s, 1H), 7.31 (d, J = 8.4 Hz, 1H), 6.75 (s, 2H), 4.78 (d, J = 15.4 Hz, 1H), 4.62 (d, J = 15.4 Hz, 1H), 3.82 - 3.72 (m, 4H), 3.31 - 3.27 (m, 4H), 2.08 (s, 3H), 1.66 - 1.50 (m, 4H); 553.20 [M+H] |
| 30 | 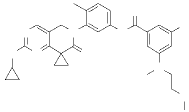 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-methoxyethyl)(methyl)ami no)-5-(trifluoromethyl)benzamid e | B | ¹H NMR (400 MHz, DMSO-*d₆*) δ 10.35 (s, 1H), 8.18 (s, 1H), 7.70 (d, J = 2.0 Hz, 1H), 7.64 (dd, J = 8.3, 2.1 Hz, 1H), 7.45 (d, J = 2.5 Hz, 2H), 7.30 (d, J = 8.5 Hz, 1H), 7.09 (s, 1H), 4.81 (d, J = 15.5 Hz, 1H), 4.63 (d, J = 15.5 Hz, 1H), 3.63 (t, J = 5.4 Hz, 2H), 3.52 (t, J = 5.4 Hz, 2H), 3.49 - 3.35 (m, 1H), 3.25 (s, 3H), 3.03 (s, 3H), 2.08 (s, 3H), 1.69 - 1.51 (m, 4H), 0.69 - 0.60 (m, 2H), 0.50 - 0.40 (m, 2H); 595.25 [M+H] |
| 31 | 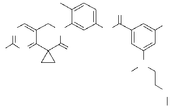 | N-(3-(2'-amino-7'-oxo-5'H-spiro[cyclopropane-1,8'-pyrido[4,3-d]pyrimidine]-6'(7'H)-yl)-4-methylphenyl)-3-((2-methoxyethyl)(methyl)ami no)-5-(trifluoromethyl)benzamid e | C | ¹H NMR (400 MHz, DMSO-*d₆*) δ 10.35 (s, 1H), 8.14 (s, 1H), 7.70 (d, J = 2.0 Hz, 1H), 7.63 (dd, J = 8.3, 2.1 Hz, 1H), 7.45 (d, J = 2.5 Hz, 2H), 7.30 (d, J = 8.4 Hz, 1H), 7.09 (s, 1H), 6.83 (s, 2H), 4.79 (d, J = 15.5 Hz, 1H), 4.62 (d, J = 15.5 Hz, 1H), 3.63 (t, J = 5.4 Hz, 2H), 3.52 (t, J = 5.4 Hz, 2H), 3.25 (s, 3H), 3.03 (s, 3H), 2.08 (s, 3H), 1.67 - 1.51 (m, 4H); 555.15 [M+H] |
| 32 | 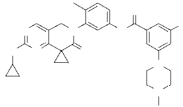 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamid e | B | ¹H NMR (400 MHz, DMSO-*d₆*) δ 10.41 (s, 1H), 9.72 (s, 1H), 8.17 (s, 1H), 7.76 (s, 1H), 7.73 (s, 1H), 7.69 - 7.62 (m, 2H), 7.51 (s, 1H), 7.36 (d, J = 2.7 Hz, 1H), 7.32 (d, J = 9.2 Hz, 1H), 4.84 (d, J = 15.7 Hz, 1H), 4.67 (d, J = 15.8 Hz, 1H), 4.05 (d, J = 11.3 Hz, 2H), 3.51 (d, J = 10.6 Hz, 2H), 3.26 - 2.95 (m, 5H), 2.83 (s, 3H), 2.04 (s, 3H), 1.86 - 1.61 (m, 4H), 0.70 - 0.59 (m, 2H), 0.49 - 0.37 (m, 2H); 606.25 [M+H] |
| 33 | 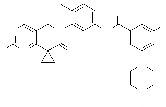 | N-(3-(2'-amino-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamid e | C | ¹H NMR (400 MHz, DMSO-*d₆*) δ 10.37 (s, 1H), 8.11 (s, 1H), 7.72 (d, J = 1.5 Hz, 1H), 7.67 (s, 1H), 7.64 (d, J = 2.2 Hz, 1H), 7.40 (s, 2H), 7.31 (d, J = 9.1 Hz, 1H), 6.57 (s, 2H), 4.77 (d, J = 15.4 Hz, 1H), 4.62 (d, J = 15.5 Hz, 1H), 4.09 (d, J = 9.5 Hz, 2H), 3.55 (d, J = 9.5 Hz, 2H), 3.16 - 3.09 (m, 4H), 2.88 (s, 3H), 2.09 (s, 3H), 1.64 - 1.48 (m, 4H); 566.10 [M+H] |
| 34 | 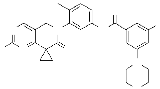 | N-(3-(2'-amino-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamid e | C | ¹H NMR (400 MHz, DMSO-*d₆*) δ 10.44 (s, 1H), 8.12 (s, 1H), 7.74 (s, 1H), 7.72 (s, 1H), 7.66 (s, 1H), 7.64 (s, 1H), 7.49 (s, 1H), 7.32 (d, J = 8.1 Hz, 1H), 4.77 (d, J = 15.4 Hz, 1H), 4.62 (d, J = 15.4 Hz, 1H), 3.61 - 3.52 (m, J = 4.5 Hz, 4H), 3.30 - 3.20 (m, 4H), 2.08 (s, 3H), 1.66 - 1.47 (m, 4H); 552.20 [M+H] |
| 35 | 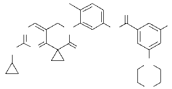 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamid e | B | ¹H NMR (400 MHz, DMSO-*d₆*) δ 10.45 (s, 1H), 8.18 (s, 1H), 7.75 (s, 1H), 7.72 (s, 1H), 7.67 (s, 1H), 7.50 (s, 1H), 7.44 (s, 1H), 7.32 (d, J = 8.3 Hz, 1H), 4.80 (d, J = 15.5 Hz, 1H), 4.64 (d, J = 15.5 Hz, 1H), 3.88 (s, 1H), 3.54 - 3.53 (m, 4H), 3.33 - 3.19 (m, 4H), 2.09 (s, 3H), 1.70 - 1.50 (m, J = 16.3, 8.3 Hz, 4H), 0.73 - 0.60 (m, 2H), 0.52 - 0.37 (m, 2H); 592.35 [M+H] |
| 36 | 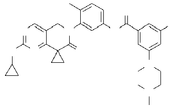 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-(dimethylamino)ethyl)(met hyl)amino)-5-(trifluoromethyl)benzamid e | B | ¹H NMR (400 MHz, DMSO-*d₆*) δ 10.42 (s, 1H), 8.17 (s, 1H), 7.70 (s, 1H), 7.65 (dd, J = 8.3, 2.0 Hz, 1H), 7.55 (s, 1H), 7.50 (s, 1H), 7.47 (s, 1H), 7.31 (d, J = 8.5 Hz, 1H), 4.79 (d, J = 15.4 Hz, 1H), 4.63 (d, J = 15.4 Hz, 1H), 3.84 - 3.73 (m, 2H), 3.30 - 3.21 (m, 2H), 3.04 (s, 6H), 3.01 (s, 3H), 2.69 - 2.62 (m, 132H), 2.33 (s, 19H), 2.08 (s, 3H), 1.99 (d, J = 8.6 Hz, 83H), 1.59 (dd, J = 15.3, 7.8 Hz, 4H), 0.64 (td, J = 6.8, 4.9 Hz, 2H), 0.46 - 0.40 (m, 2H); 608.25 [M+H] |
| 37 | 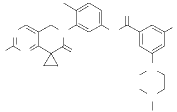 | N-(3-(2'-amino-7'-oxo-5'H-spiro[cyclopropane-1,8'-pyrido[4,3-d]pyrimidine]-6'(7'H)-yl)-4-methylphenyl)-3-((2-(dimethylamino)ethyl)(met hyl)amino)-5-(trifluoromethyl)benzamid e | C | 568.26 [M+H] |
| 38 | 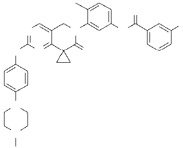 | N-(4-methyl-3-(2'-((4-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.54 (s, 1H), 9.62 (s, 1H), 9.44 (s, 1H), 8.30 (s, 2H), 8.26 (d, J = 7.9 Hz, 1H), 7.97 (d, J = 7.8 Hz, 1H), 7.89 - 7.74 (m, 2H), 7.63 (dd, J = 8.3, 2.0 Hz, 1H), 7.60 (d, J = 9.1 Hz, 2H), 7.32 (d, J = 8.5 Hz, 1H), 6.96 (d, J = 9.1 Hz, 2H), 4.87 (d, J = 15.7 Hz, 1H), 4.69 (d, J = 15.7 Hz, 1H), 3.74 (d, J = 13.4 Hz, 2H), 3.51 (d, J = 12.1 Hz, 2H), 3.18 - 3.10 (m, 2H), 2.94 - 2.80 (m, 5H), 2.10 (s, 3H), 1.72 - 1.50 (m, 4H); 642.00 [M+H] |
| 39 | 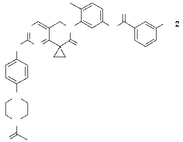 | N-(3-(2'-((4-(4-acetylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.53 (s, 1H), 9.45 (s, 1H), 8.30 (s, 2H), 8.26 (d, J = 7.9 Hz, 1H), 7.97 (d, J = 7.8 Hz, 1H), 7.79 (t, J = 7.9 Hz, 1H), 7.76 (d, J = 2.1 Hz, 1H), 7.65 (dd, J = 8.3, 2.1 Hz, 1H), 7.60 (d, J = 8.9 Hz, 2H), 7.33 (d, J = 8.5 Hz, 1H), 6.99 (d, J = 8.7 Hz, 2H), 4.87 (d, J = 15.6 Hz, 1H), 4.70 (d, J = 15.6 Hz, 1H), 3.63 - 3.58 (m, 4H), 3.16 - 3.02 (m, 4H), 2.11 (s, 3H), 2.04 (s, 3H), 1.77 - 1.54 (m, 4H); 670.05 [M+H] |
| 40 | 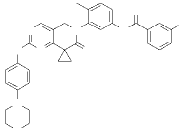 | N-(4-methyl-3-(2'-((4-morpholinophenyl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.53 (s, 1H), 9.41 (s, 1H), 8.30 (d, J = 2.3 Hz, 2H), 8.26 (d, J = 7.9 Hz, 1H), 7.97 (d, J = 7.7 Hz, 1H), 7.79 (t, J = 7.8 Hz, 1H), 7.76 (d, J = 2.1 Hz, 1H), 7.65 (dd, J = 8.3, 2.1 Hz, 1H), 7.58 (d, J = 8.9 Hz, 2H), 7.33 (d, J = 8.5 Hz, 1H), 6.94 (d, J = 8.8 Hz, 2H), 4.87 (d, J = 15.6 Hz, 1H), 4.69 (d, J = 15.7 Hz, 1H), 3.80 - 3.68 (m, 4H), 3.14 - 2.98 (m, 4H), 2.10 (s, 3H), 1.76 - 1.53 (m, 4H); 629.05 [M+H] |
| 41 | 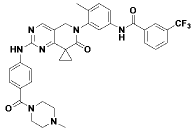 | N-(4-methyl-3-(2'-((4-(4-methylpiperazine-1-carbonyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.54 (s, 1H), 9.94 (s, 1H), 9.76 (s, 1H), 8.40 (s, 1H), 8.30 (s, 1H), 8.26 (d, J = 8.0 Hz, 1H), 7.98 (d, J = 7.8 Hz, 1H), 7.85 - 7.76 (m, 4H), 7.63 (dd, J = 8.3, 2.1 Hz, 1H), 7.43 (d, J = 8.7 Hz, 2H), 7.34 (d, J = 8.5 Hz, 1H), 4.92 (d, J = 15.9 Hz, 1H), 4.75 (d, J = 15.9 Hz, 1H), 4.22 (s, 4H), 3.18 - 2.98 (m, 4H), 2.83 (s, 3H), 2.11 (s, 3H), 1.77 - 1.63 (m, 4H); 670.00 [M+H] |
| 42 | 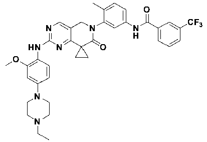 | N-(3-(2'-((4-(4-ethylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.53 (s, 1H), 9.40 (s, 1H), 8.30 (s, 1H), 8.26 (t, J = 3.8 Hz, 2H), 7.98 (d, J = 9.5 Hz, 2H), 7.83 - 7.74 (m, 3H), 7.63 (dd, J = 8.3, 2.1 Hz, 1H), 7.32 (d, J = 8.5 Hz, 1H), 6.72 (d, J = 2.5 Hz, 1H), 6.57 (dd, J = 8.8, 2.5 Hz, 1H), 4.87 (d, J = 15.7 Hz, 1H), 4.69 (d, J = 15.8 Hz, 1H), 3.85 (d, J = 12.6 Hz, 5H), 3.58 (d, J = 11.8 Hz, 2H), 3.26 - 3.19 (m, 2H), 3.13 (dd, J = 21.0, 9.4 Hz, 2H), 2.94 (t, J = 11.8 Hz, 2H), 2.10 (s, 3H), 1.69 - 1.53 (m, 4H), 1.26 (t, J = 7.3 Hz, 3H); 686.15 [M+H] |
| 43 | 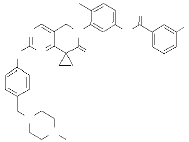 | N-(4-methyl-3-(2'-((4-((4-methylpiperazin-1-yl)methyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.55 (s, 1H), 9.76 (s, 1H), 8.37 (s, 1H), 8.30 (s, 1H), 8.26 (d, J = 7.9 Hz, 1H), 7.98 (d, J = 7.8 Hz, 1H), 7.83 - 7.77 (m, 2H), 7.75 (d, J = 8.5 Hz, 2H), 7.63 (dd, J = 8.3, 2.1 Hz, 1H), 7.36 - 7.26 (m, 3H), 4.92 (d, J = 15.8 Hz, 1H), 4.74 (d, J = 15.9 Hz, 1H), 4.37 (s, 2H), 3.67 (s, 4H), 3.24 (s, 4H), 2.89 (s, 3H), 2.11 (s, 3H), 1.82 - 1.56 (m, 4H); 656.15 [M+H] |
| 44 | 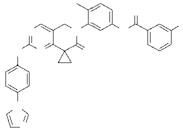 | N-(3-(2'-((4-(1H-imidazol-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.55 (s, 1H), 10.00 (s, 1H), 9.47 (s, 1H), 8.42 (s, 1H), 8.30 (s, 1H), 8.26 (d, J = 8.0 Hz, 1H), 8.18 (s, 1H), 7.98 (d, J = 8.4 Hz, 1H), 7.95 (d, J = 9.1 Hz, 2H), 7.83 (s, 1H), 7.82 - 7.77 (m, 2H), 7.72 (d, J = 9.1 Hz, 2H), 7.63 (dd, J = 8.3, 2.1 Hz, 1H), 7.34 (d, J = 8.5 Hz, 1H), 4.93 (d, J = 15.9 Hz, 1H), 4.75 (d, J = 15.9 Hz, 1H), 2.11 (s, 3H), 1.77 - 1.64 (m, 4H); 610.10 [M+H] |
| 45 | 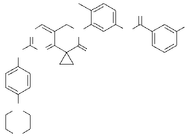 | N-(4-methyl-3-(7'-oxo-2'-((4-(piperazin-1-yl)phenyl)amino)-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.54 (s, 1H), 9.44 (s, 1H), 8.69 (s, 2H), 8.30 (s, 2H), 8.26 (d, J = 7.9 Hz, 1H), 7.98 (d, J = 7.8 Hz, 1H), 7.80 (d, J = 7.8 Hz, 1H), 7.78 (d, J = 2.1 Hz, 1H), 7.63 (dd, J = 8.3, 2.1 Hz, 1H), 7.60 (d, J = 9.1 Hz, 2H), 7.33 (d, J = 8.5 Hz, 1H), 6.96 (d, J = 9.1 Hz, 2H), 4.88 (d, J = 15.7 Hz, 1H), 4.70 (d, J = 15.7 Hz, 1H), 3.26 (s, 8H), 2.10 (s, 3H), 1.75 - 1.56 (m, 4H); 628.15 [M+H] |
| 46 | 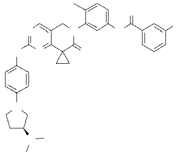 | (S)-N-(3-(2'-((4-(3-(dimethylamino)pyrrolidin -1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMF-*d₇*) δ 11.33 (s, 1H), 10.55 (s, 1H), 9.16 (s, 1H), 8.31 - 8.20 (m, 3H), 7.87 - 7.79 (m, 2H), 7.71 (t, J = 7.8 Hz, 1H), 7.63 (d, J = 8.2 Hz, 1H), 7.54 (d, J = 8.9 Hz, 2H), 7.22 (d, J = 8.4 Hz, 1H), 6.54 (d, J = 9.0 Hz, 2H), 4.85 (d, J = 15.5 Hz, 1H), 4.65 (d, J = 15.5 Hz, 1H), 4.07 (s, 1H), 3.65 - 3.52 (m, 2H), 3.42 (dd, J = 8.9, 6.1 Hz, 1H), 3.18 (dd, J = 16.9, 8.2 Hz, 1H), 2.69 (d, J = 5.2 Hz, 1H), 2.51 - 2.38 (m, 1H), 2.36 - 2.23 (m, 1H), 2.09 - 1.96 (m, 4H); 656.2 [M+H] |
| 47 | 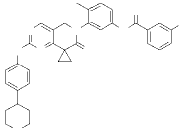 | N-(4-methyl-3-(7'-oxo-2'-((4-(piperidin-4-yl)phenyl)amino)-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, MeOD) δ 8.26 (s, 2H), 8.20 (d, J = 7.7 Hz, 1H), 7.90 (d, J = 7.9 Hz, 1H), 7.74 (t, J = 7.9 Hz, 1H), 7.64 (d, J = 8.6 Hz, 2H), 7.59 (dd, J = 8.3, 2.2 Hz, 1H), 7.36 (d, J = 8.4 Hz, 1H), 7.21 (d, J = 8.6 Hz, 2H), 4.98 (d, J = 15.6 Hz, 1H), 4.76 (d, J = 15.8 Hz, 1H), 3.50 (d, J = 13.3 Hz, 2H), 3.14 (dd, J = 13.1, 10.3 Hz, 2H), 2.88 (dd, J = 13.8, 10.5 Hz, 1H), 2.09 (d, J = 14.4 Hz, 2H), 1.96 - 1.87 (m, 2H), 1.86 - 1.71 (m, 4H); 527.5 [M+H] |
| 48 | 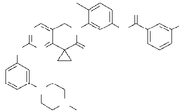 | N-(4-methyl-3-(2'-((3-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, MeOD) δ 8.25 (s, 2H), 8.20 (d, J = 7.9 Hz, 1H), 7.89 (d, J = 7.8 Hz, 1H), 7.81 (d, J = 2.2 Hz, 1H), 7.73 (t, J = 7.8 Hz, 1H), 7.58 (dd, J = 8.3, 2.1 Hz, 1H), 7.54 (s, 1H), 7.34 (d, J = 8.4 Hz, 1H), 7.22 (t, J = 8.0 Hz, 1H), 7.16 (d, J = 8.6 Hz, 1H), 6.68 (dd, J = 8.0, 1.6 Hz, 1H), 4.96 (d, J = 15.8 Hz, 1H), 4.75 (d, J = 15.8 Hz, 1H), 3.86 (d, J = 12.7 Hz, 2H), 3.62 (d, J = 11.7 Hz, 2H), 3.30 - 3.21 (m, J = 11.8 Hz, 2H), 3.07 (t, J = 12.3 Hz, 2H), 2.98 (s, 3H), 2.19 (s, 3H), 1.90 - 1.72 (m, 4H); 542.3 [M+H] |
| 49 | 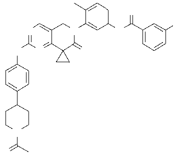 | N-(3-(2'-((4-(1-acetylpiperidin-4-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.60 - 10.47 (m, 6H), 9.56 (s, 1H), 8.32 (d, J = 9.7 Hz, 2H), 8.26 (d, J = 7.9 Hz, 1H), 7.97 (d, J = 7.8 Hz, 1H), 7.79 (t, J = 7.9 Hz, 1H), 7.76 (d, J = 2.0 Hz, 1H), 7.64 (dd, J = 13.0, 5.3 Hz, 3H), 7.33 (d, J = 8.5 Hz, 1H), 7.15 (d, J = 8.6 Hz, 2H), 4.88 (d, J = 15.7 Hz, 1H), 4.71 (d, J = 15.8 Hz, 1H), 4.51 (d, J = 13.1 Hz, 1H), 3.90 (d, J = 13.5 Hz, 1H), 3.10 (t, J = 11.9 Hz, 1H), 2.71 - 2.62 (m, 1H), 2.61 - 2.54 (m, 1H), 2.10 (s, 3H), 2.02 (s, 3H), 1.84 - 1.61 (m, 6H), 1.60 - 1.49 (m, 1H), 1.46 - 1.34 (m, 1H); 669.2 [M+H] |
| 50 | 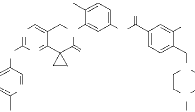 | N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-4-((4-methylpiperazin-1-yl)methyl)-3-(trifluoromethyl)benzamide | B | ¹H NMR (400 MHz, MeOD) δ 9.39 (d, J = 2.5 Hz, 1H), 8.44 (s, 1H), 8.41 (d, J = 2.5 Hz, 1H), 8.28 (s, 1H), 8.20 (d, J = 8.0 Hz, 1H), 7.99 (d, J = 8.2 Hz, 1H), 7.83 - 7.78 (m, 1H), 7.78 (s, 1H), 7.57 (dd, J = 8.3, 2.1 Hz, 1H), 7.37 (d, J = 8.4 Hz, 1H), 5.05 (d, J = 16.2 Hz, 1H), 4.85 (d, J = 6.2 Hz, 1H), 3.87 (s, 2H), 3.49 (d, J = 11.8 Hz, 2H), 3.16 (dd, J = 28.7, 17.9 Hz, 2H), 3.08 - 2.97 (m, 2H), 2.91 (s, 3H), 2.71 (s, 6H), 2.52 (t, J = 11.8 Hz, 2H), 2.21 (s, 3H), 2.16 (s, 2H), 1.94 - 1.79 (m, 4H); 671.20 [M+H] |
| 51 | 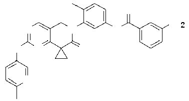 | N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.57 (s, 1H), 10.36 (s, 1H), 9.11 (s, 1H), 8.47 (s, 1H), 8.34 (d, *J* = 8.9 Hz, 1H), 8.31 (s, 1H), 8.27 (d, *J* = 7.6 Hz, 1H), 7.99 (d, *J* = 7.6 Hz, 1H), 7.84 - 7.75 (m, 2H), 7.71 (d, *J* = 7.4 Hz, 1H), 7.63 (dd, J = 8.3, 2.0 Hz, 1H), 7.34 (d, *J* = 8.4 Hz, 1H), 4.96 (d, *J* = 16.2 Hz, 1H), 4.78 (d, *J* = 16.1 Hz, 1H), 2.59 (s, 3H), 2.11 (s, 3H), 1.82 - 1.63 (m, 4H); 559.2 [M+H] |
| 52 | 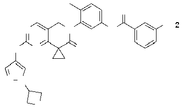 | N-(4-methyl-3-(2'-((1-(oxetan-3-yl)-1H-pyrazol-4-yl)amino)-7'-oxo-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.54 (s, 1H), 9.55 (s, 1H), 8.29 (s, 2H), 8.25 (d, J = 7.9 Hz, 1H), 7.97 (d, J = 7.8 Hz, 1H), 7.94 (s, 1H), 7.79 (t, J = 7.8 Hz, 1H), 7.75 (d, J = 2.0 Hz, 1H), 7.68 - 7.61 (m, 2H), 7.32 (d, J = 8.5 Hz, 1H), 5.55 (dd, J = 14.0, 7.2 Hz, 1H), 4.95 - 4.81 (m, J = 18.0, 6.2 Hz, 5H), 4.69 (d, J = 15.7 Hz, 1H), 2.10 (s, 3H); 590.2 [M+H] |
| 53 | 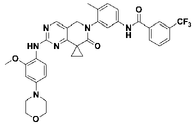 | N-(3-(2'-((2-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-d₆) δ 10.54 (s, 1H), 8.30 (s, 1H), 8.26 (d, J = 7.7 Hz, 2H), 8.02 (s, 1H), 7.98 (d, J = 7.6 Hz, 1H), 7.79 (t, J = 7.8 Hz, 1H), 7.75 (d, J = 2.0 Hz, 1H), 7.71 (d, J = 8.7 Hz, 1H), 7.64 (dd, J = 8.3, 2.1 Hz, 1H), 7.32 (d, J = 8.4 Hz, 1H), 6.67 (d, J = 2.3 Hz, 1H), 6.52 (dd, J = 8.8, 2.3 Hz, 1H), 4.86 (d, J = 15.7 Hz, 1H), 4.68 (d, J = 15.7 Hz, 1H), 3.82 (s, 3H), 3.78 - 3.70 (m, 4H), 3.14 - 3.07 (m, 4H), 2.09 (s, 3H), 1.68 - 1.53 (m, 4H); 659.05 [M+H] |
| 54 | 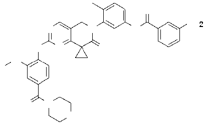 | N-(3-(2'-((2-methoxy-4-(morpholine-4-carbonyl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-d₆) δ 10.54 (s, 1H), 8.38 (s, 1H), 8.31 (s, 1H), 8.26 (d, J = 7.8 Hz, 1H), 8.23 (d, J = 8.2 Hz, 1H), 8.16 (s, 1H), 7.98 (d, J = 7.8 Hz, 1H), 7.80 (d, J = 7.8 Hz, 1H), 7.77 (d, J = 2.0 Hz, 1H), 7.65 (dd, J = 8.2, 2.1 Hz, 1H), 7.33 (d, J = 8.6 Hz, 1H), 7.09 (d, J = 1.6 Hz, 1H), 7.04 (dd, J = 8.2, 1.6 Hz, 1H), 4.92 (d, J = 16.0 Hz, 1H), 4.74 (d, J = 15.9 Hz, 1H), 3.90 (s, 3H), 3.61 (s, 4H), 3.52 (s, 4H), 2.11 (s, 3H), 1.73 - 1.62 (m, 4H); 687.05 [M+H] |
| 55 | 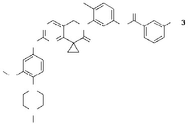 | N-(3-(2'-((3-methoxy-4-(4-methylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-d₆) δ 10.55 (s, 1H), 9.56 (s, 1H), 9.47 (s, 1H), 8.34 (s, 1H), 8.30 (s, 1H), 8.26 (d, J = 8.2 Hz, 1H), 7.98 (d, J = 7.7 Hz, 1H), 7.83 - 7.76 (m, 2H), 7.63 (dd, J = 8.3, 2.0 Hz, 1H), 7.60 (d, J = 1.9 Hz, 1H), 7.33 (d, J = 8.4 Hz, 1H), 7.18 (dd, J = 9.1, 1.8 Hz, 1H), 6.89 (d, J = 8.7 Hz, 1H), 4.93 (d, J = 15.8 Hz, 1H), 4.74 (d, J = 15.9 Hz, 1H), 3.84 (s, 3H), 3.51 (t, J = 14.5 Hz, 4H), 3.24 (t, J = 11.5 Hz, 2H), 2.95 (d, J = 11.2 Hz, 2H), 2.89 (s, 3H), 2.13 (s, 3H), 1.86 - 1.60 (m, 4H); 672.05 [M+H] |
| 56 | 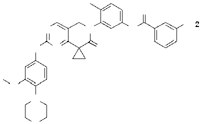 | N-(3-(2'-((3-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-d₆) δ 10.55 (s, 1H), 9.58 (s, 1H), 8.34 (s, 1H), 8.31 (s, 1H), 8.26 (d, J = 7.7 Hz, 1H), 7.98 (d, J = 8.1 Hz, 1H), 7.79 (dd, J = 12.9, 4.9 Hz, 2H), 7.68 - 7.56 (m, 2H), 7.33 (d, J = 8.5 Hz, 1H), 7.17 (dd, J = 9.4, 1.5 Hz, 1H), 6.94 (s, 1H), 4.89 (d, J = 15.8 Hz, 1H), 4.72 (d, J = 15.6 Hz, 1H), 3.83 (s, 3H), 3.75 (s, 4H), 3.00 (s, 4H), 2.11 (s, 3H), 1.79 - 1.59 (m, 4H); 659.00 [M+H] |
| 57 | 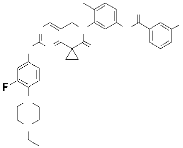 | N-(3-(2'-((4-(4-ethylpiperazin-1-yl)-3-fluorophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.54 (s, 1H), 9.79 (s, 1H), 9.46 (s, 1H), 8.35 (s, 1H), 8.30 (s, 1H), 8.26 (d, J = 7.9 Hz, 1H), 7.94 (d, J = 7.9 Hz, 1H), 7.82 - 7.73 (m, 2H), 7.68 (dd, J = 15.3, 2.2 Hz, 1H), 7.63 (dd, J = 8.3, 2.1 Hz, 1H), 7.44 (dd, J = 8.8, 1.7 Hz, 1H), 7.31 (d, J = 8.5 Hz, 1H), 7.11 - 7.02 (m, 1H), 4.90 (d, J = 15.8 Hz, 1H), 4.72 (d, J = 15.8 Hz, 1H), 3.56 (d, J = 11.6 Hz, 2H), 3.43 (d, J = 12.9 Hz, 2H), 3.29 - 3.08 (m, 4H), 2.99 (t, J = 11.7 Hz, 2H), 2.10 (s, 3H), 1.81 - 1.58 (m, 4H), 1.24 (t, J = 7.3 Hz, 3H); 674.10 [M+H] |
| 58 | 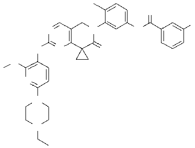 | N-(3-(2'-((6-(4-ethylpiperazin-1-yl)-2-methoxypyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 9.54 (s, 1H), 8.39 (s, 1H), 7.30 (s, 1H), 7.26 (d, J = 8.0 Hz, 1H), 7.22 (d, J = 3.0 Hz, 1H), 6.98 (d, J = 7.5 Hz, 1H), 6.86 (d, J = 8.4 Hz, 1H), 6.82 - 6.72 (m, 2H), 6.62 (dd, J = 8.3, 2.0 Hz, 1H), 6.32 (d, J = 8.6 Hz, 1H), 5.49 (d, J = 8.6 Hz, 1H), 4.89 (d, J = 15.9 Hz, 1H), 4.70 (d, J = 15.9 Hz, 1H), 4.37 (d, J = 12.2 Hz, 2H), 3.85 (s, 3H), 3.58 (d, J = 10.7 Hz, 2H), 3.25 - 2.98 (m, 6H), 2.09 (s, 3H), 1.78 - 1.59 (m, 4H), 1.25 (t, J = 7.3 Hz, 3H); 687.95 [M+H] |
| 59 | 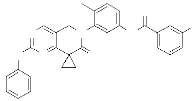 | N-(4-methyl-3-(7'-oxo-2'-(phenylamino)-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.58 (s, 1H), 9.85 (s, 1H), 8.39 (s, 1H), 8.33 (s, 1H), 8.29 (d, J = 7.9 Hz, 1H), 7.97 (d, J = 7.8 Hz, 1H), 7.80 (dd, J = 13.5, 5.0 Hz, 2H), 7.72 (d, J = 7.8 Hz, 2H), 7.68 (dd, J = 8.3, 2.0 Hz, 1H), 7.37 - 7.29 (m, 3H), 7.00 (t, J = 7.3 Hz, 1H), 4.94 (d, J = 15.7 Hz, 1H), 4.75 (d, J = 15.8 Hz, 1H), 2.13 (s, 3H), 1.81 - 1.66 (m, 4H); 544.10 [M+H] |
| 60 | 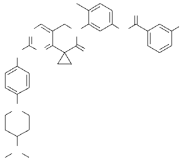 | N-(3-(2'-((4-(4-(dimethylamino)piperidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.55 (s, 1H), 9.42 (s, 1H), 8.35 - 8.21 (m, 3H), 7.98 (d, J = 8.0 Hz, 1H), 7.83 - 7.75 (m, 2H), 7.63 (dd, J = 8.3, 2.0 Hz, 1H), 7.56 (d, J = 9.0 Hz, 2H), 7.33 (d, J = 8.5 Hz, 1H), 6.94 (d, J = 9.0 Hz, 2H), 4.87 (d, J = 15.6 Hz, 1H), 4.70 (d, J = 15.7 Hz, 1H), 3.75 (d, J = 12.5 Hz, 2H), 3.49 - 3.35 (m, 3H), 2.79 (d, J = 4.9 Hz, 6H), 2.27 (d, J = 11.0 Hz, 2H), 2.10 (s, 3H), 2.00 (d, J = 10.9 Hz, 2H), 1.78 - 1.59 (m, 4H); 670.25 [M+H] |
| 61 | 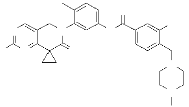 | N-(3-(2'-amino-7'-oxo-5'H-spiro[cyclopropane-1,8'-pyrido[4,3-d]pyrimidine]-6'(7'H)-yl)-4-methylphenyl)-4-((4-methylpiperazin-1-yl)methyl)-3-(trifluoromethyl)benzamid e | C | ¹H NMR (400 MHz, MeOD) δ 8.29 (s, 1H), 8.19 (d, J = 8.1 Hz, 1H), 8.13 (s, 1H), 7.98 (d, J = 8.2 Hz, 1H), 7.78 (d, J = 2.0 Hz, 1H), 7.57 (dd, J = 8.3, 2.1 Hz, 1H), 7.35 (d, J = 8.4 Hz, 1H), 4.95 (s, 1H), 4.73 (d, J = 15.8 Hz, 1H), 3.87 (s, 2H), 3.50 (d, J = 12.0 Hz, 2H), 3.19 (t, J = 11.3 Hz, 2H), 3.05 (d, J = 12.8 Hz, 2H), 2.92 (s, 3H), 2.51 (t, J = 12.0 Hz, 2H), 2.19 (s, 3H), 1.93 - 1.76 (m, 4H); 580.3 [M+H] |
| 62 | 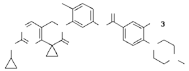 | N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamid e | B | ¹H NMR (400 MHz, DMSO-d₆) δ 10.49 (s, 1H), 9.76 (s, 1H), 8.30 (s, 1H), 8.29 - 8.25 (m, 1H), 8.18 (s, 1H), 7.72 (d, J = 1.9 Hz, 1H), 7.70 (d, J = 8.5 Hz, 1H), 7.64 (dd, J = 8.3, 2.0 Hz, 1H), 7.46 (s, 1H), 7.32 (d, J = 8.6 Hz, 1H), 4.91 (d, J = 15.9 Hz, 1H), 4.72 (d, J = 15.7 Hz, 1H), 3.55 (d, J = 8.4 Hz, 2H), 3.26 - 3.10 (m, 7H), 2.91 (s, 3H), 2.09 (s, 3H), 1.91 - 1.72 (m, 4H), 0.70 - 0.61 (m, 2H), 0.52 - 0.40 (m, 2H); 606.55 [M+H] |
| 63 | 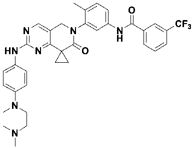 | N-(3-(2'-((4-((2-(dimethylamino)ethyl)(met hyl)amino)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | ¹H NMR (400 MHz, DMSO-*d₆*) δ 10.56 (s, 1H), 10.00 (s, 1H), 9.60 (s, 1H), 8.31 (d, J = 10.5 Hz, 2H), 8.26 (d, J = 7.8 Hz, 1H), 7.93 (d, J = 7.8 Hz, 1H), 7.82 (d, J = 2.0 Hz, 1H), 7.76 (t, J = 7.8 Hz, 1H), 7.62 (dd, J = 8.3, 2.0 Hz, 1H), 7.56 (d, J = 8.5 Hz, 2H), 7.31 (d, J = 8.5 Hz, 1H), 6.99 (d, J = 8.7 Hz, 2H), 4.90 (d, J = 15.6 Hz, 1H), 4.71 (d, J = 15.7 Hz, 1H), 3.75 - 3.60 (m, 2H), 3.30 - 3.15 (m, 2H), 2.96 (s, 3H), 2.83 (s, 6H), 2.10 (s, 3H), 1.85 - 1.60 (m, 4H); 644.10 [M+H] |
| 64 | 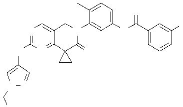 | N-(4-methyl-3-(7'-oxo-2'-((1-(2,2,2-trifluoroethyl)-1H-pyrazol-4-yl)amino)-5'H-spiro[cyclopropane-1,8'-pyrido[4,3-d]pyrimidine]-6'(7'H)-yl)phenyl)-3-(trifluoromethyl)benzamid e | A | ¹H NMR (400 MHz, DMSO-*d₆*) δ 10.55 (s, 1H), 9.63 (s, 1H), 8.31 (s, 2H), 8.27 (d, J = 7.9 Hz, 1H), 7.99 (d, J = 7.7 Hz, 2H), 7.80 (t, J = 8.0 Hz, 1H), 7.77 (d, J = 1.9 Hz, 1H), 7.65 (dd, J = 8.3, 2.0 Hz, 1H), 7.61 (s, 1H), 7.33 (d, J = 8.4 Hz, 1H), 5.13 (dd, J = 18.2, 9.4 Hz, 2H), 4.88 (d, J = 15.6 Hz, 1H), 4.71 (d, J = 15.6 Hz, 1H), 2.11 (s, 3H), 1.80 - 1.55 (m, 4H); 616.00 [M+H] |

**[Table 2]**

| Example No. | Structural formula | Compound name | scheme | ¹H NMR; MS[M+H] |
|---|---|---|---|---|
| 65 | 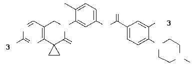 | N-(3-(2'-amino-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide | C | ¹H NMR (400 MHz, DMSO-*d₆*) δ 10.49 (s, 1H), 9.76 (s, 1H), 8.30 (s, 1H), 8.29 - 8.24 (m, 1H), 8.14 (s, 1H), 7.72 (d, J = 2.0 Hz, 1H), 7.70 (d, J = 8.5 Hz, 1H), 7.64 (dd, J = 8.2, 2.0 Hz, 1H), 7.32 (d, J = 8.5 Hz, 1H), 6.80 (s, 2H), 4.78 (d, J = 15.4 Hz, 1H), 4.62 (d, J = 15.5 Hz, 1H), 3.31 - 3.04 (m, J = 20.4, 9.9 Hz, 8H), 2.91 (d, J = 2.9 Hz, 3H), 2.09 (s, 3H), 1.72 - 1.44 (m, 4H); 566.25 [M+H]+ |
| 66 | 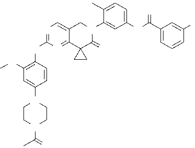 | N-(3-(2'-((4-(4-acetylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide | A | ¹H NMR (400 MHz, DMSO-*d₆*) δ 10.55 (s, 1H), 8.30 (s, 1H), 8.26 (s, 1H), 8.01 (s, 1H), 7.98 (d, J = 8.0 Hz, 1H), 7.79 (t, J = 7.9 Hz, 1H), 7.75 (s, 1H), 7.72 (s, 1H), 7.67 - 7.62 (m, 1H), 7.32 (d, J = 8.4 Hz, 1H), 6.69 (s, 1H), 6.53 (d, J = 8.5 Hz, 1H), 4.86 (d, J = 15.5 Hz, 1H), 4.69 (d, J = 15.7 Hz, 1H), 3.82 (s, 3H), 3.18 - 3.11 (m, 4H), 3.11 - 3.05 (m, 4H), 2.24 (s, 3H), 1.92 (s, 3H), 1.70 - 1.52 (m, 4H); 699.95[M+H] |
| 67 | 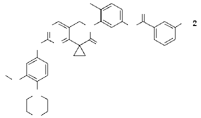 | N-(3-(2'-((3-methoxy-4-(piperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide | A | ¹H NMR (400 MHz, DMSO-*d₆*) δ 10.58 (s, 1H), 9.56 (s, 1H), 8.82 (s, 2H), 8.33 (s, 1H), 8.30 (s, 1H), 8.26 (d, J = 7.9 Hz, 1H), 7.97 (d, J = 7.8 Hz, 1H), 7.82 - 7.75 (m, J = 8.5, 4.9 Hz, 1H), 7.69 - 7.62 (m, 1H), 7.60 (s, 1H), 7.33 (d, J = 8.4 Hz, 1H), 7.16 (d, J = 8.4 Hz, 1H), 6.87 (d, J = 8.6 Hz, 1H), 4.88 (d, J = 15.8 Hz, 1H), 4.71 (d, J = 15.7 Hz, 1H), 3.81 (s, 3H), 3.22 (s, 4H), 3.09 (s, 4H), 2.10 (s, 3H), 1.75 - 1.59 (m, 4H); 658.6 [M+H] |
| 68 | 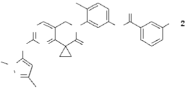 | N-(3-(2'-((1,3-dimethyl-1H-pyrazol-5-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide | A | ¹H NMR (400 MHz, DMSO-*d₆*) δ 10.57 (s, 1H), 9.08 (s, 1H), 8.46 (s, 1H), 8.30 (s, 1H), 8.26 (d, J = 7.8 Hz, 1H), 7.98 (d, J = 7.2 Hz, 1H), 7.82 - 7.79 (m, 1H), 7.71 - 7.57 (m, J = 8.3 Hz, 2H), 7.34 (d, J = 8.5 Hz, 1H), 4.95 (d, J = 16.0 Hz, 1H), 4.77 (d, J = 16.2 Hz, 1H), 3.32 (s, 14H), 2.57 (s, 3H), 2.11 (s, 3H), 1.80 - 1.64 (m, 4H); 560.0 [M-H] |
| 69 | 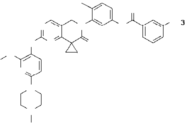 | N-(3-(2'-((2-methoxy-6-(4-methylpiperazin-1-yl)pyrimidin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide | A | ¹H NMR (400 MHz, DMSO-*d₆*) δ 10.55 (s, 1H), 8.30 (s, 1H), 8.26 (d, J = 7.9 Hz, 1H), 8.20 (s, 1H), 8.12 (s, 1H), 7.97 (d, J = 7.7 Hz, 1H), 7.79 (t, J = 7.8 Hz, 1H), 7.74 (s, 1H), 7.67 - 7.61 (m, 1H), 7.32 (d, J = 8.4 Hz, 1H), 6.33 (d, J = 8.5 Hz, 1H), 4.84 (d, J = 15.6 Hz, 1H), 4.66 (d, J = 15.6 Hz, 1H), 3.81 (s, 3H), 2.54 - 2.42 (m, 4H), 2.43 - 2.37 (m, 4H), 2.21 (s, 3H), 1.89 (s, 3H), 1.69 - 1.49 (m, J = 34.3, 23.5, 6.8 Hz, 4H); 673.05 [M+H] |
| 70 | 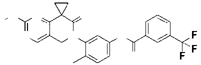 | N-(3-(2'-(hydroxyamino)-7'-oxo-5'H-spiro[cyclopropane-1,8'-pyrido[4,3-d]pyrimidine]-6'(7'H)-yl)-4-methylphenyl)-3-(trifluoromethyl)benzamide | B | 484.5 [ M+1] |
| 71 | 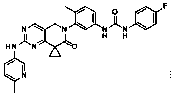 | 1-(4-Fluorophenyl)-3-(4-methyl-3-(2'-((6-methylpyrimidin-3-yl)amino)-7'-oxo-5'H-spiro[cyclopropane-1,8'-pyrido[4,3-d]pyrimidine]-6'(7'H)-yl)phenyl)urea | A | ¹H NMR (400 MHz, MeOD) δ 8.73 (d, J = 2.5 Hz, 1H), 8.25 (s, 1H), 8.05 (dd, J = 8.5, 2.6 Hz, 1H), 7.51 (s, 1H), 7.44 - 7.33 (m, 2H), 7.28 - 7.17 (m, J = 8.7 Hz, 3H), 7.05 - 6.95 (m, 2H), 4.91 (d, J = 15.8 Hz, 1H), 4.71 (d, J = 15.9 Hz, 1H), 2.47 (s, 3H), 2.16 (s, 1H), 2.13 (s, 3H), 1.83 - 1.72 (m, 4H); 524.20 [M+H] |
| 72 | 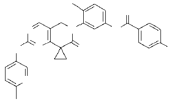 | 4-chloro-N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)benzamide | A | ¹H NMR (400 MHz, DMSO) δ 10.41 (s, 1H), 9.12 (s, 1H), 8.47 (s, 1H), 8.35 (d, *J* = 7.2 Hz, 1H), 7.98 (d, *J* = 8.5 Hz, 2H), 7.82 (s, 1H), 7.72 (d, *J* = 7.5 Hz, 1H), 7.66 - 7.54 (m, 3H), 7.32 (d, *J* = 7.5 Hz, 1H), 4.95 (d, *J =* 15.3 Hz, 1H), 4.77 (d, *J* = 15.9 Hz, 1H), 2.60 (s, 3H), 2.10 (s, 3H), 1.82 - 1.64 (m, 4H); 526.15 [M+H] |
| 73 | 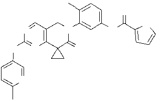 | N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)thiophene-2-carboxamide | A | ¹H NMR (400 MHz, MeOD) δ 9.42 (d, *J* = 2.3 Hz, 1H), 8.47 - 8.39 (m, 2H), 7.92 (d, *J* = 3.8 Hz, 1H), 7.83 - 7.79 (m, 2H), 7.76 (d, *J* = 4.2 Hz, 1H), 7.54 (d, *J=* 8.3 Hz, 1H), 7.37 (d, *J* = 8.4 Hz, 1H), 7.21 (dd, *J* = 4.9, 3.9 Hz, 1H), 5.07 (d, *J* = 16.1 Hz, 2H), 4.85 (d, *J* = 16.2 Hz, 1H), 2.73 (d, *J* = 5.7 Hz, 6H), 1.95 - 1.77 (m, 4H); 497.20 [M+H] |
| 74 | 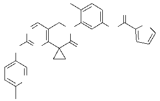 | N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)furan-2-carboxamide | A | ¹H NMR (400 MHz, MeOD) δ 9.44 (d, *J* = 2.5 Hz, 1H), 8.44 (dd, *J* = 9.4, 3.1 Hz, 2H), 7.84 - 7.79 (m, 2H), 7.78 (s, 1H), 7.60 - 7.55 (m, 1H), 7.37 (d, *J* = 8.5 Hz, 1H), 7.29 (d, *J=* 3.4 Hz, 1H), 6.67 (dd, *J* = 3.4, 1.7 Hz, 1H), 5.07 (d, *J* = 15.9 Hz, 1H), 4.85 (d, *J =* 16.0 Hz, 1H), 2.74 (d, *J* = 5.4 Hz, 5H), 1.95 - 1.81 (m, 4H); 481.20 [M+H] |

**[Table 3]**

| Exam ple No. | Structural formula | Compound name | scheme | ¹H NMR; MS[M+H] |
|---|---|---|---|---|
| 75 | 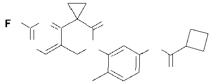 | N-(3-(2'-amino-7'-oxo-5'H-spiro[cyclopropane-1,8'-pyrido[4,3-d]pyrimidine]-6'(7'H)-yl)-4-methylphenyl)cyclobutane carboxamide | C | 378.05 [M+H] |
| 76 | 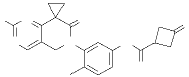 | N-(3-(2'-amino-7'-oxo-5'H-spiro[cyclopropane-1,8'-pyrido[4,3-d]pyrimidine]-6'(7'H)-yl)-4-methylphenyl)-3-oxocyclobutane-1-carboxamide | C | 392.10 [M+H] |
| 77 | 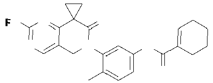 | N-(3-(2'-amino-7'-oxo-5'H-spiro[cyclopropane-1,8'-pyrido[4,3-d]pyrimidine]-6'(7'H)-yl)-4-methylphenyl)cyclohex-1-ene-1-carboxamide | C | 404.05 [M+H] |
| 78 | 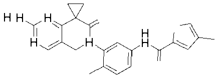 | N-(3-(2'-amino-7'-oxo-5'H-spiro[cyclopropane-1,8'-pyrido[4,3-d]pyrimidine]-6'(7'H)-yl)-4-methylphenyl)-4-methylthiophene-2-carboxamide | C | 420.10 [M+H] |
| 79 | 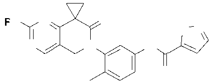 | N-(3-(2'-amino-7'-oxo-5'H-spiro[cyclopropane-1,8'-pyrido[4,3-d]pyrimidine]-6'(7'H)-yl)-4-methylphenyl)isoxazole-5-carboxamide | C | 391.05 [M+H] |
| 80 | 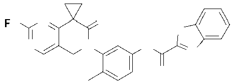 | N-(3-(2'-amino-7'-oxo-5'H-spiro[cyclopropane-1,8'-pyrido[4,3-d]pyrimidine]-6'(7'H)-yl)-4-methylphenyl)benzo[d]thia zole-2-carboxamide | C | 457.05 [M+H] |
| 81 | 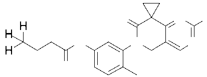 | N-(3-(2'-amino-7'-oxo-5'H-spiro[cyclopropane-1,8'-pyrido[4,3-d]pyrimidine]-6'(7'H)-yl)-4-methylphenyl)-4,4,4-trifluorobutanamide | C | 420.10 [M+H] |
| 82 | 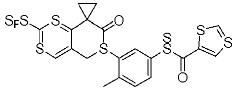 | N-(3-(2'-amino-7'-oxo-5'H-spiro[cyclopropane-1,8'-pyrido[4,3-d]pyrimidine]-6'(7'H)-yl)-4-methylphenyl)thiazole-5-carboxamide | C | 407.05 [M+H] |
| 83 | 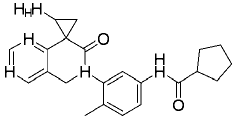 | N-(3-(2-amino-7'-oxo-5'H-spiro[cyclopropane-1,8'-pyrido[4,3-d]pyrimidine]-6'(7'H)-yl)-4-methylphenyl)cyclopentan ecarboxamide | C | 394.15 [M+H] |
| 84 | 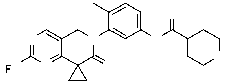 | N-(3-(2-amino-7'-oxo-5'H-spiro[cyclopropane-1,8'-pyrido[4,3-d]pyrimidine]-6'(7'H)-yl)-4-methylphenyl)tetrahydro-2H-pyran-4-carboxamide | C | 408.10 [M+H] |
| 85 | 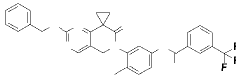 | N-(3-(2'-(benzylamino)-7'-oxo-5'H-spiro[cyclopropane-1,8'-pyrido[4,3-d]pyrimidine]-6'(7'H)-yl)-4-methylphenyl)-3-(trifluoromethyl)benzamid e | B | 558 [M+H] |
| 86 | 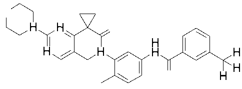 | N-(4-methyl-3-(2'-morpholino-7'-oxo-5'H-spiro[cyclopropane-1,8'-pyrido[4,3-d]pyrimidine]-6'(7'H)-yl)phenyl)-3-(trifluoromethyl)benzamid e | B | 538 [M+H] |
| 87 | 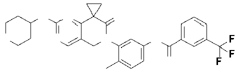 | N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-3-(trifluoromethyl)benzamid e | B | 552 [M+H] |
| 88 | 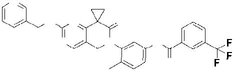 | N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-3-(trifluoromethyl)benzamid e | B | 559 [M+H] |
| 89 | 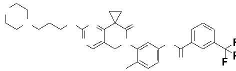 | N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-3-(trifluoromethyl)benzamid e | B | 595 [M+H] |
| 90 | 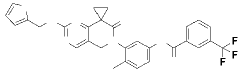 | N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | B | 548 [M+H] |
| 91 | 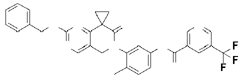 | N-(3-(2'-(benzylamino)-7'-oxo-5'H-spiro[cyclopropane-1,8'-pyrido[4,3-d]pyrimidine]-6'(7'H)-yl)-4-methylphenyl)-5-(trifluoromethyl)nicotinam ide | B | 559 [M+H] |
| 92 | 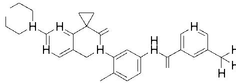 | N-(4-methyl-3-(2'-morpholino-7'-oxo-5'H-spiro[cyclopropane-1,8'-pyrido[4,3-d]pyrimidine]-6'(7'H)-yl)phenyl)-5-(trifluoromethyl)nicotinam ide | B | 539 [M+H] |
| 93 | 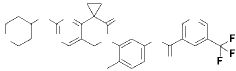 | N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-5-(trifluoromethyl)nicotinam ide | B | 553 [M+H] |
| 94 | 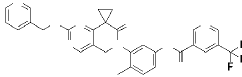 | N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-5-(trifluoromethyl)nicotinam ide | B | 560 [M+H] |
| 95 | 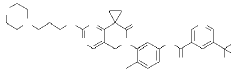 | N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-5-(trifluoromethyl)nicotinam ide | B | 596 [M+H] |
| 96 | 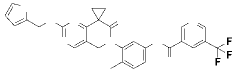 | N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinam ide | B | 549 [M+H] |
| 97 | 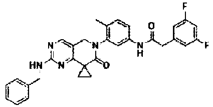 | N-(3-(2'-(benzylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide | B | 540 [M+H] |
| 98 | 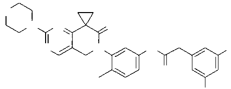 | 2-(3,5-difluorophenyl)-N-(4-methyl-3-(2'-morpholino-7'-oxo-5'H-spiro[cyclopropane-1,8'-pyrido[4,3-d]pyrimidine]-6'(7'H)-yl)phenyl)acetamide | B | 520 [M+H] |
| 99 | 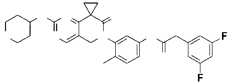 | 2-(3,5-difluorophenyl)-N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)acetamide | B | 534 [M+H] |
| 100 | 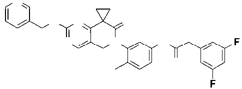 | 2-(3,5-difluorophenyl)-N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)acetamide | B | 541 [M+H] |
| 101 | 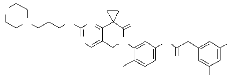 | 2-(3,5-difluorophenyl)-N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)acetamide | B | 577 [M+H] |
| 102 | 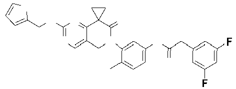 | 2-(3,5-difluorophenyl)-N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)acetamide | B | 530 [M+H] |
| 103 | 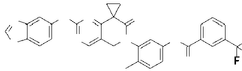 | N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | 601 [M+H] |
| 104 | 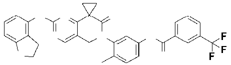 | N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamid e | A | 586 [M+H] |
| 105 | 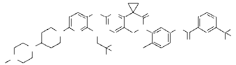 | N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamid e | A | 824 [M+H] |
| 106 | 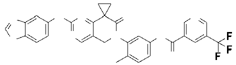 | N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-5-(trifluoromethyl)nicotinam ide | A | 602 [M+H] |
| 107 | 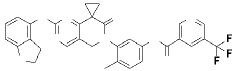 | N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinam ide | A | 587 [M+H] |
| 108 | 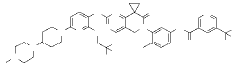 | N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-5-(trifluoromethyl)nicotinam ide | A | 825 [M+H] |
| 109 | 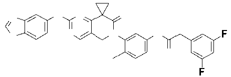 | N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide | A | 583 [M+H] |
| 110 | 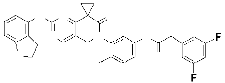 | 2-(3,5-difluorophenyl)-N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)acetamide | A | 568 [M+H] |
| 111 | 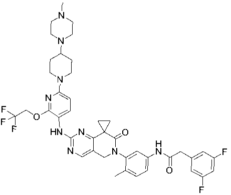 | 2-(3,5-difluorophenyl)-N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)acetamide | A | 806 [M+H] |

**[Table 4]**

| Example No. | Structural formula | Compound name | scheme | ¹H NMR; MS[M+H] |
|---|---|---|---|---|
| 112 | 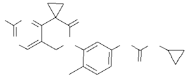 | 1-(3-(2'-amino-7'-oxo-5'H-spiro[cyclopropane-1,8'-pyrido[4,3-d]pyrimidine]-6'(7'H)-yl)-4-methylphenyl)-3-cyclopropylurea | D | ¹H NMR (400 MHz, DMSO-d₆) δ 8.30 (s, 1H), 8.11 (s, 1H), 7.42 (d, J = 2.1 Hz, 1H), 7.25 - 7.18 (m, 1H), 7.14 (d, J = 8.4 Hz, 1H), 6.67 (s, 2H), 6.43 (s , 1H), 4.74 (d, J = 15.6 Hz, 1H), 4.56 (d, J = 15.4 Hz, 1H), 2. 57 (s, 1H), 2.01 (s, 2H), 1.70 - 1.44 (m, 4H), 0.63 (dt, J = 6.3 , 4.8 Hz, 2H), 0.40 (dt, J = 7.3, 4.8 Hz, 2H); 379.10 [M+1] |
| 113 | 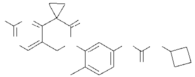 | 1-(3-(2'-amino-7'-oxo-5'H-spiro[cyclopropane-1,8'-pyrido[4,3-d]pyrimidine]-6'(7'H)-yl)-4-methylphenyl)-3-cyclobutylurea | D | 1H NMR (400 MHz, DMSO-d ) δ 8.33 (s, 1H), 8.13 (s, 1H), 7 41 (s, 1H), 7.14 (s, 1H), 6.90 (s, 3H), 6.43 (d, J = 8.3 Hz, 1H), 4.75 (d, J = 15.3 Hz, 1H), 4.56 (d, J = 15.6 Hz, 1H), 4.10 (dd, J= 16.0, 7.8 Hz, 1H), 2.18 ( td, J = 10.2, 2.3 Hz, 2H), 2.00 (s, 3H), 1.91 1.78 (m, 2H), 1.6 8-1.37 (m, 6H); 393.15[M+1] |
| 114 | 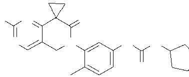 | 1-(3-(2'-amino-7'-oxo-5'H-spiro[cyclopropane-1,8'-pyrido[4,3-d]pyrimidine]-6'(7'H)-yl)-4-methylphenyl)-3-cyclopentylurea | D | 1H NMR (400 MHz, DMSO-d 6) δ 8.27 (s, 1H), 8.12 (s, 1H), 7.43 (s, 1H), 7.13 (s, 1H), 6.7 2 (s, 3H), 6.18 (d, J = 6.9 Hz, 1H), 4.74 (d, J = 15.3 Hz, 1H), 4.55 (d, J = 15.4 Hz, 1H), 3.91 (dd, J = 13.5, 6.3 Hz, 1H), 2.00 (s, 3H), 1.87 - 1.77 (m, 2H), 1.72 1.45 (m, 8H), 1.40 - 1. 31 (m, 2H); 407.10[M+1] |
| 115 | 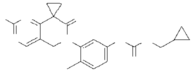 | 1-(3-(2'-amino-7'-oxo-5'H-spiro[cyclopropane-1,8'-pyrido[4,3-d]pyrimidine]-6'(7'H)-yl)-4-methylphenyl)-3-(cyclopropylmethyl)urea | D | 393.10 [M+H] |

**[Table 5]**

| Exa mple No. | Structural formula | Compound name | scheme | ¹H NMR; MS[M+H] |
|---|---|---|---|---|
| 116 | 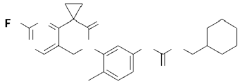 | 1-(3-(2'-amino-7'-oxo-5'H-spiro[cyclopropane-1,8'-pyrido[4,3-d]pyrimidine]-6'(7'H)-yl)-4-methylphenyl)-3-(cyclohexylmethyl)urea | D | 435.15 [M+H] |
| 117 | 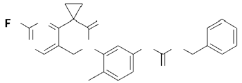 | 1-(3-(2'-amino-7'-oxo-5'H-spiro[cyclopropane-1,8'-pyrido[4,3-d]pyrimidine]-6'(7'H)-yl)-4-methylphenyl)-3-benzylurea | D | 429.15 [M+H] |
| 118 | 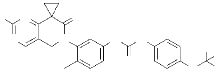 | 1-(3-(2'-amino-7'-oxo-5'H-spiro[cyclopropane-1,8'-pyrido[4,3-d]pyrimidine]-6'(7'H)-yl)-4-methylphenyl)-3-(4 (trifluoromethoxy)phenyl )urea | D | 499.10 [M+H] |
| 119 | 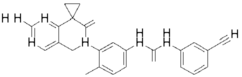 | 1-(3-(2'-amino-7'-oxo-5'H-spiro[cyclopropane-1,8'-pyrido[4,3-d]pyrimidine]-6'(7'H)-yl)-4-methylphenyl)-3-(3-cyanophenyl)urea | D | 440.10 [M+H] |
| 120 | 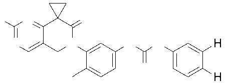 | 1-(3-(2'-amino-7'-oxo-5'H-spiro[cyclopropane-1,8'-pyrido[4,3-d]pyrimidine]-6'(7'H)-yl)-4-methylphenyl)-3-(3,4-difluorophenyl)urea | D | 451.10 [M+H] |
| 121 | 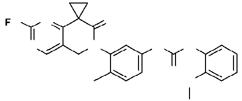 | 1-(3-(2'-amino-7'-oxo-5'H-spiro[cyclopropane-1,8'-pyrido[4,3-d]pyrimidine]-6'(7'H)-yl)-4-methylphenyl)-3-(2-methoxyphenyl)urea | D | ¹H NMR (400 MHz, DMSO-d₆) δ 9.35 (s, 1H), 8.24 (s, 1H), 8.15-8.07 (m, 2H), 7.51 (s, 1H), 7.21 (s, 2H), 7.02 (dd, J = 8.1, 1.1 Hz, 1H), 6.98-6.92 (m, 1H), 6.91-6.85 (m, 1H), 6.65 (s, 2H), 4.79 (d, J = 15.2 Hz, 1H), 4.58 (d, J = 15.2 Hz, 1H), 2.03 (s, 3H), 1.65-1.45 (m, 4H); 445.10 [M+1] |
| 122 | 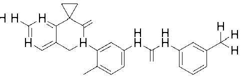 | 1-(3-(2'-amino-7'-oxo-5'H-spiro[cyclopropane-1,8'-pyrido[4,3-d]pyrimidine]-6'(7'H)-yl)-4-methylphenyl)-3-(3-(trifluoromethyl)phenyl)u rea | D | ¹H NMR (400 MHz, DMSO-d₆) δ 9.11 (s, 1H), 8.84 (s, 1H), 8.12 (s, 1H), 8.05 (s, 1H), 7.62-7.44 (m, J = 6.6, 2.9, 1.2 Hz, 3H), 7.31 (d, J = 7.1 Hz, 1H), 7.28-7.16 (m, 2H), 6.63 (s, 2H), 4.80 (d, J = *15.0* Hz, 1H), 4.58 (d, J = 15.5 Hz, 1H), 2.08 (s, 3H), 1.66-1.45 (m, 4H); 483.15[M+1] |
| 123 | 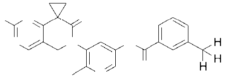 | N-(5-(2'-amino-7'-oxo-5'H-spiro[cyclopropane-1,8'-pyrido[4,3-d]pyrimidine]-6'(7'H)-yl)-6-methylpyridin-3-yl)-3-(trifluoromethyl)benzami de | E | 469.10 [M+H] |
| 124 | 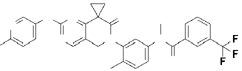 | N-methyl-N-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(trifluoromethyl)benzami de | F | 573 [M+H] |
| 125 | 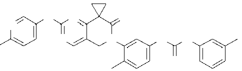 | 1-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(m-tolyl)urea | D | 520 [M+H] |
| 126 | 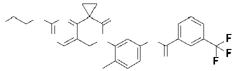 | N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzami de | B | ¹H NMR (400 MHz, MeOD) δ 8.25 (s, 1H), 8.19 (d, J = 7.8 Hz, 1H), 8.14 (s, 1H), 7.89 (d, J = 7.8 Hz, 1H), 7.78 (d, J = 2.0 Hz, 1H), 7.73 (t, J = 7.8 Hz, 1H), 7.57 (dd, J = 8.3, 2.0 Hz, 1H), 7.34 (d, J = 8.4 Hz, 1H), 4.94 (d, J = 15.7 Hz, 1H), 4.72 (d, J = 15.7 Hz, 1H), 3.72 (t, J = 5.6 Hz, 2H), 3.54 (t, J = 5.6 Hz, 2H), 2.18 (s, 3H), 1.95 - 1.74 (m, 3H); 512.1 [M+H] |
| 127 | 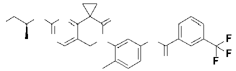 | (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzami de | B | 526 [M+H] |
| 128 | 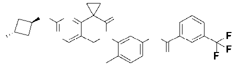 | N-(3-(2'-(((1r,3r)-3-hydroxycyclobutyl)amino )-7'-oxo-5'H-spiro[cyclopropane-1,8'-pyrido[4,3-d]pyrimidine]-6'(7'H)-yl)-4-methylphenyl)-3-(trifluoromethyl)benzami de | B | 538 [M+H] |

**[Table 6]**

| No. | Structural formula | Compound name | scheme | ¹H NMR; MS[M+H] |
|---|---|---|---|---|
| 129 | 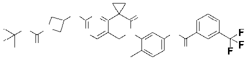 | tert-butyl 3-((6'-(2-methyl-5-(3-(trifluoromethyl)benzami do)phenyl)-7'-oxo-6',7'-dihydro-5'H-spiro[cyclopropane-1,8'-pyrido[4,3-d]pyrimidin]-2'-yl)amino)azetidine-1-carboxylate | B | 623 [M+H] |
| 130 | 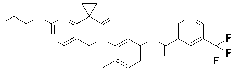 | N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotina mide | B | 513 [M+H] |
| 131 | 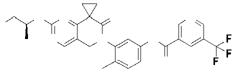 | (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotina mide | B | 527 [M+H] |
| 132 | 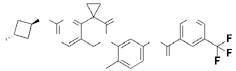 | N-(3-(2'-(((1r,3r)-3-hydroxycyclobutyl)amin o)-7'-oxo-5'H-spiro[cyclopropane-1,8'-pyrido[4,3-d]pyrimidine]-6'(7'H)-yl)-4-methylphenyl)-5-(trifluoromethyl)nicotina mide | B | 539 [M+H] |
| 133 | 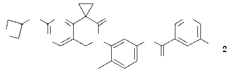 | tert-butyl 3-((6'-(2-methyl-5-(5-(trifluoromethyl)nicotina mido)phenyl)-7'-oxo-6',7'-dihydro-5'H-spiro[cyclopropane-1,8'-pyrido[4,3-d]pyrimidin]-2'-yl)amino)azetidine-1-carboxylate | B | 624 [M+H] |
| 134 | 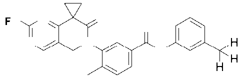 | 3-(2'-amino-7'-oxo-5'H-spiro[cyclopropane-1,8'-pyrido[4,3-d]pyrimidine]-6'(7'H)-yl)-4-methyl-N-(3-(trifluoromethyl)phenyl)b enzamide | G | 468 [M+H] |

### <Experimental Example 1> Evaluation of Enzyme Activity Inhibition

Table 7 below shows the results of enzyme activity inhibition experiments for kinases ACK1, BMX, and Src conducted on selected example compounds by Reaction Biology (RBC) in the United States.
[* <10 nM: A, <100 nM: B, >100 nM: C].

**[Table 7]**

| Example No. | Enzyme activity IC₅₀ (nM) | | |
|---|---|---|---|
| | ACK1 | BMX | Src |
| 1 | A | A | A |
| 2 | B | B | B |
| 3 | B | A | B |
| 6 | A | A | A |
| 7 | A | A | A |
| 8 | A | A | B |
| 19 | A | A | A |
| 20 | B | B | B |
| 24 | A | A | A |
| 25 | B | C | C |
| 26 | B | A | A |
| 28 | B | A | C |
| 29 | B | A | A |
| 31 | B | A | B |
| 51 | B | A | A |
| 70 | B | N.A | N.A |

### <Experimental Example 2> Cell Line Inhibition Analysis

C4-2B and 22Rv1 cells were purchased from ATCC and cultured according to the manufacturer's instructions. The cells were seeded in 96-well plates at 7×10³cells/100 µl/well and allowed to attach for one day. After removing the culture medium, 90 µl/well of fresh culture medium was added, followed by addition of 10 µl/well of culture medium containing compounds at nine concentrations (0.076-500 µM) serially diluted 3-fold and DMSO control to achieve final concentrations of 0-50 µM, and then cultured for 72 hours in a 37 °C CO₂ incubator. Cell proliferation after 72 hours was assessed by adding 10 µl/well of CCK-8 solution, orbital shaking for 30 seconds, then incubating at 37 °C CO₂ incubator for 2 hours and measuring absorbance at 450 nm using a microplate reader. The measured absorbance values were corrected by subtracting the absorbance of wells containing only culture medium and CCK-8 solution, and GI₅₀ values were calculated using GraphPad Prism 8 software.

Meanwhile, in a separate 96-well plate, cells were seeded at 7 × 10³ cells/100 µl/well, allowed to attach for one day, CCK-8 solution was added, and absorbance was measured to establish the 'Growth (%) = 0' value.

The results are shown in Table 3 below [* <1 µM: A, <5 µM: B, <10 µM: C, >10 µM: D].

**[Table 8]**

| Example No. | Cell growth inhibition GI₅₀ (µM) | |
|---|---|---|
| | C4-2B | 22Rv1 |
| 1 | B | N.A |
| 2 | B | B |
| 3 | B | D |
| 4 | B | B |
| 5 | B | B |
| 6 | B | A |
| 7 | A | A |
| 8 | A | A |
| 9 | D | N.A |
| 10 | D | N.A |
| 11 | D | N.A |
| 12 | B | N.A |
| 13 | D | N.A |
| 14 | D | N.A |
| 15 | C | N.A |
| 16 | D | N.A |
| 17 | D | N.A |
| 18 | D | N.A |
| 19 | B | B |
| 20 | A | N.A |
| 21 | D | N.A |
| 22 | D | N.A |
| 23 | C | N.A |
| 24 | B | C |
| 25 | D | N.A |
| 26 | A | A |
| 27 | C | N.A |
| 28 | B | N.A |
| 29 | B | N.A |
| 30 | B | N.A |
| 31 | B | N.A |
| 32 | B | N.A |
| 33 | C | N.A |
| 34 | C | N.A |
| 35 | C | N.A |
| 36 | C | N.A |
| 37 | D | N.A |

Furthermore, experiments were conducted on A172, A549, HCI-H460, AsPC-1, MIA-Paca-2, DLD-1, HCT-116, HT-29, SW480, SW620, MDA-MB-231, and SKOV3 cell lines using the same method, with results shown in Tables 9 and 10 below [* <1 µM: A, <5 µM: B, <10 µM: C, >10 µM: D].

**[Table 9]**

| | Cell growth inhibition GI₅₀ (µM) | | | | | |
|---|---|---|---|---|---|---|
| Example No. | A172 | A549 | HCI-H460 | AsPC-1 | MIA-Paca-2 | DLD-1 |
| 2 | A | D | B | A | A | B |
| 3 | N.A | N.A | N.A | B | N.A | N.A |
| 7 | N.A | N.A | N.A | A | N.A | N.A |
| 8 | A | D | B | B | A | B |
| 24 | A | D | B | B | B | B |
| 28 | N.A | N.A | N.A | C | N.A | N.A |
| 38 | N.A | N.A | N.A | A | N.A | N.A |
| 43 | N.A | N.A | N.A | A | N.A | N.A |
| 51 | A | B | A | A | A | B |
| 64 | N.A | N.A | N.A | A | N.A | N.A |
| 126 | B | B | N.A | N.A | N.A | N.A |
| 127 | A | D | N.A | N.A | N.A | N.A |
| 130 | B | D | N.A | N.A | N.A | N.A |
| 131 | B | D | N.A | N.A | N.A | N.A |

**[Table 10]**

| | Cell growth inhibition GI50 (µM) | | | | | |
|---|---|---|---|---|---|---|
| Example No. | HCT-116 | HT-29 | SW480 | SW620 | MDA-MB-231 | SKOV3 |
| 2 | A | D | B | B | B | B |
| 3 | B | ND | B | B | B | N.A |
| 7 | B | N.A | B | B | B | N.A |
| 8 | A | D | B | B | B | B |
| 24 | A | D | B | A | B | B |
| 28 | A | N.A | B | B | N.A | N.A |
| 38 | A | N.A | A | A | N.A | N.A |
| 43 | A | N.A | N.A | N.A | N.A | N.A |
| 51 | A | B | A | B | A | A |
| 64 | A | N.A | A | A | A | N.A |
| 126 | N.A | C | N.A | N.A | N.A | N.A |
| 127 | N.A | D | N.A | N.A | N.A | N.A |
| 130 | N.A | D | N.A | N.A | N.A | N.A |
| 131 | N.A | D | N.A | N.A | N.A | N.A |

### <Experimental Example 3> Measurement of Kinase Inhibition Activity

To measure the inhibitory activity of protein kinases against the compounds of the present invention, a biochemical assay was performed using a full kinase panel. The inhibitory efficacy of kinases was measured when Example compounds 8 and 51 were treated at a single concentration of 1 µM, and residual enzyme activity values were calculated. Kinases were identified when the calculated residual enzyme activity values were 40% or less, i.e., inhibited by 60% or more. The results are shown in Tables 11 to 14 below.

Tables 11 and 12 below show the results for Example compound 8.

**[Table 11]**

| | | | | |
|---|---|---|---|---|
| **ABL1** | O | | **LYN B** | O |
| **ABL2/ARG** | O | | **MAK** | X |
| **ACK1** | O | | **MAPKAPK2** | X |
| **AKT1** | X | | **MAPKAPK3** | X |
| **AKT2** | X | | **MAPKAPKS/PRAK** | X |
| **AKT3** | X | | **MARK1** | X |
| **ALK** | X | | **MARK2/PAR-IBA** | X |
| **ALK1/ACVRL1** | X | | **MARK3** | X |
| **ALK2/ACVR1** | X | | **MARK4** | X |
| **ALK3/BMPR1A** | X | | **MAST3** | X |
| **ALK4/ACVR1B** | X | | **MASTL** | X |
| **ALK5/TGFBR1** | X | | **MEK1** | X |
| **ALK6/BMPR1B** | X | | **MEK2** | X |
| **ARAF** | O | | **MEK3** | X |
| **ARK5/NUAK1** | X | | **MEK5** | O |
| **ASK1/MAP3K5** | X | | **MEKK1** | X |
| **AURORA A** | X | | **MEKK2** | O |
| **AURORA B** | X | | **MEKK3** | O |
| **AURORA C** | X | | **MEKK6** | X |
| **AXL** | X | | **MELK** | X |
| **BLK** | O | | **MINK/MINK1** | O |
| **BMPR2** | X | | **MKK4** | X |
| **BMX/ETK** | O | | **MKK6** | X |
| **BRAF** | O | | **MKK7** | X |
| **BRK** | O | | **MLCK/MYLK** | X |
| **BRSK1** | X | | **MLCK2/MYLK2** | O |
| **BRSK2** | X | | **MLK1/MAP3K9** | X |
| **BTK** | O | | **MLK2/MAP3K10** | X |
| **C-KIT** | O | | **MLK3/MAP3K11** | O |
| **C-MER** | X | | **MLK4** | X |
| **C-MET** | X | | **MNK1** | X |
| **C-SRC** | O | | **MNK2** | X |
| **CAMK1A** | X | | **MRCKA/CDC42BPA** | X |
| **CAMK1B** | X | | **MRCKB/CDC42BPB** | X |
| **CAMK1D** | X | | **MSK1/RPS6KA5** | X |
| **CAMKIG** | X | | **MSK2/RPS6KA4** | X |
| **CAMK2A** | X | | **MSSK1/STK23** | X |
| **CAMK2B** | X | | **MST1/STK4** | X |
| **CAMK2D** | X | | **MST2/STK3** | X |
| **CAMK2G** | X | | **MST3/STK24** | X |
| **CAMK4** | X | | **MST4** | X |
| **CAMKK1** | X | | **MUSK** | O |
| **CAMKK2** | X | | **MYLK3** | X |
| **CDC7/DBF4** | X | | **MYLK4** | X |
| **CDK1/CYCLIN A** | X | | **MYO3A** | X |
| **CDK1/CYCLIN B** | X | | **MYO3B** | X |
| **CDK1/CYCLIN E** | X | | **NEK1** | X |
| **CDK14/CYCLIN Y (PFTK1)** | X | | **NEK11** | X |
| **CDK16/CYCLIN Y (PCTAIRE)** | X | | **NEK2** | X |
| **CDK17/CYCLIN Y (PCTK2)** | X | | **NEK3** | X |
| **CDK18/CYCLIN Y (PCTK3)** | X | | **NEK4** | O |
| **CDK2/CYCLIN A** | X | | **NEK5** | X |
| **CDK2/CYCLIN A1** | X | | **NEK6** | X |
| **CDK2/CYCLIN E** | X | | **NEK7** | X |
| **CDK2/CYCLIN E2** | X | | **NEK9** | X |
| **CDK2/CYCLIN O** | X | | **NIM1** | X |
| **CDK3/CYCLIN E** | X | | **NLK** | X |
| **CDK3/CYCLIN E2** | X | | **OSR1/OXSR1** | X |
| **CDK4/CYCLIN D1** | X | | **P38A/MAPK14** | O |
| **CDK4/CYCLIN D2** | X | | **P38B/MAPK11** | O |
| **CDK4/CYCLIN D3** | X | | **P38D/MAPK13** | X |
| **CDK5/P25** | X | | **P38G** | X |
| **CDK5/P35** | X | | **P70S6K/RPS6KB1** | X |
| **CDK6/CYCLIN D1** | X | | **P70S6KB/RPS6KB2** | X |
| **CDK6/CYCLIN D2** | X | | **PAK1** | X |
| **CDK6/CYCLIN D3** | X | | **PAK2** | X |
| **CDK7/CYCLIN H** | X | | **PAK3** | X |
| **CDK9/CYCLIN K** | X | | **PAK4** | X |
| **CDK9/CYCLIN T1** | X | | **PAK5** | X |
| **CDK9/CYCLIN T2** | X | | **PAK6** | X |
| **CHK1** | X | | **PASK** | X |
| **CHK2** | X | | **PBK/TOPK** | X |
| **CK1A1** | X | | **PDGFRA** | O |
| **CK1A1L** | X | | **PDGFRB** | O |
| **CK1D** | X | | **PDK1/PDPK1** | X |
| **CKIEPSILON** | X | | **PHKG1** | X |
| **CK1G1** | X | | | |
| **CK1G2** | X | | | |
| **CKIG3** | X | | | |
| **CK2A** | X | | | |
| **CK2A2** | X | | | |
| **CLK1** | X | | | |
| **CLK2** | X | | | |
| **CLK3** | X | | | |
| **CLK4** | X | | | |
| **COT1/MAP3K8** | X | | | |
| **CSK** | O | | | |
| **CTK/MATK** | X | | | |
| **DAPK1** | X | | | |
| **DAPK2** | X | | | |
| **DCAMKL1** | X | | | |
| **DCAMKL2** | X | | | |
| **DDR1** | O | | | |
| **DDR2** | O | | | |
| **DMPK** | X | | | |
| **DMPK2** | X | | | |
| **DRAK1/STK17A** | X | | | |
| **DYRK1/DYRK1A** | X | | | |
| **DYRK1B** | X | | | |
| **DYRK2** | X | | | |
| **DYRK3** | X | | | |
| **DYRK4** | X | | | |
| **EGFR** | O | | | |
| **EPHA1** | O | | | |
| **EPHA2** | O | | | |
| **EPHA3** | O | | | |
| **EPHA4** | O | | | |
| **EPHAS** | O | | | |
| **EPHA6** | O | | | |
| **EPHA7** | O | | | |
| **EPHAS** | O | | | |
| **EPHB1** | O | | | |
| **EPHB2** | O | | | |
| **EPHB3** | O | | | |
| **EPHB4** | O | | | |

**[Table 12]**

| | |
|---|---|
| **PHKG2** | X |
| **PIM1** | X |
| **PIM2** | X |
| **PIM3** | X |
| **PKA** | X |
| **PKACB** | X |
| **PKACG** | O |
| **PKCA** | X |
| **PKCB1** | X |
| **PKCB2** | X |
| **PKCD** | X |
| **PKCEPSILON** | X |
| **PKCETA** | X |
| **PKCG** | X |
| **PKCIOTA** | X |
| **PKCMU/PRKD1** | X |
| **PKCNU/PRKD3** | X |
| **PKCTHETA** | X |
| **PKCZETA** | X |
| **PKD2/PRKD2** | X |
| **PKG1A** | X |
| **PKG1B** | X |
| **PKG2/PRKG2** | X |
| **PKN1/PRK1** | X |
| **PKN2/PRK2** | X |
| **PKN3/PRK3** | X |
| **PLK1** | X |
| **PLK2** | X |
| **PLK3** | X |
| **PLK4/SAK** | X |
| **PRKX** | X |
| **PYK2** | O |
| **RAF1** | O |
| **RET** | O |
| **RIPK2** | X |
| **RIPK4** | X |
| **RIPK5** | X |
| **ROCK1** | X |
| **ROCK2** | X |

| | | | | |
|---|---|---|---|---|
| **ERBB2/HER2** | O | | **RON/MSTIR** | X |
| **ERBB4/HER4** | O | | **ROS/ROS1** | O |
| **ERK1** | X | | **RSK1** | O |
| **ERK2/MAPK1** | X | | **RSK2** | X |
| **ERK5/MAPK7** | X | | **RSK3** | X |
| **ERK7/MAPK15** | X | | **RSK4** | X |
| **ERN1/IRE1** | X | | **SBK1** | X |
| **ERN2/IRE2** | X | | **SGK1** | X |
| **FAK/PTK2** | O | | **SGK2** | X |
| **FER** | O | | **SGK3/SGKL** | X |
| **FES/FPS** | O | | **SIK1** | O |
| **FGFR1** | O | | **SIK2** | X |
| **FGFR2** | O | | **SIK3** | O |
| **FGFR3** | O | | **SLK/STK2** | O |
| **FGFR4** | O | | **SNARK/NUAK2** | X |
| **FGR** | O | | **SNRK** | X |
| **FLT1/VEGFR1** | O | | **SRMS** | O |
| **FLT3** | O | | **SRPK1** | X |
| **FLT4/VEGFR3** | O | | **SRPK2** | X |
| **FMS** | O | | **SSTK/TSSK6** | X |
| **FRK/PTKS** | O | | **STK16** | X |
| **FYN** | O | | **STK21/CIT** | X |
| **GCK/MAP4K2** | O | | **STK22D/TSSK1** | X |
| **GLK/MAP4K3** | O | | **STK25/YSK1** | X |
| **GRK1** | X | | **STK32B/YANK2** | O |
| **GRK2** | X | | **STK32C/YANK3** | X |
| **GRK3** | X | | **STK33** | X |
| **GRK4** | X | | **STK38/NDR1** | X |
| **GRK5** | X | | **STK38L/NDR2** | X |
| **GRK6** | X | | **STK39/STLK3** | X |
| **GRK7** | X | | **SYK** | X |
| **GSK3A** | X | | **TAK1** | O |
| **GSK3B** | X | | TAOK1 | O |
| **HASPIN** | X | | **TAOK2/TAO1** | O |
| **HCK** | O | | **TAOK3/JIK** | O |
| **HGK/MAP4K4** | O | | **TBK1** | X |
| **HIPK1** | X | | **TEC** | O |
| **HIPK2** | X | | **TESK1** | O |
| **HIPK3** | X | | **TESK2** | O |
| **HIPK4** | X | | **TGFBR2** | X |
| **HPK1/MAP4K1** | O | | **TIE2/TEK** | O |
| **IGFIR** | X | | **TLK1** | X |
| **IKKA/CHUK** | X | | **TLK2** | X |
| **IKKB/IKBKB** | X | | **TNIK** | O |
| **IKKE/IKBKE** | X | | **TNK1** | O |
| **IR** | X | | **TRKA** | O |
| **IRAK1** | X | | **TRKB** | O |
| **IRAK4** | X | | **TRKC** | O |
| **IRR/INSRR** | X | | **TSSK2** | X |
| **ITK** | X | | **TSSK3/STK22C** | X |
| **JAK1** | O | | **TTBK1** | X |
| **JAK2** | O | | **TTBK2** | X |
| **JAK3** | O | | **TXK** | O |
| **JNK1** | X | | **TYK1/LTK** | O |
| **JNK2** | X | | **TYK2** | O |
| **JNK3** | X | | **TYRO3/SKY** | X |
| **KDR/VEGFR2** | O | | **ULK1** | X |
| **KHS/MAP4K5** | O | | **ULK2** | X |
| **KSR1** | X | | **ULK3** | X |
| **KSR2** | X | | **VRK1** | X |
| **LATS1** | X | | **VRK2** | X |
| **LATS2** | O | | **WEE1** | X |
| **LCK** | O | | **WNK1** | X |
| **LCK2/ICK** | X | | **WNK2** | X |
| **LIMK1** | O | | **WNK3** | X |
| **LIMK2** | O | | **YES/YES1** | O |
| **LKB1** | X | | **YSK4/MAP3K19** | O |
| **LOK/STK10** | O | | **ZAK/MLTK** | O |
| **LRRK2** | X | | **ZAP70** | X |
| **LYN** | O | | **ZIPK/DAPK3** | X |

Tables 13 and 14 below show the results for Example compound 51.

**[Table 13]**

| | | | | |
|---|---|---|---|---|
| ABL1 | O | | **MAK** | X |
| **ABL2/ARG** | O | | **MAPKAPK2** | X |
| ACK1 | O | | **MAPKAPK3** | X |
| **AKT1** | X | | **MAPKAPKS/PRAK** | X |
| **AKT2** | X | | **MARK1** | X |
| **AKT3** | X | | **MARK2/PAR-IBa** | X |
| ALK | X | | **MARK3** | X |
| **ALK1/ACVRL1** | X | | **MARK4** | X |
| **ALK2/ACVR1** | X | | **MAST3** | X |
| **ALK3/BMPR1A** | X | | **MASTL** | X |
| **ALK4/ACVR1B** | X | | **MEK1** | X |
| **ALK5/TGFBR1** | X | | **MEK2** | X |
| **ALK6/BMPR1B** | X | | **MEK3** | X |
| **ARAF** | O | | **MEK5** | O |
| **ARKS/NUAKl** | X | | **MEKK1** | X |
| **ASK1/MAP3K5** | X | | **MEKK2** | O |
| **Aurora A** | X | | **MEKK3** | O |
| **AURORA B** | X | | **MEKK6** | X |
| AURORA C | X | | **MELK** | X |
| **AXL** | X | | **MINK/MINK1** | O |
| **BLK** | O | | **MKK4** | X |
| **BMPR2** | X | | **MKK6** | X |
| BMX/ETK | O | | **MKK7** | X |
| BRAF | O | | **MLCK/MYLK** | X |
| **BRK** | O | | **MLCK2/MYLK2** | O |
| **BRSK1** | X | | **MLK1/MAP3K9** | O |
| **BRSK2** | X | | **MLK2/MAP3K10** | O |
| **BTK** | O | | **MLK3/MAP3K11** | O |
| c-Kit | O | | **MLK4** | X |
| c-MER | X | | **MNK1** | X |
| **c-MET** | X | | **MNK2** | X |
| **c-Src** | O | | **MRCKa/CDC42BPA** | X |
| **CAMKla** | X | | **MRCKb/CDC42BPB** | X |
| **CAMK1b** | X | | **MSK1/RPS6KA5** | X |
| **CAMK1d** | X | | **MSK2/RPS6KA4** | X |
| **CAMK1g** | X | | **MSSK1/STK23** | X |
| **CAMK2a** | X | | **MST1/STK4** | X |
| **CAMK2b** | X | | **MST2/STK3** | X |
| **CAMK2d** | X | | **MST3/STK24** | X |
| **CAMK2g** | X | | **MST4** | X |
| **CAMK4** | X | | **MUSK** | O |
| **CAMKK1** | X | | **MYLK3** | X |
| **CAMKK2** | X | | **MYLK4** | X |
| **CDC7/DBF4** | X | | **MYO3A** | X |
| **CDK1/cyclin A** | X | | **MYO3b** | X |
| **CDK1/cyclin B** | X | | **NEK1** | X |
| **CDK1/cyclin E** | X | | **NEK11** | O |
| **CDK14/cyclin Y (PFTK1)** | X | | **NEK2** | X |
| **CDK16/cyclin Y (PCTAIRE)** | X | | **NEK3** | X |
| **CDK17/cyclin Y (PCTK2)** | X | | NEK4 | O |
| **CDK18/cyclin Y (PCTK3)** | X | | **NEK5** | X |
| **CDK19/cyclin C** | X | | **NEK6** | X |
| **CDK2/CYCLIN A** | X | | **NEK7** | X |
| **CDK2/Cyclin A1** | X | | **NEK9** | X |
| **CDK2/CYCLIN E** | X | | **NIM1** | X |
| **CDK2/cyclin E2** | X | | **NLK** | X |
| **CDK2/cyclin O** | X | | **OSR1/OXSR1** | X |
| CDK3/cyclin E | X | | **P38a/MAPK14** | O |
| **CDK3/cyclin E2** | X | | **P38b/MAPK11** | O |
| CDK4/cyclin D1 | X | | **P38d/MAPK13** | X |
| **CDK4/cyclin D3** | X | | **P38g** | X |
| **CDK5/P25** | X | | **p70S6K/RPS6KB1** | O |
| **CDK5/p35** | X | | **p70S6Kb/RPS6KB2** | X |
| **CDK6/cyclin D1** | X | | **PAK1** | X |
| CDK6/cyclin D3 | X | | **PAK2** | X |
| **CDK7/cyclin H** | X | | **PAK3** | X |
| **CDK8/cyclin C** | X | | **PAK4** | X |
| **CDK9/CYCLIN K** | X | | **PAK5** | X |
| **CDK9/cyclin T1** | X | | **PAK6** | X |
| **CDK9/cyclin T2** | X | | **PASK** | X |
| **CHK1** | X | | **PBK/TOPK** | X |
| **CHK2** | X | | **PDGFRa** | O |
| **CKlal** | X | | **PDGFRb** | O |
| **CKlalL** | X | | **PDK1/PDPK1** | X |
| CKld | X | | **PHKgl** | X |
| **CKlepsilon** | X | | **PHKg2** | X |
| | | | | |
| **CK1g1** | X | | | |
| **CKlg2** | X | | | |
| **CKIG3** | X | | | |
| **CK2a** | X | | | |
| **CK2a2** | X | | | |
| **CLK1** | X | | | |
| **CLK2** | X | | | |
| **CLK3** | X | | | |
| **CLK4** | X | | | |
| **COT1/MAP3K8** | X | | | |
| CSK | O | | | |
| CTK/MATK | X | | | |
| **DAPK1** | X | | | |
| **DAPK2** | X | | | |
| **DCAMKL1** | X | | | |
| **DCAMKL2** | X | | | |
| **DDR1** | O | | | |
| **DDR2** | O | | | |
| **DLK/MAP3K12** | X | | | |
| **DMPK** | X | | | |
| **DMPK2** | X | | | |
| **DRAK1/STK17A** | X | | | |
| **DYRK1/DYRK1A** | X | | | |
| **DYRK1B** | X | | | |
| DYRK2 | X | | | |
| DYRK3 | X | | | |
| DYRK4 | X | | | |
| **EGFR** | O | | | |
| **EPHA1** | O | | | |
| EPHA2 | O | | | |
| **EPHA3** | O | | | |
| **EPHA4** | O | | | |
| **EPHA5** | O | | | |
| **EPHA6** | O | | | |
| **EPHA7** | O | | | |
| **EPHA8** | O | | | |
| **EPHB1** | O | | | |
| **EPHB2** | O | | | |
| EPHB3 | O | | | |
| **PIM1** | X | | | |
| **PIM2** | X | | | |
| **PIM3** | X | | | |
| **PKA** | X | | | |
| **PKAcb** | X | | | |
| **PKAcg** | O | | | |
| **PKCa** | X | | | |
| **PKCbl** | X | | | |
| **PKCb2** | X | | | |
| **PKCd** | X | | | |
| **PKCepsilon** | X | | | |
| **PKCeta** | X | | | |
| **PKCg** | X | | | |
| **PKCiota** | X | | | |
| **PKCmu/PRKD1** | X | | | |
| **PKCnu/PRKD3** | X | | | |
| **PKCtheta** | X | | | |
| **PKCzeta** | X | | | |
| **PKD2/PRKD2** | X | | | |
| **PKGla** | X | | | |
| **PKG1b** | X | | | |
| **PKG2/PRKG2** | X | | | |
| **PKMYT1** | X | | | |
| **PKN1/PRK1** | X | | | |
| **PKN2/PRK2** | X | | | |
| **PKN3/PRK3** | X | | | |
| **PLK1** | X | | | |
| **PLK2** | X | | | |
| **PLK3** | X | | | |
| **PLK4/SAK** | X | | | |
| **PRKX** | X | | | |
| **PYK2** | O | | | |
| **RAF1** | O | | | |
| **RET** | O | | | |
| **RIPK2** | X | | | |
| **RIPK3** | O | | | |
| **RIPK4** | X | | | |
| **RIPK5** | X | | | |
| **ROCK1** | X | | | |

**[Table 14]**

| | | | | |
|---|---|---|---|---|
| **EPHB4** | O | | **ROCK2** | X |
| ERBB2/HER2 | O | | **RON/MSTIR** | X |
| **ERBB4/HER4** | O | | **ROS/ROS1** | O |
| **ERK1** | X | | **RSK1** | O |
| **ERK2/MAPK1** | X | | **RSK2** | X |
| ERK5/MAPK7 | X | | **RSK3** | X |
| **ERK7/MAPK15** | X | | **RSK4** | X |
| **ERN1/IRE1** | X | | **SBK1** | X |
| **ERN2/IRE2** | X | | **SGK1** | X |
| **FAK/PTK2** | O | | **SGK2** | X |
| **FER** | O | | **SGK3/SGKL** | X |
| **FES/FPS** | O | | **SIK1** | O |
| **FGFR1** | O | | **SIK2** | O |
| **FGFR2** | O | | **SIK3** | O |
| **FGFR3** | O | | **SLK/STK2** | O |
| **FGFR4** | O | | **SNARK/NUAK2** | X |
| **FGR** | O | | **SNRK** | X |
| **FLT1/VEGFR1** | O | | **SRMS** | O |
| **FLT3** | O | | **SRPK1** | X |
| **FLT4/VEGFR3** | O | | **SRPK2** | X |
| **FMS** | O | | **SSTK/TSSK6** | X |
| **FRK/PTKS** | O | | **STK16** | X |
| **FYN** | O | | **STK21/CIT** | X |
| **GCK/MAP4K2** | O | | **STK22D/TSSK1** | X |
| **GLK/MAP4K3** | O | | **STK25/YSK1** | X |
| **GRK1** | X | | **STK32B/YANK2** | O |
| **GRK2** | X | | **STK32C/YANK3** | X |
| **GRK3** | X | | **STK33** | X |
| **GRK4** | X | | **STK38/NDR1** | X |
| **GRK5** | X | | **STK38L/NDR2** | X |
| **GRK6** | X | | **STK39/STLK3** | X |
| **GRK7** | X | | **SYK** | O |
| **GSK3a** | X | | **TAK1** | O |
| **GSK3b** | X | | **TAOK1** | O |
| **Haspin** | X | | **TAOK2/TAO1** | O |
| **HCK** | O | | **TAOK3/JIK** | O |
| **HGK/MAP4K4** | O | | **TBK1** | X |
| **HIPK1** | X | | **TEC** | O |
| **HIPK2** | X | | **TESK1** | O |
| **HIPK3** | X | | **TESK2** | O |
| **HIPK4** | O | | **TGFBR2** | X |
| **HPK1/MAP4K1** | O | | **TIE2/TEK** | O |
| **IGFIR** | X | | **TLK1** | X |
| **IKKa/CHUK** | X | | **TLK2** | X |
| **IKKb/IKBKB** | X | | **TNIK** | O |
| **IKKe/IKBKE** | X | | **TNK1** | O |
| **IR** | X | | **TRKA** | O |
| **IRAK1** | X | | **TRKB** | O |
| **IRAK4** | X | | **TRKC** | O |
| **IRR/INSRR** | X | | **TSSK2** | X |
| **ITK** | X | | **TSSK3/STK22C** | X |
| **JAK1** | O | | **TTBK1** | X |
| **JAK2** | O | | **TTBK2** | X |
| **JAK3** | O | | **TXK** | O |
| **JNK1** | O | | **TYK1/LTK** | X |
| **INK2** | O | | **TYK2** | O |
| **JNK3** | O | | **TYRO3/SKY** | O |
| **KDR/VEGFR2** | O | | **ULK1** | X |
| **KHS/MAP4K5** | O | | **ULK2** | X |
| **KSR1** | X | | **ULK3** | X |
| **KSR2** | X | | **VRK1** | X |
| **LATS1** | X | | **VRK2** | X |
| **LATS2** | O | | **WEE1** | X |
| **LCK** | O | | **WNK1** | X |
| **LCK2/ICK** | X | | **WNK2** | X |
| **LIMK1** | O | | **WNK3** | X |
| **LIMK2** | O | | **YES/YES1** | O |
| **LKB1** | X | | **YSK4/MAP3K19** | O |
| **LOK/STK10** | O | | **ZAK/MLTK** | O |
| **LRRK2** | O | | **ZAP70** | X |
| **LYN** | O | | **ZIPK/DAPK3** | X |
| **LYN B** | O | | | |

Hereinafter, formulation examples of compositions comprising compound 8 according to the present invention are described; however, the present invention is not intended to be limited thereto but is merely intended to provide specific description.

### <Formulation Example 1> Formulation example of a pharmaceutical composition

### <Formulation Example 1-1> Preparation of powder

Compound 8 (20 mg), lactose (100 mg), and talc (10 mg) were mixed and filled into airtight sachets to prepare a powder.

### <Prescription Example 1-2> Preparation of Tablets

Compound 8 (10 mg), corn starch (100 mg), lactose (100 mg), and magnesium stearate (2 mg) were mixed and then compressed into tablets according to conventional tablet manufacturing methods.

### <Prescription Example 1-3> Preparation of Capsules

Compound 8 (10 mg), corn starch (100 mg), lactose (100 mg), and magnesium stearate (2 mg) were mixed according to conventional capsule preparation methods, and the mixture was filled into gelatin capsules to prepare capsules.

### <Prescription Examples 1-4> Preparation of Injection

Compound 8 (10 mg), an appropriate amount of sterile distilled water for injection, and an appropriate amount of pH adjuster were mixed and then prepared according to a conventional method for preparing injections to contain the above components per ampoule (2 mL).

### <Formulation Example 2> Health Supplement

### <Formulation Example 2-1> Preparation of Health Food

Compound 8 (1 mg), vitamin mixture in appropriate amounts (vitamin A acetate 70 µg, vitamin E 1.0 mg, vitamin B1 0.13 mg, vitamin B2 0.15 mg, vitamin B6 0.5 mg, vitamin B12 0.2 µg, vitamin C 10 mg, biotin 10 µg, nicotinamide 1.7 mg, folic acid 50 µg, calcium pantothenate 0.5 mg) and mineral mixture in appropriate amounts (ferrous sulfate 1.75 mg, zinc oxide 0.82 mg, magnesium carbonate 25.3 mg, monopotassium phosphate 15 mg, dicalcium phosphate 55 mg, potassium citrate 90 mg, calcium carbonate 100 mg, magnesium chloride 24.8 mg) were mixed, then granules were prepared and a health food was manufactured according to conventional methods.

### <Formulation Example 2-2> Preparation of Health Beverage

Compound 8 (1 mg), citric acid (1000 mg), oligosaccharide (100 g), Japanese apricot concentrate (2 g), taurine (1 g), and purified water were added to make a total volume of 900 mL. The above components were mixed according to a conventional health beverage preparation method, then stirred and heated at 85°C for about 1 hour. The resulting solution was filtered, collected in a sterilized 2 L container, sealed, sterilized, and stored under refrigeration.

The foregoing has described specific aspects of the present invention in detail. It will be apparent to those skilled in the art that such specific descriptions are merely preferred embodiments and do not limit the scope of the present invention. Therefore, the substantial scope of the present invention should be defined by the appended claims and their equivalents.

## Claims

1. A compound selected from the group consisting of a compound represented by Formula 1, a stereoisomer thereof, a pharmaceutically acceptable salt thereof, a prodrug thereof, or a solvate thereof: 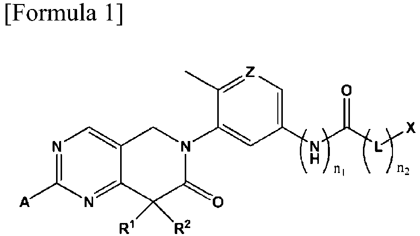 wherein:
R¹ and R² may each independently selected from hydrogen or (C₁-C₄)alkyl, or may be taken together to form a 3- to 5-membered rings;
X is selected from (substituted or unsubstituted) (C₁-C₄)alkyl, (C₃-C₆)cycloalkyl, (C₄-C₆)cycloalkenyl, phenyl, benzyl, or 5- or 6-membered single or bicyclic heterocyclic rings,
wherein the substituent is selected from one or more members of the group consisting of (C₁-C₄)alkyl, (C₁-C₄)alkoxy, (C₃-C₆)cycloalkyl, trifluoromethyl, trifluoromethyl(C₁-C₂)alkoxy, halo, hydroxy, imidazolyl, (C₁-C₂)alkylimidazolyl, (C₁-C₂)alkoxyphenyl, morpholino, cyano, oxo(=O), (C₁-C₂)alkoxy(C₁-C₂)alkylamino, piperazinyl, (C₁-C₂)alkylpiperazinyl, ((C₁-C₂)alkylpiperazin-1-yl)(C₁-C₂)alkyl and di(C₁-C₂)alkylamino(C₁-C₂)alkylamino;
A is selected from morpholino or NH-Y, wherein Y is hydrogen, or substituted or unsubstituted group selected from phenyl, benzyl, (C₁-C₄)alkyl, (C₂-C₃)alkenyl, (C₃-C₆)cycloalkyl, pyridinyl, pyrazolyl, tetrahydropyranyl, benzothiazolyl, benzofuranyl, or azetidine, wherein the substituent is selected from one or more members of the group consisting of (C₁-C₂)alkyl, (C₁-C₄)alkoxy, piperazinyl, (C₁-C₂)alkylpiperazinyl, acetylpiperazinyl, morpholino, morpholine-4-carbonyl, (C₁-C₂)alkylpiperazine-1-carbonyl, oxetanyl, ((C₁-C₂)alkylpiperazin-1-yl)(C₁-C₂)alkyl, imidazolyl, pyrrolidinyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, halo, trifluoro(C₁-C₂)alkyl, trifluoro(C₁-C₂)alkoxy, di(C₁-C₂)alkylamino(C₁-C₂)alkylamino, pyridinyl, furanyl, hydroxy and tert-butoxycarbonyl (BOC);
Z is CH or N;
L is CH₂ or NH;
n₁ or n₂ is an integer from 0 to 1.

2. The compound of claim 1, wherein the compound is represented by Formula 1-1: 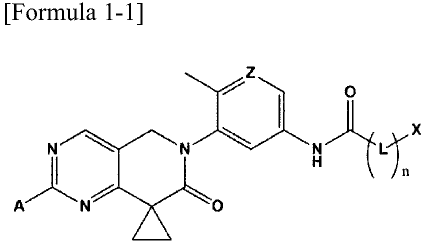 wherein:
X is selected from (substituted or unsubstituted) (C₁-C₄)alkyl, (C₃-C₆)cycloalkyl, (C₄-C₆)cycloalkenyl, phenyl, benzyl, or 5- or 6-membered monocyclic or bicyclic heterocyclic compounds, wherein the substituent is selected from one or more members of the group consisting of (C₁-C₄)alkyl, (C₁-C₄)alkoxy, (C₃-C₆)cycloalkyl, trifluoromethyl, trifluoromethyl(C₁-C₂)alkoxy, halo, hydroxy, imidazolyl, (C₁-C₂)alkylimidazolyl, (C₁-C₂)alkoxyphenyl, morpholino, cyano, oxo(=O), (C₁-C₂)alkoxy(C₁-C₃)alkyl, (C₁-C₂)alkylamino, piperazinyl, (C₁-C₂)alkylpiperazinyl, ((C₁-C₂)alkylpiperazin-1-yl)(C₁-C₂)alkyl and di(C₁-C₂)alkylamino (C₁-C₂)alkyl(C₁-C₂)alkylamino;
A is selected from morpholino or -NH-Y,
wherein Y is selected from hydrogen, or substituted or unsubstituted phenyl, benzyl, (C₁-C₄)alkyl, (C₂-C₃)alkenyl, (C₃-C₆)cycloalkyl, pyridinyl, pyrazolyl, tetrahydropyranyl, benzothiazolyl, benzofuranyl, or azetidinyl, wherein the substituent is selected from one or more members of the group consisting of (C₁-C₂)alkyl, (C₁-C₄)alkoxy, piperazinyl, (C₁-C₂)alkylpiperazinyl, acetylpiperazinyl, morpholino, morpholine-4-carbonyl, (C₁-C₂)alkylpiperazine-1-carbonyl, oxetanyl, ((C₁-C₂)alkylpiperazin-1-yl)(C₁-C₂)alkyl, imidazolyl, pyrrolidinyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, halo, trifluoro(C₁-C₂)alkyl, trifluoro(C₁-C₂)alkoxy, di(C₁-C₂)alkylamino(C₁-C₂)alkyl(C₁-C₂)alkylamino, pyridinyl, furanyl, hydroxy and tert-butoxycarbonyl (BOC);
Z is CH or N;
L is CH₂ or NH;
n is an integer from 0 to 1.

3. The compound of claim 1,
wherein the 5- or 6-membered monocyclic or bicyclic heterocyclic compound is selected from the group consisting of thiophene, furan, pyrazole, oxazole, thiazole, pyridine, pyran, tetrahydropyran, oxazine, thiazine, pyrimidine, piperazine, and benzothiazole.

4. The compound of claim 1, wherein the compound is represented by Formula 1-2: 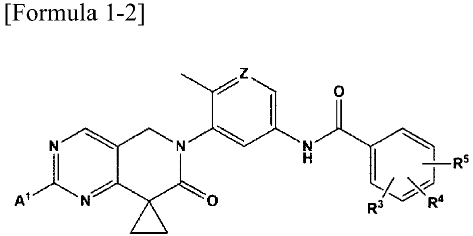 wherein:
R₃ to R₅ may each be the same or different, and are selected from the group consisting of hydrogen, (C₁-C₂)alkyl, (C₁-C₂) alkoxy, trifluoromethyl, halo, hydroxy, (C₁-C₂)alkylimidazolyl, (C₁-C₂)alkoxyphenyl, morpholino, (C₁-C₂)alkoxy(C₁-C₂)alkyl(C₁-C₂)alkylamino, piperazinyl, (C₁-C₂)alkylpiperazinyl, ((C₁-C₂)alkylpiperazin-1-yl)(C₁-C₂)alkyl and di(C₁-C₂)alkylamino(C₁-C₂)alkyl(C₁-C₂)alkylamino;
A¹ is selected from morpholino or NH-Y¹,
wherein Y¹ is selected from hydrogen, (C₃-C₄)cycloalkyl, benzyl, 1H-pyrazolyl, di(C₁-C₂)alkyl-1H-pyrazolyl, (oxetan-3-yl)-1H-pyrazolyl, trifluoro(C₁-C₂)alkyl-1H-pyrazolyl, pyridinyl(C₁-C₂)alkyl, hydroxy(C₁-C₂)alkyl, hydroxy(C₂-C₄)alkenyl, hydroxy(C₃-C₆)cycloalkyl, morpholino(C₃-C₄)alkyl, furanyl(C₁-C₂)alkyl, tert-butoxycarbonyl (BOC) substituted azetidine, tetrahydropyranyl, benzothiazolyl, benzofuranyl, and substituted or unsubstituted phenyl or pyridinyl, wherein the substituent is selected from (C₁-C₂)alkyl, (C₁-C₂)alkoxy, halo, trifluoro(C₁-C₂)alkoxy, piperazinyl, (C₁-C₂)alkylpiperazinyl, acetylpiperazinyl, morpholino, morpholine-4-carbonyl, (C₁-C₂)alkylpiperazine-1-carbonyl, ((C₁-C₂)alkylpiperazin-1-yl)(C₁-C₂)alkyl, imidazolyl, di(C₁-C₂)alkylaminopyrrolidinyl, piperidinyl, acetylpiperidinyl, di(C₁-C₂)alkylaminopiperidinyl, ((C₁-C₂)alkylpiperazin-1-yl)piperidinyl, and di(C₁-C₂)alkylamino(C₁-C₂)alkyl(C₁-C₂)alkylamino.

5. The compound of claim 1, wherein the compound is represented by Formula 1-3: 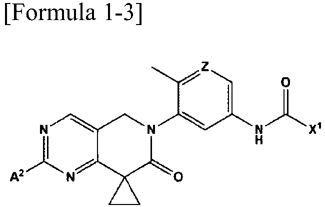 wherein:
X¹ is selected from (C₃-C₆)cycloalkyl, oxo(C₃-C₆)cycloalkyl, (C₄-C₆)cycloalkenyl, thiophene, (C₁-C₂)alkylthiophene, furan, thiazole, oxazole, benzothiazole, tetrahydropyran, trifluoro(C₁-C₃)alkyl; or pyridinyl substituted with one or more substituents selected from the group consisting of (C₁-C₂)alkyl, (C₁-C₂)alkoxy, trifluoromethyl, halo and hydroxy;
A² is selected from morpholino or NH-Y²,
wherein Y² is one or more selected from the group consisting of hydrogen, benzyl, tetrahydropyranyl, benzothiazolyl, benzofuranyl, (C₁-C₂)alkylpyridinyl, pyridinyl(C₁-C₂)alkyl, hydroxy(C₁-C₂)alkyl, hydroxy(C₂-C₄)alkenyl, hydroxy(C₃-C₆)cycloalkyl, morpholino(C₃-C₄)alkyl, furanyl(C₁-C₂)alkyl, ((C₁-C₂)alkylpiperazin-1-yl)piperidinyl-trifluoro(C₁-C₂)alkoxypyridinyl, and tert-butoxycarbonyl (BOC) substituted azetidine.

6. The compound of claim 1, wherein the compound is represented by Formula 1-4: 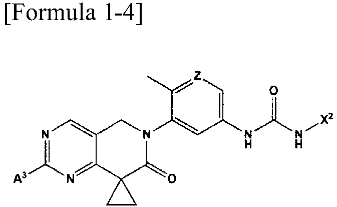 wherein:
X² is selected from (C₃-C₆) cycloalkyl, (C₃-C₆)cycloalkyl(C₁-C₂)alkyl, benzyl, or substituted or unsubstituted phenyl, wherein the substituent is one or more selected from the group consisting of (C₁-C₂) alkyl, (C₁-C₂)alkoxy, halo, trifluoro(C₁-C₂)alkyl,trifluoro(C₁-C₂)alkoxy and cyano,
A³ is selected from morpholino or NH-Y³,
wherein Y³ is one or more selected from hydrogen or (C₁-C₂)alkylpyridinyl.

7. The compound of claim 1, wherein the compound is represented by Formula 1-5: 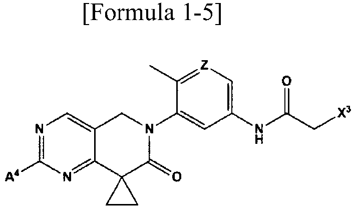 wherein:
X³ is selected from substituted or unsubstituted phenyl, wherein the substituent is selected from one or more members of the group consisting of (C₁-C₂) alkyl, (C₁-C₂) alkoxy and halo,
A⁴ is selected from morpholino or NH-Y⁴, wherein Y⁴ is one or more selected from hydrogen, benzyl, tetrahydropyranyl, benzothiazolyl, benzofuranyl, morpholino(C₃-C₄)alkyl, furanyl(C₁-C₂)alkyl or pyridinyl(C₁-C₂)alkyl.

8. The compound of claim 1, wherein the compound is represented by Formula 1-6: 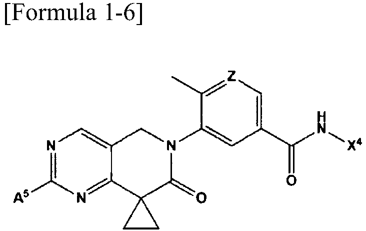 wherein:
X⁴ is selected from substituted or unsubstituted phenyl, wherein the substitution is selected from one or more members of the group consisting of (C₁-C₂) alkyl, (C₁-C₂)alkoxy, halo and trifluoromethyl,
A⁵ is selected from morpholino or NH-Y⁵, wherein Y⁵ is hydrogen, or one or more selected from (C₁-C₂)alkylpyridinyl.

9. The compound of claim 1 selected from the group consisting of N-(3-(2'-((1,3-dimethyl-1H-pyrazol-5-yl)amino)-7'-oxo-5'H-spiro[cyclopropane-1,8'-pyrido[4,3-d]pyrimidin]-6'(7'H)-yl)-4-methylphenyl)-3-(trifluoromethyl)benzamide (compound 1), N-(3-(2'-amino-7'-oxo-5'H-spiro[cyclopropane-1,8'-pyrido[4,3-d]pyrimidine]-6'(7'H)-yl)-4-methylphenyl)-3-(trifluoromethyl)benzamide (compound 2), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (compound 3), N-(3-(2'-amino-7'-oxo-5'H-spiro[cyclopropane-1,8'-pyrido[4,3-d]pyrimidine]-6'(7'H)-yl)-4-methylphenyl)-4-fluoro-3-(trifluoromethyl)benzamide (compound 4), N-(3-(2'-amino-7'-oxo-5'H-spiro[cyclopropane-1,8'-pyrido[4,3-d]pyrimidine]-6'(7'H)-yl)-4-methylphenyl)-4-chloro-3-(trifluoromethyl)benzamide (compound 5), N-(3-(2'-amino-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide (compound 6), N-(3-(2'-amino-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamide (compound 7), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methyl-1H-imidazol-1-yl)-5-(trifluoromethyl)benzamide (compound 8), N-(3-(2'-amino-7'-oxo-5'H-spiro[cyclopropane-1,8'-pyrido[4,3-d]pyrimidine]-6'(7'H)-yl)-4-methylphenyl)-3,4-difluorobenzamide (compound 9), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,4-difluorobenzamide (compound 10), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3,5-difluorobenzamide (compound 11), N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-methyl-3-(trifluoromethyl)benzamide (compound 12), 3-bromo-N-(3-(2'-(cyclopropylamino)-7'-oxo-5'H-spiro[cyclopropane-1,8'-pyrido[4,3-d]pyrimidine]-6'(7'H)-yl)-4-methylphenyl)-5-(trifluoromethyl)benzamide (compound 13), N-(3-(2'-amino-7'-oxo-5'H-spiro[cyclopropane-1,8'-pyrido[4,3-d]pyrimidine]-6'(7'H)-yl)-4-methylphenyl)-2,4-difluorobenzamide (compound 14), N-(3-(2'-amino-7'-oxo-5'H-spiro[cyclopropane-1,8'-pyrido[4,3-d]pyrimidine]-6'(7'H)-yl)-4-methylphenyl)-3,5-difluorobenzamide (compound 15), N-(3-(2'-amino-7'-oxo-5'H-spiro[cyclopropane-1,8'-pyrido[4,3-d]pyrimidine]-6'(7'H)-yl)-4-methylphenyl)-3-methoxybenzamide (compound 16), N-(3-(2'-amino-7'-oxo-5'H-spiro[cyclopropane-1,8'-pyrido[4,3-d]pyrimidine]-6'(7'H)-yl)-4-methylphenyl)-3-chloro-2-fluoro-5-(trifluoromethyl)benzamide (compound 17), N-(3-(2'-amino-7'-oxo-5'H-spiro[cyclopropane-1,8'-pyrido[4,3-d]pyrimidine]-6'(7'H)-yl)-4-methylphenyl)-2-fluoro-5-(trifluoromethyl)benzamide (compound 18), N-(3-(2'-amino-7'-oxo-5'H-spiro[cyclopropane-1,8'-pyrido[4,3-d]pyrimidine]-6'(7'H)-yl)-4-methylphenyl)-3-methoxy-5-(trifluoromethyl)benzamide (compound 19), N-(3-(2'-amino-7'-oxo-5'H-spiro[cyclopropane-1,8'-pyrido[4,3-d]pyrimidine]-6'(7'H)-yl)-4-methylphenyl)-4'-methoxy-5-(trifluoromethyl)-[1,1'-biphenyl]-3-carboxamide (compound 20), N-(3-(2'-amino-7'-oxo-5'H-spiro[cyclopropane-1,8'-pyrido[4,3-d]pyrimidine]-6'(7'H)-yl)-4-methylphenyl)-6-(trifluoromethyl)picolinamide (compound 21), N-(3-(2'-amino-7'-oxo-5'H-spiro[cyclopropane-1,8'-pyrido[4,3-d]pyrimidine]-6'(7'H)-yl)-4-methylphenyl)-2-(trifluoromethyl)isonicotinamide (compound 22), N-(3-(2'-amino-7'-oxo-5'H-spiro[cyclopropane-1,8'-pyrido[4,3-d]pyrimidine]-6'(7'H)-yl)-4-methylphenyl)-3-hydroxy-5-(trifluoromethyl)benzamide (compound 23), N-(3-(2'-amino-7'-oxo-5'H-spiro[cyclopropane-1,8'-pyrido[4,3-d]pyrimidine]-6'(7'H)-yl)-4-methylphenyl)-5-(trifluoromethyl)nicotinamide (compound 24), N-(3-(2'-amino-7'-oxo-5'H-spiro[cyclopropane-1,8'-pyrido[4,3-d]pyrimidine]-6'(7'H)-yl)-4-methylphenyl)-2-chloro-5-(trifluoromethyl)benzamide (compound 25), N-(3-(2'-amino-7'-oxo-5'H-spiro[cyclopropane-1,8'-pyrido[4,3-d]pyrimidine]-6'(7'H)-yl)-4-methylphenyl)-3-chloro-5-(trifluoromethyl)benzamide (compound 26), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-fluoro-5-(trifluoromethyl)benzamide (compound 27), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide (compound 28), N-(3-(2'-amino-7'-oxo-5'H-spiro[cyclopropane-1,8'-pyrido[4,3-d]pyrimidine]-6'(7'H)-yl)-4-methylphenyl)-3-morpholino-5-(trifluoromethyl)benzamide (compound 29), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide (compound 30), N-(3-(2'-amino-7'-oxo-5'H-spiro[cyclopropane-1,8'-pyrido[4,3-d]pyrimidine]-6'(7'H)-yl)-4-methylphenyl)-3-((2-methoxyethyl)(methyl)amino)-5-(trifluoromethyl)benzamide (compound 31), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide (compound 32), N-(3-(2'-amino-7'-oxo-5'H-spiro[cyclopropane-1,8'-pyrido[4,3-d]pyrimidine]-6'(7'H)-yl)-4-methylphenyl)-3-(4-methylpiperazin-1-yl)-5-(trifluoromethyl)benzamide (compound 33), N-(3-(2'-amino-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide (compound 34), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-(piperazin-1-yl)-5-(trifluoromethyl)benzamide (compound 35), N-(3-(2'-(cyclopropylamino)-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide (compound 36), N-(3-(2'-amino-7'-oxo-5'H-spiro[cyclopropane-1,8'-pyrido[4,3-d]pyrimidine]-6'(7'H)-yl)-4-methylphenyl)-3-((2-(dimethylamino)ethyl)(methyl)amino)-5-(trifluoromethyl)benzamide (compound 37), N-(4-methyl-3-(2'-((4-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 38), N-(3-(2'-((4-(4-acetylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (compound 39), N-(4-methyl-3-(2'-((4-morpholinophenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 40), N-(4-methyl-3-(2'-((4-(4-methylpiperazine-1-carbonyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (compound 41), N-(3-(2'-((4-(4-ethylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (compound 42), N-(4-methyl-3-(2'-((4-((4-methylpiperazin-1-yl)methyl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 43), N-(3-(2'-((4-(1H-imidazol-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 44), N-(4-methyl-3-(7'-oxo-2'-((4-(piperazin-1-yl)phenyl)amino)-5'H-spiro[cyclopropane-1,8'-pyrido[4,3-d]pyrimidine]-6'(7'H)-yl)phenyl)-3-(trifluoromethyl)benzamide (Compound 45), (S)-N-(3-(2'-((4-(3-(dimethylamino)pyrrolidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 46), N-(4-methyl-3-(7'-oxo-2'-((4-(piperidin-4-yl)phenyl)amino)-5'H-spiro[cyclopropane-1,8'-pyrido[4,3-d]pyrimidine]-6'(7'H)-yl)phenyl)-3-(trifluoromethyl)benzamide (Compound 47), N-(4-methyl-3-(2'-((3-(4-methylpiperazin-1-yl)phenyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 48), N-(3-(2'-((4-(1-acetylpiperidin-4-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 49), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-4-((4-methylpiperazin-1-yl)methyl)-3-(trifluoromethyl)benzamide (Compound 50), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 51), N-(4-methyl-3-(2'-((1-(oxetan-3-yl)-1H-pyrazol-4-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 52), N-(3-(2'-((2-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 53), N-(3-(2'-((2-methoxy-4-(morpholine-4-carbonyl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 54), N-(3-(2'-((3-methoxy-4-(4-methylpiperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 55), N-(3-(2'-((3-methoxy-4-morpholinophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 56), N-(3-(2'-((4-(4-ethylpiperazin-1-yl)-3-fluorophenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 57), N-(3-(2'-((6-(4-ethylpiperazin-1-yl)-2-methoxypyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 58), N-(4-methyl-3-(7'-oxo-2'-(phenylamino)-5'H-spiro[cyclopropane-1,8'-pyrido[4,3-d]pyrimidine]-6'(7'H)-yl)phenyl)-3-(trifluoromethyl)benzamide (Compound 59), N-(3-(2'-((4-(4-(dimethylamino)piperidin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 60), N-(3-(2'-amino-7'-oxo-5'H-spiro[cyclopropane-1,8'-pyrido[4,3-d]pyrimidine]-6'(7'H)-yl)-4-methylphenyl)-4-((4-methylpiperazin-1-yl)methyl)-3-(trifluoromethyl)benzamide (Compound 61), N-(3-(2'-(cyclopropylamino)-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide (Compound 62), N-(3-(2'-((4-((2-(dimethylamino)ethyl)(methyl)amino)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 63), N-(4-methyl-3-(7'-oxo-2'-((1-(2,2,2-trifluoroethyl)-1H-pyrazol-4-yl)amino)-5'H-spiro[cyclopropane-1,8'-pyrido[4,3-d]pyrimidine]-6'(7'H)-yl)phenyl)-3-(trifluoromethyl)benzamide (Compound 64), N-(3-(2'-amino-7'-oxo-5'H-spiro[cyclopropane-1,8'-pyrido[4,3-d]pyrimidine]-6'(7'H)-yl)-4-methylphenyl)-4-(4-methylpiperazin-1-yl)-3-(trifluoromethyl)benzamide (Compound 65), N-(3-(2'-((4-(4-acetylpiperazin-1-yl)-2-methoxyphenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 66), N-(3-(2'-((3-methoxy-4-(piperazin-1-yl)phenyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 67), N-(3-(2'-((1,3-dimethyl-1H-pyrazol-5-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 68), N-(3-(2'-((2-methoxy-6-(4-methylpiperazin-1-yl)pyridin-3-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 69), N-(3-(2'-(hydroxyamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 70), 1-(4-fluorophenyl)-3-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)urea (Compound 71), 4-chloro-N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)benzamide (Compound 72), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)thiophene-2-carboxamide (Compound 73), N-(4-methyl-3-(2'-((6-methylpyridin-3-yl)amino)-7'-oxo-5'H-spiro[cyclopropane-1,8'-pyrido[4,3-d]pyrimidine]-6'(7'H)-yl)phenyl)furan-2-carboxamide (Compound 74), N-(3-(2'-amino-7'-oxo-5'H-spiro[cyclopropane-1,8'-pyrido[4,3-d]pyrimidine]-6'(7'H)-yl)-4-methylphenyl)cyclobutanecarboxamide (Compound 75), N-(3-(2'-amino-7'-oxo-5'H-spiro[cyclopropane-1,8'-pyrido[4,3-d]pyrimidine]-6'(7'H)-yl)-4-methylphenyl)-3-oxocyclobutane-1-carboxamide (Compound 76), N-(3-(2'-amino-7'-oxo-5'H-spiro[cyclopropane-1,8'-pyrido[4,3-d]pyrimidine]-6'(7'H)-yl)-4-methylphenyl)cyclohex-1-ene-1-carboxamide (Compound 77), N-(3-(2'-amino-7'-oxo-5'H-spiro[cyclopropane-1,8'-pyrido[4,3-d]pyrimidine]-6'(7'H)-yl)-4-methylphenyl)-4-methylthiophene-2-carboxamide (Compound 78), N-(3-(2'-amino-7'-oxo-5'H-spiro[cyclopropane-1,8'-pyrido[4,3-d]pyrimidine]-6'(7'H)-yl)-4-methylphenyl)isoxazole-5-carboxamide (Compound 79), N-(3-(2'-amino-7'-oxo-5'H-spiro[cyclopropane-1,8'-pyrido[4,3-d]pyrimidine]-6'(7'H)-yl)-4-methylphenyl)benzo[d]thiazole-2-carboxamide (Compound 80), N-(3-(2'-amino-7'-oxo-5'H-spiro[cyclopropane-1,8'-pyrido[4,3-d]pyrimidine]-6'(7'H)-yl)-4-methylphenyl)-4,4,4-trifluorobutanamide (Compound 81), N-(3-(2'-amino-7'-oxo-5'H-spiro[cyclopropane-1,8'-pyrido[4,3-d]pyrimidine]-6'(7'H)-yl)-4-methylphenyl)thiazole-5-carboxamide (Compound 82), N-(3-(2-amino-7'-oxo-5'H-spiro[cyclopropane-1,8'-pyrido[4,3-d]pyrimidine]-6'(7'H)-yl)-4-methylphenyl)cyclopentanecarboxamide (Compound 83), N-(3-(2-amino-7'-oxo-5'H-spiro[cyclopropane-1,8'-pyrido[4,3-d]pyrimidine]-6'(7'H)-yl)-4-methylphenyl)tetrahydro-2H-pyran-4-carboxamide (Compound 84), N-(3-(2'-(benzylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 85), N-(4-methyl-3-(2'-morpholino-7'-oxo-5'H-spiro[cyclopropane-1,8'-pyrido[4,3-d]pyrimidine]-6'(7'H)-yl)phenyl)-3-(trifluoromethyl)benzamide (Compound 86), N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-3-(trifluoromethyl)benzamide (Compound 87), N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-3-(trifluoromethyl)benzamide (Compound 88), N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 89), N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 90), N-(3-(2'-(benzylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 91), N-(4-methyl-3-(2'-morpholino-7'-oxo-5'H-spiro[cyclopropane-1,8'-pyrido[4,3-d]pyrimidine]-6'(7'H)-yl)phenyl)-5-(trifluoromethyl)nicotinamide (Compound 92), N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)-5-(trifluoromethyl)nicotinamide (Compound 93), N-(4-methyl-3-(7'-oxo-2'-((pyridin-3-ylmethyl)amino)-5'H-spiro[cyclopropane-1,8'-pyrido[4,3-d]pyrimidine]-6'(7'H)-yl)phenyl)-5-(trifluoromethyl)nicotinamide (Compound 94), N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)-5-(trifluoromethyl)nicotinamide (Compound 95), N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 96), N-(3-(2'-(benzylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide (Compound 97), 2-(3,5-difluorophenyl)-N-(4-methyl-3-(2'-morpholino-7'-oxo-5'H-spiro[cyclopropane-1,8'-pyrido[4,3-d]pyrimidine]-6'(7'H)-yl)phenyl)acetamide (Compound 98), 2-(3,5-difluorophenyl)-N-(4-methyl-3-(7'-oxo-2'-((tetrahydro-2H-pyran-4-yl)amino)-5'H-spiro[cyclopropane-1,8'-pyrido[4,3-d]pyrimidine]-6'(7'H)-yl)phenyl)acetamide (Compound 99), 2-(3,5-difluorophenyl)-N-(4-methyl-3-(7'-oxo-2'-((pyridin-3-ylmethyl)amino)-5'H-spiro[cyclopropane- 1,8'-pyrido[4,3-d]pyrimidine]-6'(7'H)-yl)phenyl)acetamide (Compound 100), 2-(3,5-difluorophenyl)-N-(4-methyl-3-(2'-((3-morpholinopropyl)amino)-7'-oxo-5'H-spiro[cyclopropane-1,8'-pyrido[4,3-d]pyrimidine]-6'(7'H)-yl)phenyl)acetamide (Compound 101), 2-(3,5-difluorophenyl)-N-(3-(2'-((furan-2-ylmethyl)amino)-7'-oxo-5'H-spiro[cyclopropane-1,8'-pyrido[4,3-d]pyrimidine]-6'(7'H)-yl)-4-methylphenyl)acetamide (Compound 102), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 103), N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 104), N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-3-(trifluoromethyl)benzamide (Compound 105), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 106), N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 107), N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)-5-(trifluoromethyl)nicotinamide (Compound 108), N-(3-(2'-(benzo[d]thiazol-5-ylamino)-7'-oxo-5'H-spiro[cyclopropane-1,8'-pyrido[4,3-d]pyrimidine]-6'(7'H)-yl)-4-methylphenyl)-2-(3,5-difluorophenyl)acetamide (Compound 109), 2-(3,5-difluorophenyl)-N-(3-(2'-((2,3-dihydrobenzofuran-4-yl)amino)-7'-oxo-5'H-spiro[cyclopropane-1,8'-pyrido[4,3-d]pyrimidine]-6'(7'H)-yl)-4-methylphenyl)acetamide (Compound 110), 2-(3,5-difluorophenyl)-N-(4-methyl-3-(2'-((6-(4-(4-methylpiperazin-1-yl)piperidin-1-yl)-2-(2,2,2-trifluoroethoxy)pyridin-3-yl)amino)-7'-oxo-5'H-spiro[cyclopropane-1,8'-pyrido[4,3-d]pyrimidine]-6'(7'H)-yl)phenyl)acetamide (Compound 111), 1-(3-(2'-amino-7'-oxo-5'H-spiro[cyclopropane-1,8'-pyrido[4,3-d]pyrimidine]-6'(7'H)-yl)-4-methylphenyl)-3-cyclopropylurea (Compound 112), 1-(3-(2'-amino-7'-oxo-5'H-spiro[cyclopropane-1,8'-pyrido[4,3-d]pyrimidine]-6'(7'H)-yl)-4-methylphenyl)-3-cyclobutylurea (Compound 113), 1-(3-(2'-amino-7'-oxo-5'H-spiro[cyclopropane-1,8'-pyrido[4,3-d]pyrimidine]-6'(7'H)-yl)-4-methylphenyl)-3-cyclopentylurea (Compound 114), 1-(3-(2'-amino-7'-oxo-5'H-spiro[cyclopropane-1,8'-pyrido[4,3-d]pyrimidine]-6'(7'H)-yl)-4-methylphenyl)-3-(cyclopropylmethyl)urea (Compound 115), 1-(3-(2'-amino-7'-oxo-5'H-spiro[cyclopropane-1,8'-pyrido[4,3-d]pyrimidine]-6'(7'H)-yl)-4-methylphenyl)-3-(cyclohexylmethyl)urea (Compound 116), 1-(3-(2'-amino-7'-oxo-5'H-spiro[cyclopropane-1,8'-pyrido[4,3-d]pyrimidine]-6'(7'H)-yl)-4-methylphenyl)-3-benzylurea (Compound 117), 1-(3-(2'-amino-7'-oxo-5'H-spiro[cyclopropane-1,8'-pyrido[4,3-d]pyrimidine]-6'(7'H)-yl)-4-methylphenyl)-3-(4-(trifluoromethoxy)phenyl)urea (Compound 118), 1-(3-(2'-amino-7'-oxo-5'H-spiro[cyclopropane-1,8'-pyrido[4,3-d]pyrimidine]-6'(7'H)-yl)-4-methylphenyl)-3-(3-cyanophenyl)urea (Compound 119), 1-(3-(2'-amino-7'-oxo-5'H-spiro[cyclopropane-1,8'-pyrido[4,3-d]pyrimidine]-6'(7'H)-yl)-4-methylphenyl)-3-(3,4-difluorophenyl)urea (Compound 120), 1-(3-(2'-amino-7'-oxo-5'H-spiro[cyclopropane-1,8'-pyrido[4,3-d]pyrimidine]-6'(7'H)-yl)-4-methylphenyl)-3-(2-methoxyphenyl)urea (Compound 121), 1-(3-(2'-amino-7'-oxo-5'H-spiro[cyclopropane-1,8'-pyrido[4,3-d]pyrimidine]-6'(7'H)-yl)-4-methylphenyl)-3-(3-(trifluoromethyl)phenyl)urea (Compound 122), N-(5-(2'-amino-7'-oxo-5'H-spiro[cyclopropane-1,8'-pyrido[4,3-d]pyrimidine]-6'(7'H)-yl)-6-methylpyridin-3-yl)-3-(trifluoromethyl)benzamide (Compound 123),
N-methyl-N-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(trifluoromethyl)benzamide
(Compound 124), 1-(4-methyl-3-(2'-((6-methylpyridin-3-yl)amino)-7'-oxo-5'H-spiro[cyclopropane-1,8'-pyrido[4,3-d]pyrimidin]-6'(7'H)-yl)phenyl)-3-(m-tolyl)urea
(Compound 125), N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 126), (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 127), N-(3-(2'-(((1r,3r)-3-hydroxycyclobutyl)amino)-7'-oxo-5'H-spiro[cyclopropane-1,8'-pyrido[4,3-d]pyrimidine]-6'(7'H)-yl)-4-methylphenyl)-3-(trifluoromethyl)benzamide (Compound 128), tert-butyl 3-((6'-(2-methyl-5-(3-(trifluoromethyl)benzamido)phenyl)-7'-oxo-6',7'-dihydro-5'H-spiro[cyclopropane-1,8'-pyrido[4,3-d]pyrimidin]-2'-yl)amino)azetidine-1-carboxylate (Compound 129), N-(3-(2'-((2-hydroxyethyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 130), (S)-N-(3-(2'-((1-hydroxypropan-2-yl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 131), N-(3-(2'-(((1r,3r)-3-hydroxycyclobutyl)amino)-7'-oxo-5'H-spiro[cyclopropane-1,8'-pyrido[4,3-d]pyrimidine]-6'(7'H)-yl)-4-methylphenyl)-5-(trifluoromethyl)nicotinamide (Compound 132), tert-butyl 3-((6'-(2-methyl-5-(5-(trifluoromethyl)nicotinamido)phenyl)-7'-oxo-6',7'-dihydro-5'H-spiro[cyclopropane-1,8'-pyrido[4,3-d]pyrimidin]-2'-yl)amino)azetidine-1-carboxylate(Compound 133), and 3-(2'-amino-7'-oxo-5'H-spiro[cyclopropane-1,8'-pyrido[4,3-d]pyrimidine]-6'(7'H)-yl)-4-methyl-N-(3-(trifluoromethyl)phenyl)benzamide (Compound 134).

10. The compound of claim 1, wherein the compound inhibits one or more protein kinases selected from the group consisting of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES 1, YSK4/MAP3K19, and ZAK/MLTK.

11. A pharmaceutical composition for treating or preventing protein kinase-related diseases, comprising the compound of claim 1.

12. The pharmaceutical composition of claim 11,
wherein the protein kinase is one or more protein kinases selected from the group consisting of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19, and ZAK/MLTK.

13. The pharmaceutical composition of claim 11, wherein the protein kinase-related diseases are **characterized by** being cancerous diseases.

14. The pharmaceutical composition of claim 13, wherein the cancerous diseases are selected from the group consisting of prostate cancer, endometrial cancer, bladder cancer, gastric cancer, lung cancer, liver cancer, colorectal cancer, small intestine cancer, pancreatic cancer, brain cancer, bone cancer, melanoma, breast cancer, sclerosing adenoma, head and neck cancer, esophageal cancer, thyroid cancer, parathyroid cancer, renal cancer, sarcoma, urethral cancer, leukemia, multiple myeloma, hematologic cancer, lymphoma, and fibroadenoma.

15. The pharmaceutical composition of claim 13, wherein the pharmaceutical composition is **characterized in that** it is administered as a combination therapy with one or more anticancer agents selected from a group consisting of cytotoxic anticancer agents, targeted anticancer agents, immuno-anticancer agents and metabolic anticancer agents.

16. The pharmaceutical composition of claim 11, wherein the said compound is **characterized in that** the inhibition rate of the compound is more than 60% when treated with a single concentration of 1 µM for protein kinases.

17. A health functional food for improving or preventing protein kinase-related diseases comprising a compound according to claim 1.

18. The health functional food of claim 17, wherein the protein kinase is one or more protein kinases selected from the group consisting of ABL1, ABL2/ARG, ACK1, ARAF, BLK, BMX/ETK, BRAF, BRK, BTK, C-KIT, C-SRC, CSK, DDR1, DDR2, EGFR, EPHA1, EPHA2, EPHA3, EPHA4, EPHA5, EPHA6, EPHA7, EPHA8, EPHB1, EPHB2, EPHB3, EPHB4, ERBB2/HER2, ERBB4/HER4, FAK/PTK2, FER, FES/FPS, FGFR1, FGFR2, FGFR3, FGFR4, FGR, FLT1/VEGFR1, FLT3, FLT4/VEGFR3, FMS, FRK/PTK5, FYN, GCK/MAP4K2, GLK/MAP4K3, HCK, HGK/MAP4K4, HIPK4, HPK1/MAP4K1, JAK1, JAK2, JAK3, JNK1, JNK2, JNK3, KDR/VEGFR2, KHS/MAP4K5, LATS2, LCK, LIMK1, LIMK2, LOK/STK10, LRRK2, LYN, LYN B, MEK5, MEKK2, MEKK3, MINK/MINK1, MLCK2/MYLK2, MLK1/MAP3K9, MLK2/MAP3K10, MLK3/MAP3K11, MUSK, NEK4, P38A/MAPK14, P38B/MAPK11, PDGFRA, PDGFRB, PKAcg, PYK2, RAF1, RET, RIPK3, ROS/ROS1, RSK1, SIK1, SIK2, SIK3, SLK/STK2, SRMS, STK32B/YANK2, SYK, TAK1, TAOK1, TAOK2/TAO1, TAOK3/JIK, TEC, TESK2, TIE2/TEK, TNIK, TNK1, TRKA, TRKB, TRKC, TXK, TYK1/LTK, TYK2, TYRO3/SKY, YES/YES1, YSK4/MAP3K19, and ZAK/MLTK.

19. The health functional food of claim 17, wherein the protein kinase-related disease is cancer.
